# EUROPEAN PATENT APPLICATION

(11) **EP 4 644 380 A1**
(43) Date of publication of application: **05.11.2025**
(21) Application number: 23910742.8
(22) Date of filing: 27.12.2023
(51) Int. Cl.: C07D 401/12, C07D 237/34, C07D 471/04, C07D 487/04, A61K 31/502, A61K 31/5025, A61K 31/5377, A61P 19/02, A61P 19/06, A61P 9/10, A61P 25/16, A61P 25/28, A61P 3/10, A61P 9/00

(54) **PYRIDAZINE NLRP3 INHIBITOR COMPOUND, PHARMACEUTICAL COMPOSITION, AND PREPARATION METHOD THEREFOR AND USE THEREOF**

(30) Priority: 28.12.2022 CN 202211701092; 17.01.2023 CN 202310058136; 10.03.2023 CN 202310230219; 26.05.2023 CN 202310607935; 27.09.2023 CN 202311262132; 21.12.2023 CN 202311779751
(71) Applicant: Changchun Genescience Pharmaceutical Co., Ltd., Changchun, Jilin 130012 (CN)
(72) Inventor: YANG, Fanglong, Changchun, Jilin 130012 (CN); HUANG, Yue, Changchun, Jilin 130012 (CN); HE, Weiming, Changchun, Jilin 130012 (CN); WU, Yueting, Changchun, Jilin 130012 (CN); WANG, Siqin, Changchun, Jilin 130012 (CN); JIN, Lei, Changchun, Jilin 130012 (CN)
(74) Representative: Gille Hrabal Partnerschaftsgesellschaft mbB Patentanwälte
(86) International application number: PCT/CN2023/142422
(87) International publication number: WO 2024/140824

(57) **Abstract**

A pyridazine compound represented by formula (I), a pharmaceutical composition, and a preparation method therefor and a use thereof. The compound has a good NLRP3 inhibitory effect, can be used alone or in combination with other drugs to adjust an NLRP3 protein, and is used for treating NLRP3-mediated symptoms and/or diseases, and preparing a drug for preventing or treating such symptoms or diseases.

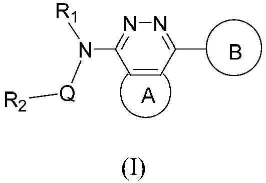

## Description

The present application claims priority to any of the following patent applications:
a prior application filed with the China National Intellectual Property Administration on December 28, 2022, having Patent Application No. 202211701092.7 and entitled "Pyridazine NLRP3 inhibitor compound, pharmaceutical composition, preparation method therefor and use thereof";
a prior application filed with the China National Intellectual Property Administration on January 17, 2023, having Patent Application No. 202310058136.7 and entitled "Pyridazine NLRP3 inhibitor compound, pharmaceutical composition, preparation method therefor and use thereof";
a prior application filed with the China National Intellectual Property Administration on March 10, 2023, having Patent Application No. 202310230219.X and entitled "Pyridazine NLRP3 inhibitor compound, pharmaceutical composition, preparation method therefor and use thereof";
a prior application filed with the China National Intellectual Property Administration on May 26, 2023, having Patent Application No. 202310607935.5 and entitled "Pyridazine NLRP3 inhibitor compound, pharmaceutical composition, preparation method therefor and use thereof";
a prior application filed with the China National Intellectual Property Administration on September 27, 2023, having Patent Application No. 202311262132.7 and entitled "Pyridazine NLRP3 inhibitor compound, pharmaceutical composition, preparation method therefor and use thereof";
a prior application filed with the China National Intellectual Property Administration on December 21, 2023, having Patent Application No. 202311779751.3 and entitled "Pyridazine NLRP3 inhibitor compound, pharmaceutical composition, preparation method therefor and use thereof";
and the above prior applications are incorporated herein by reference in their entirety.

### TECHNICAL FIELD

The present invention belongs to the field of medicine, and specifically relates to a pyridazine NLRP3 inhibitor compound, a pharmaceutical composition, and a preparation method therefor and use thereof.

### BACKGROUND

NOD-like receptor protein 3 (NLRP3) is an important member of the NOD-like receptor family. NLRP3 comprises three parts, the thermal protein domain (PYD), the nucleotide binding domain (NBD) and the leucin-rich repeat (LRR). When the cells receive the stimulation of the risk signal of aseptic inflammation, NLRP3 interacts with the apoptosis-associated speck-like protein containing a CARD (ASC) and pro-caspase-1 to form the NLRP3 inflammasome complex. Activation of NLRP3 inflammasome results in the release of IL-1β and IL-18.

Activation of NLRP3 inflammasome usually requires two steps. The first step involves triggering a signal in which a Toll-like receptor recognizes a pathogen-associated molecular pattern (PAMP) or a damage-associated molecular pattern (DAMP) and transmits the signal to the cell, mediating the activation of the NF-κB signaling pathway and thereby upregulating the transcription levels of components associated with NLRP3 inflammasome, including inactive NLRP3 and pro-IL-1β. The second step involves activating signals. After P2X7 receptors receive the stimulation of ATP, nigericin etc., NLRP3 monomer oligomerizes to form NLRP3 oligomer, and then recruits ASC and pro-caspase-1 to assemble into NLRP3 inflammasome complex. This triggers the transformation of pro-caspase-1 into caspase-1, and caspase-1 cleaves pro-IL-1β and pro-IL-18, promoting the production and secretion of mature IL-1β and IL-18.

Activation of NLRP3 inflammasome is associated with a variety of diseases. Examples include: autoinflammatory febrile syndromes such as cryopyrin-associated periodic syndrome (CAPS), sickle cell disease, systemic lupus erythematosus (SLE), chronic liver disease, nonalcoholic steatohepatitis (NASH), gout, type I and type II diabetes and related complications (e.g. nephropathy, retinopathy), neuroinflammation-related disorders (e.g. multiple sclerosis, brain infection, acute injury, neurodegenerative diseases, Alzheimer's disease), atherosclerosis and cardiovascular risk (e.g. hypertension), hidradenitis suppurativa, wound healing and scarring, and cancer (e.g. carcinoma of large intestine, lung cancer, myeloproliferative neoplasms, leukemia, myelofibrosis).

There are few NLRP3 inflammasome inhibitors currently in development, and it becomes clinically necessary to develop NLRP3 inflammasome inhibitors with higher activity and better druggability.

### SUMMARY

In order to improve the above technical problems, the present invention provides a compound represented by formula (I), a racemate, a stereoisomer, a tautomer, an isotope-labeled counterpart, a solvate, a polymorph, a pharmaceutically acceptable salt thereof or a prodrug thereof: 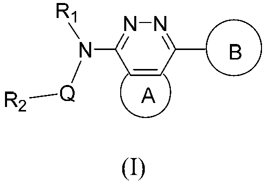
wherein ring A is selected from a C₃₋₁₄ carbocyclic ring, a C₆₋₁₄ aromatic ring, a 5- to 14-membered heteroaromatic ring, or a 3- to 14-membered heterocyclic ring, wherein the C₃₋₁₄ carbocyclic ring, C₆₋₁₄ aromatic ring, 5- to 14-membered heteroaromatic ring, and 3- to 14-membered heterocyclic ring are each independently unsubstituted or optionally substituted with one, two or more Rₐ; each Rₐ is the same or different and is independently selected from H, deuterium, OH, halogen, cyano, NH₂, or the following groups which are unsubstituted or optionally substituted with one, two or more Rₐ₁: C₁₋₁₂ alkyl, C₂₋₁₂ alkenyl, C₂₋₁₂ alkynyl, C₁₋₁₂ alkoxy, Rₐ₁₁-C(=O)-NH-, Rₐ₁₂-C(=O)-, Rₐ₁₃-S(=O)₂-NH-, Rₐ₁₄-S(=O)₂-, -P(=O)(Rₐ₁₅)(Rₐ₁₆), (Rₐ₁₇)(Rₐ₁₈)N-C(=O)-, C₆₋₁₄ aryl, 5- to 14-membered heteroaryl, 3- to 14-membered heterocyclyl, or C₃₋₁₄ cycloalkyl; each Rₐ₁ is the same or different and is independently selected from H, deuterium, OH, halogen, CN, C₁₋₁₂ alkyl, C₃₋₁₂ cycloalkyl, C₁₋₁₂ alkoxy, or halo-C₁₋₁₂ alkyl; Rₐ₁₁, Rₐ₁₂, Rₐ₁₃, Rₐ₁₄, Rₐ₁₅, Rₐ₁₆, Rₐ₁₇, and Rₐ₁₈ are the same or different and are each independently selected from H, deuterium, halogen, CN, C₁₋₁₂ alkyl, or C₁₋₁₂ alkoxy;
ring B is selected from a C₆₋₁₄ aromatic ring, a 5- to 14-membered heteroaromatic ring, a 3- to 14-membered heterocyclic ring, or a C₃₋₁₄ carbocyclic ring, wherein the C₆₋₁₄ aromatic ring, 5- to 14-membered heteroaromatic ring, 3- to 14-membered heterocyclic ring, and C₃₋₁₄ carbocyclic ring are each independently unsubstituted or optionally substituted with one, two or more R_{b}; each R_{b} is the same or different and is independently selected from H, deuterium, OH, halogen, cyano, NH₂, or the following groups which are unsubstituted or optionally substituted with one, two or more R₁₁: C₁₋₁₂ alkyl, or C₁₋₁₂ alkoxy; each R₁₁ is the same or different and is independently selected from H, deuterium, halogen, CN, C₁₋₁₂ alkyl, or C₁₋₁₂ alkoxy;
R₁ is selected from H, deuterium, halogen, CN, hydroxyl, amino, C₁₋₆ alkyl, halo-C₁₋₆ alkyl, cyano-C₁₋₆ alkyl, or C₃₋₈ cycloalkyl;
Q is selected from a chemical bond or C₁₋₆ alkylene;
R₂ is selected from the following groups which are unsubstituted or optionally substituted with one, two or more R₂ₐ: C₁₋₁₂ alkyl, C₁₋₁₂ alkoxy, C₆₋₁₄ aryl, 5- to 14-membered heteroaryl, 3- to 14-membered heterocyclyl, or C₃₋₁₄ cycloalkyl; each R₂ₐ is the same or different and is independently selected from H, deuterium, halogen, CN, OH, carboxyl, or the following groups which are unsubstituted or optionally substituted with one, two or more R₂ₐ₁: C₁₋₁₂ alkyl, C₁₋₁₂ alkoxy, or R₂₁-C(=O)-(CH₂)ₘ-; or two R₂ₐ together with the carbon atom to which they are attached form C₃₋₈ cycloalkyl or 3-8 membered heterocyclyl; each R₂ₐ₁ is the same or different and is independently selected from H, deuterium, halogen, CN, carboxyl, C₁₋₁₂ alkyl, C₁₋₁₂ alkoxy, or R₂₂-C(=O)-; each R₂₁ and R₂₂ are the same or different and are each independently selected from H, deuterium, halogen, CN, OH, amino, C₁₋₁₂ alkylamino, (C₁₋₁₂ alkyl)₂amino, C₃₋₁₂ cycloalkylamino, C₁₋₁₂ alkyl, C₁₋₁₂ alkoxy, C₃₋₈ cycloalkyl, or 3- to 14-membered heterocyclyl; m is an integer selected from 0 to 6;
alternatively, R₂ is selected from 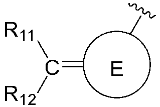 which is unsubstituted or optionally substituted with one, two or more Rₑ, wherein ring E is selected from C₃₋₈ cycloalkyl; R₁₁ and R₁₂ are the same or different and are each independently selected from H, halogen, C₁₋₆ alkyl, halo-C₁₋₆ alkyl, cyano-C₁₋₆ alkyl, C₃₋₈ cycloalkyl, halo-C₃₋₈ cycloalkyl or cyano-C₃₋₈ cycloalkyl; each Rₑ is the same or different and is independently selected from H, OH, halogen, cyano, NH₂, or the following groups which are unsubstituted or optionally substituted with one, two or more Rₑ₁: C₁₋₆ alkyl, or C₃₋₈ cycloalkyl; each Rₑ₁ is the same or different and is independently selected from H, OH, halogen, or cyano;
alternatively, R₂ is selected from 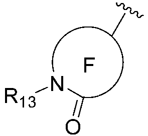 which is unsubstituted or optionally substituted with one, two or more R_{f}, wherein ring F is selected from 4- to 8-membered heterocyclic ring; R₁₃ is selected from H, or the following groups which are unsubstituted or optionally substituted with one, two or more R₁₃ₐ: C₁₋₆ alkyl, C₃₋₈ cycloalkyl, or R_{13b}-C(=O)-(CH₂)ₛ-; R_{13b} is selected from C₁₋₆ alkyl, C₃₋₈ cycloalkyl or amino; each R₁₃ₐ is the same or different and is independently selected from H, OH, halogen, cyano, or C₁₋₆ alkyl; s is selected from integers of 0 to 6; each R₁ is the same or different and is independently selected from H, OH, halogen, cyano, NH₂, or the following groups which are unsubstituted or optionally substituted with one, two or more R_{f1}: C₁₋₆ alkyl, or C₃₋₈ cycloalkyl; each R_{f1} is the same or different and is independently selected from H, OH, halogen, or cyano.

According to some embodiments, ring A is selected from a C₃₋₈ carbocyclic ring, a C₆₋₁₀ aromatic ring, a 5- to 10-membered heteroaromatic ring, or a 3- to 10-membered heterocyclic ring, wherein the C₃₋₈ carbocyclic ring, C₆₋₁₀ aromatic ring, 5- to 10-membered heteroaromatic ring, and 3- to 10-membered heterocyclic ring are each independently unsubstituted or optionally substituted with one, two or more Rₐ; each Rₐ is the same or different and is independently selected from H, deuterium, OH, halogen, cyano, NH₂, or the following groups which are unsubstituted or optionally substituted with one, two or more Rₐ₁: C₁₋₆ alkyl, C₂₋₆ alkenyl, C₂₋₆ alkynyl, C₁₋₆ alkoxy, Rₐ₁₁-C(=O)-NH-, Rₐ₁₂-C(=O)-, Rₐ₁₃-S(=O)₂-NH-, Rₐ₁₄-S(=O)₂-, -P(=O)(Rₐ₁₅)(Rₐ₁₆), (Rₐ₁₇)(Rₐ₁₈)N-C(=O)-, C₆₋₁₀ aryl, 5- to 10-membered heteroaryl, 3- to 10-membered heterocyclyl, or C₃₋₁₀ cycloalkyl; each Rₐ₁ is the same or different and is independently selected from H, deuterium, OH, halogen, CN, C₁₋₆ alkyl, C₃₋₆ cycloalkyl, or C₁₋₆ alkoxy; Rₐ₁₁, Rₐ₁₂, Rₐ₁₃, Rₐ₁₄, Rₐ₁₅, Rₐ₁₆, Rₐ₁₇, and Rₐ₁₈ are the same or different and are each independently selected from H, deuterium, halogen, CN, C₁₋₆ alkyl, or C₁₋₆ alkoxy;

According to some embodiments, ring A is selected from a C₆₋₁₀ aromatic ring, or a 5- to 10-membered heteroaromatic ring, wherein the C₆₋₁₀ aromatic ring and 5- to 10-membered heteroaromatic ring are each independently unsubstituted or optionally substituted with one, two or more Rₐ; each Rₐ is the same or different and is independently selected from H, deuterium, OH, halogen, cyano, NH₂, or the following groups which are unsubstituted or optionally substituted with one, two or more Rₐ₁: C₁₋₆ alkyl, C₂₋₆ alkenyl, C₂₋₆ alkynyl, Rₐ₁₂-C(=O)-, Rₐ₁₄-S(=O)₂-, (Rₐ₁₇)(Rₐ₁₈)N-C(=O)-, C₆₋₁₀ aryl, or 5- to 10-membered heteroaryl; each Rₐ₁ is the same or different and is independently selected from H, deuterium, OH, halogen, CN, C₁₋₆ alkyl, C₃₋₆ cycloalkyl, or C₁₋₆ alkoxy; Rₐ₁₂, Rₐ₁₄, Rₐ₁₇, and Rₐ₁₈ are the same or different and are each independently selected from H, C₁₋₆ alkyl, or C₁₋₆ alkoxy;

According to some embodiments, ring A is selected from a benzene ring or a pyridine ring which is unsubstituted or optionally substituted with one, two or more Rₐ; each Rₐ is the same or different and is independently selected from H, cyano, -C(=O)-NH₂, CH₃-S(=O)₂-, 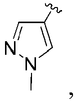 1-propynyl, 2-cyclopropylethynyl, 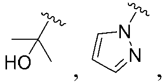 acetyl,

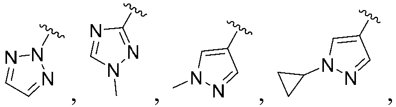

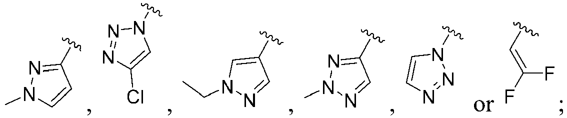

According to some embodiments, ring A is selected from

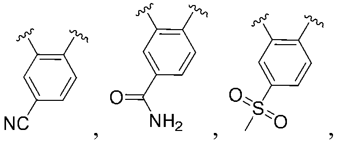

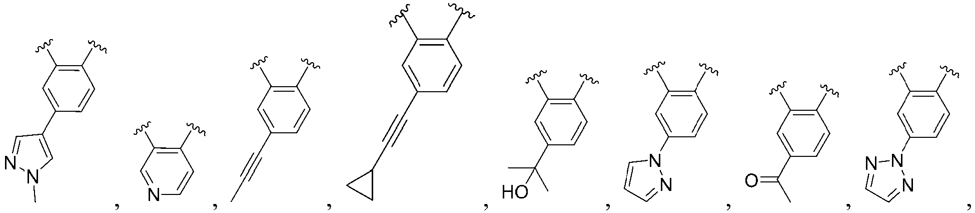

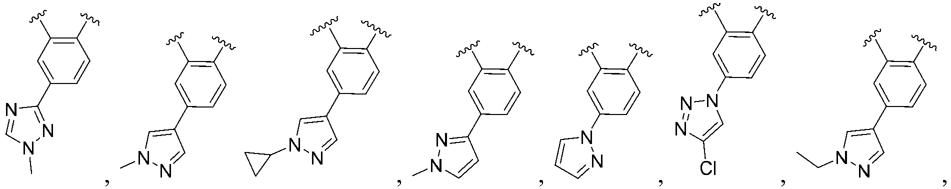

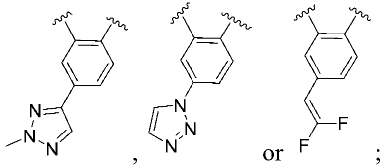

According to some embodiments, 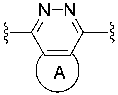 is selected from

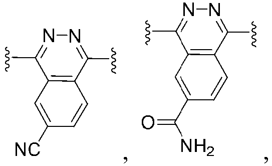

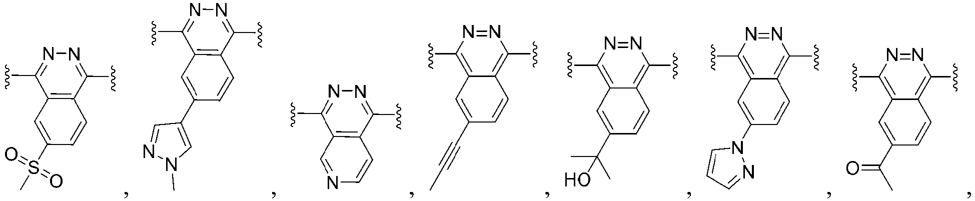

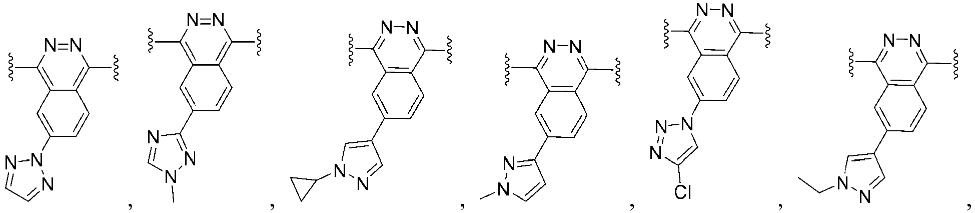

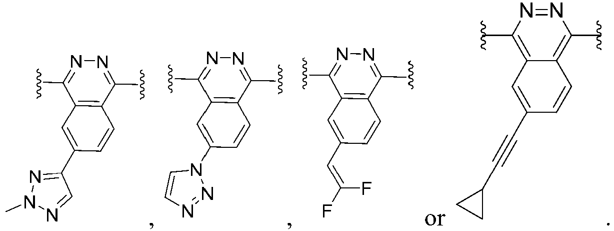

According to some embodiments, ring B is selected from a C₆₋₁₀ aromatic ring, a 5- to 10-membered heteroaromatic ring or a C₃₋₁₀ carbocyclic ring, wherein the C₆₋₁₀ aromatic ring, 5- to 10-membered heteroaromatic ring and C₃₋₁₀ carbocyclic ring are each independently unsubstituted or optionally substituted with one, two or more R_{b}; each R_{b} is the same or different and is independently selected from H, OH, halogen, cyano, NH₂, or the following groups which are unsubstituted or optionally substituted with one, two or more R_{b1}: C₁₋₆ alkyl, or C₁₋₆ alkoxy; each R₁₁ is the same or different and is independently selected from H, halogen, CN, C₁₋₆ alkyl, or C₁₋₆ alkoxy;

According to some embodiments, ring B is selected from a benzene ring, a benzothiophene ring or an indane ring which is unsubstituted or optionally substituted with one, two or more R_{b}; each R_{b} is the same or different and is independently selected from H, OH, halogen, cyano, C₁₋₆ alkyl, halo-C₁₋₆ alkyl, or halo-C₁₋₆ alkoxy;

According to some embodiments, ring B is selected from a benzene ring, a benzothiophene ring or an indane ring which is unsubstituted or optionally substituted with one, two or more R_{b}; each R_{b} is the same or different and is independently selected from H, OH, F, Cl, methyl or CF₃.

According to some embodiments, ring B is selected from 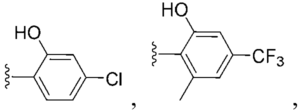 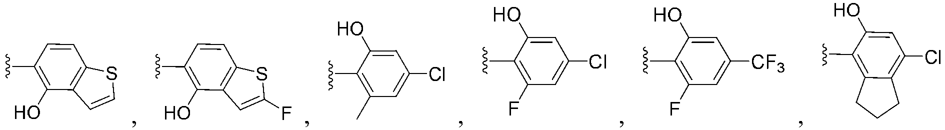 or

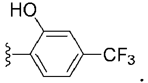

According to some embodiments, ring B is selected from

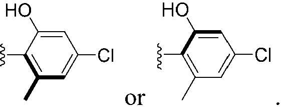

According to some embodiments, R₁ is selected from H, deuterium or methyl; preferably H.

According to some embodiments, Q is selected from a chemical bond, -(CH₂)_{q}- or -(CH₂)_{q}-CH(CH₃)-, and q is selected from 0, 1 or 2;

According to some embodiments, Q is selected from a chemical bond, -CH₂- or -CH(CH₃)-.

According to some embodiments, R₂ is selected from the following groups which are unsubstituted or optionally substituted with one, two or more R₂ₐ: C₁₋₆ alkyl, C₁₋₆ alkoxy, C₆₋₁₀ aryl, 5- to 10-membered heteroaryl, 3- to 10 membered heterocyclyl, or C₃₋₁₀ cycloalkyl; each R₂ₐ is the same or different and is independently selected from H, deuterium, halogen, CN, OH, carboxyl, or the following groups which are unsubstituted or optionally substituted with one, two or more R₂ₐ₁: C₁₋₆ alkyl, C₁₋₆ alkoxy, or R₂₁-C(=O)-(CH₂)ₘ-; or two R₂ₐ together with the carbon atom to which they are attached form C₃₋₈ cycloalkyl; each R₂ₐ₁ is the same or different and is independently selected from H, deuterium, halogen, CN, carboxyl, C₁₋₆ alkyl, C₁₋₆ alkoxy, or R₂₂-C(=O)-; each R₂₁ and R₂₂ are the same or different and are each independently selected from H, OH, amino, C₁₋₆ alkylamino, (C₁₋₆ alkyl)₂amino, C₃₋₆ cycloalkylamino, C₁₋₆ alkyl, C₁₋₆ alkoxy, C₃₋₈ cycloalkyl, or 3- to 8-membered heterocyclyl; m is selected from 0, 1 or 2.

According to some embodiments, R₂ is selected from the following groups which are unsubstituted or optionally substituted with one, two or more R₂ₐ: C₁₋₆ alkyl, C₆₋₁₀ aryl, 5- to 10-membered heteroaryl, 3- to 8- membered heterocyclyl, or C₃₋₈ cycloalkyl; each R₂ₐ is the same or different and is independently selected from H, CN, OH, carboxyl, or the following groups which are unsubstituted or optionally substituted with one, two or more R₂ₐ₁: C₁₋₆ alkyl, or R₂₁-C(=O)-(CH₂)ₘ-; or two R₂ₐ together with the carbon atom to which they are attached form C₃₋₆ cycloalkyl; each R₂ₐ₁ is the same or different and is independently selected from H, deuterium, halogen, CN, carboxyl, C₁₋₆ alkyl, C₁₋₆ alkoxy, C₃₋₆ cycloalkylamino, 3- to 6-membered N-containing heterocyclyl, or R₂₂-C(=O)-; each R₂₁ and R₂₂ are the same or different and are each independently selected from H, OH, amino, methyl, methoxy, or cyclopropyl; m is selected from 0, 1 or 2.

According to some embodiments, R₂ is selected from the following groups which are unsubstituted or optionally substituted with one, two or more R₂ₐ: C₁₋₆ alkyl, tetrahydropyrrolyl, piperidinyl, phenyl, cyclohexyl, cyclobutyl or tetrahydropyranyl; each R₂ₐ is the same or different and is independently selected from H, CN, OH, carboxyl, or the following groups which are unsubstituted or optionally substituted with one, two or more R₂ₐ₁: C₁₋₆ alkyl, or R₂₁-C(=O)-(CH₂)ₘ-; or two R₂ₐ together with the carbon atom to which they are attached form C₃₋₆ cycloalkyl; each R₂ₐ₁ is the same or different and is independently selected from H, deuterium, halogen, CN, carboxyl, C₁₋₆ alkyl, C₁₋₆ alkoxy, or R₂₂-C(=O)-; each R₂₁ and R₂₂ are the same or different and are each independently selected from H, OH, amino, methyl, methoxy, cyclopropyl, cyclopropylamino, or tetrahydropyrrolyl; m is selected from 0, 1 or 2.

According to some embodiments, R₂ is selected from the following groups which are unsubstituted or optionally substituted with one, two or more R₂ₐ: C₁₋₆ alkyl, tetrahydropyrrolyl, piperidinyl, phenyl, cyclohexyl, cyclobutyl or tetrahydropyranyl; each R₂ₐ is the same or different and is independently selected from H, OH, carboxyl, methyl, 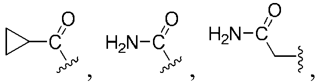 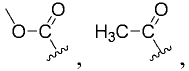 NC-CH₂-, 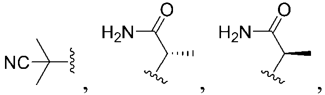 HOOC-CH₂-, HOOC-CH₂-CH₂-, 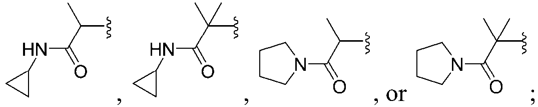 or two R₂ₐ together with the carbon atom to which they are attached form C₃₋₆ cycloalkyl.

According to some embodiments, R₂ is selected from 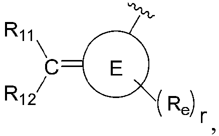 wherein ring E is selected from C₄₋₇ cycloalkyl, and r is selected from integers of 0 to 6.

According to some embodiments, R₁₁ and R₁₂ are the same or different and are each independently selected from H, or halogen (e.g. F, Cl).

According to some embodiments, each Rₑ is the same or different and is independently selected from H or OH.

According to some embodiments, R₂ is selected from 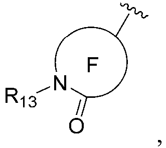 wherein ring F is selected from 4- to 7-membered heterocyclic ring.

According to some embodiments, R₂ is selected from

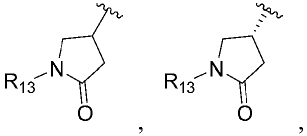

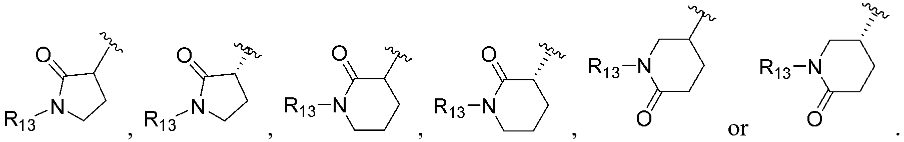

According to some embodiments, R₁₃ is selected from H, or the following groups which are unsubstituted or optionally substituted with one, two or more R₁₃ₐ: C₁₋₃ alkyl, C₃₋₆ cycloalkyl, R_{13b}-C(=O)-(CH₂)ₛ-; R_{13b} is selected from C₁₋₃ alkyl, C₃₋₆ cycloalkyl or amino; each R₁₃ₐ is the same or different and is independently selected from H, OH, halogen, cyano, or C₁₋₃ alkyl; and s is selected from 0, 1, 2 or 3.

According to some embodiments, R₁₃ is selected from hydroxy-C₁₋₆ alkyl-, or NH₂-C(=O)-(CH₂)ₛ-, and s is selected from 0, 1, 2 or 3.

According to some embodiments, R₁₃ is selected from HOCH₂CH₂-, 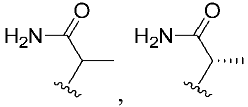 or

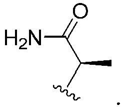

According to some embodiments, R₂ is selected from 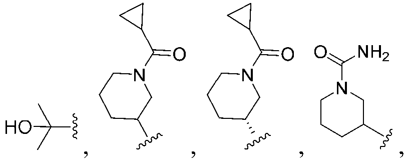 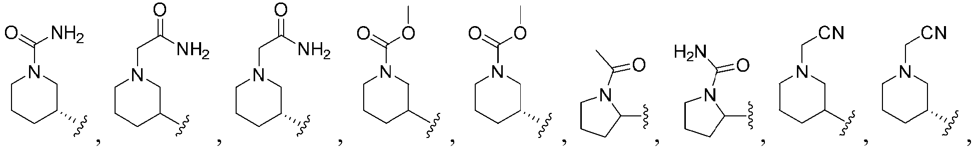 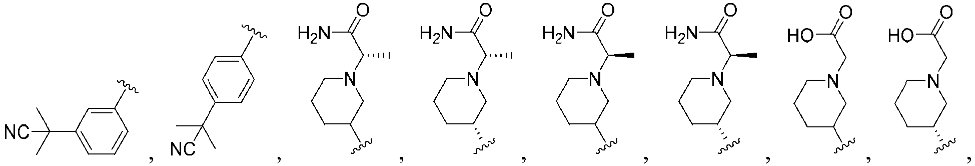 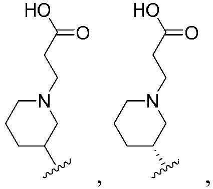 HOOC-CH₂-,

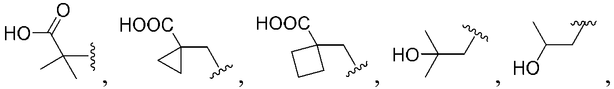

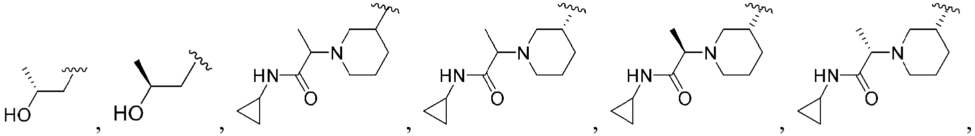

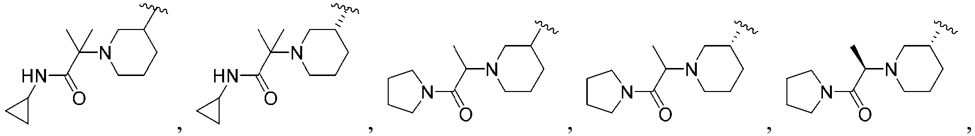

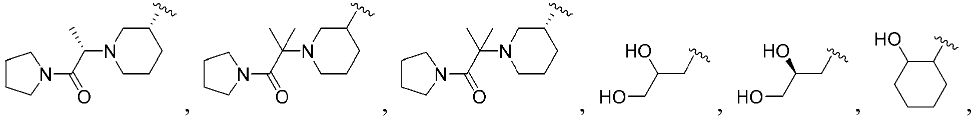

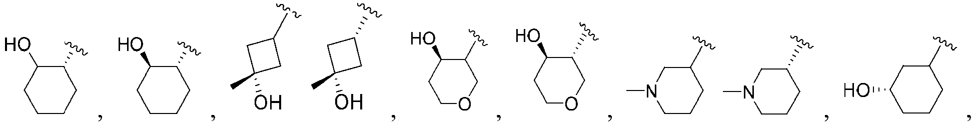

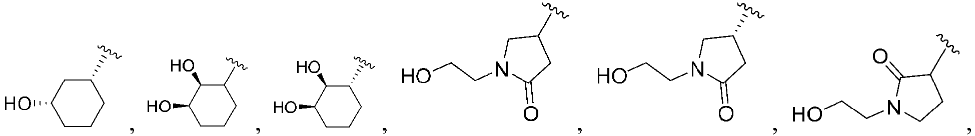

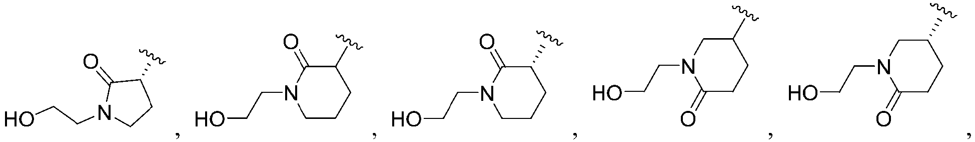

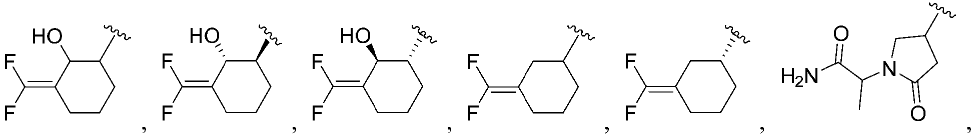

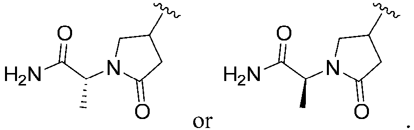

According to some embodiments, the compound represented by formula (I) is selected from the following structures: 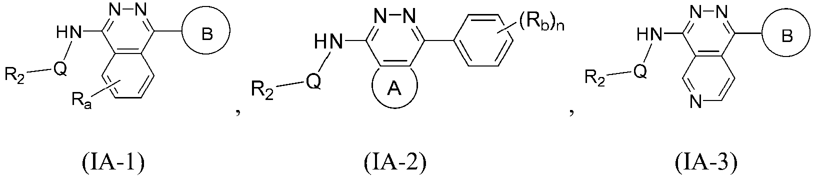 wherein ring A, ring B, Q, R₂, Rₐ, and R_{b} are as defined herein, and n is selected from 0, 1, 2, 3, 4 or 5.

According to some embodiments, the compound represented by formula (I) has the following structures: 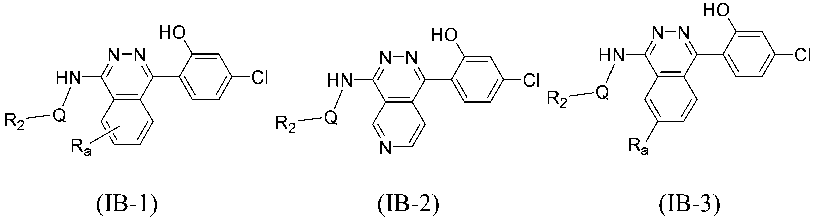 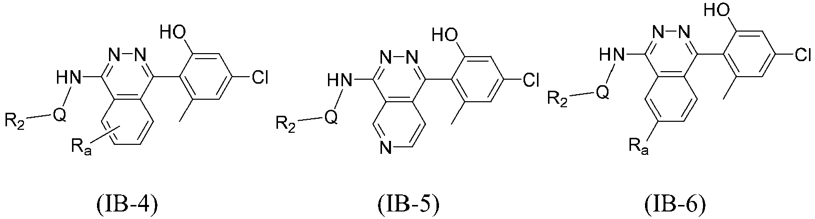 wherein Q, R₂, Rₐ are as defined herein.

According to some embodiments, the compound represented by formula (I) has the following structures: 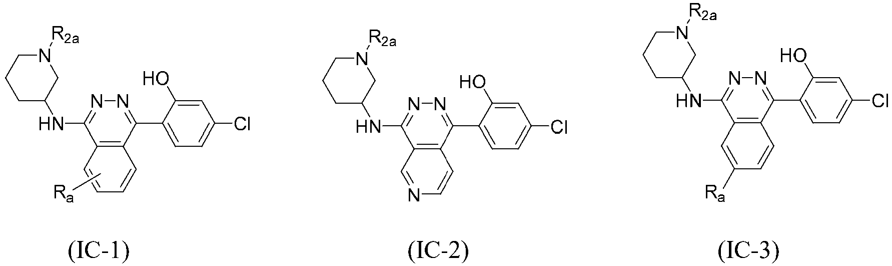 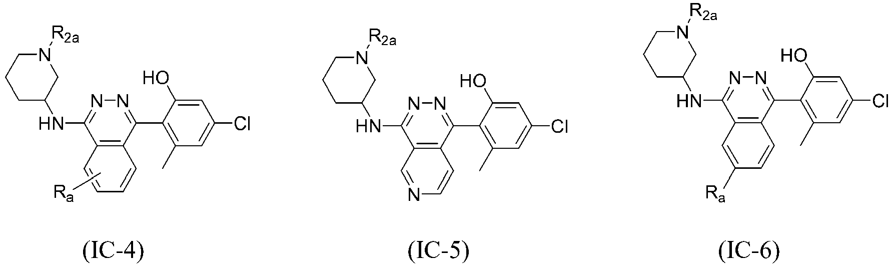 wherein R₂ₐ, Rₐ are as defined herein.

According to some embodiments, the compound represented by formula (I) has the following structures: 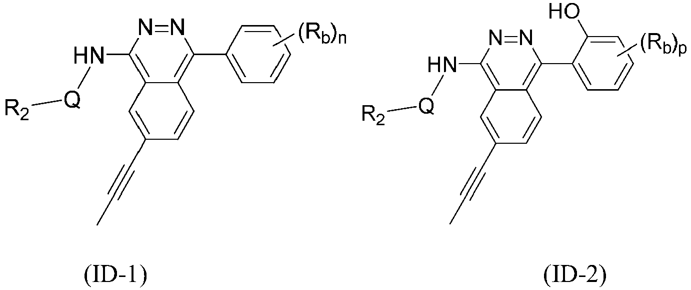 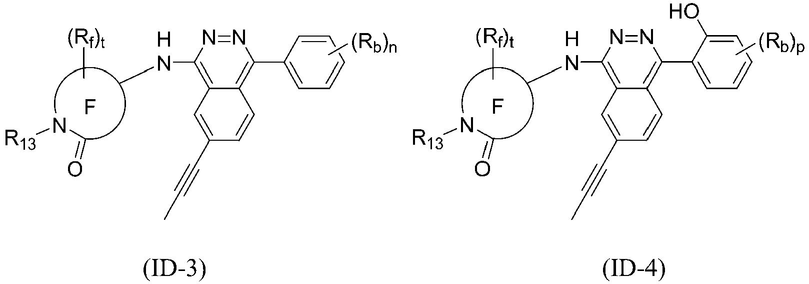 wherein ring F, Q, R₂, R₁, R₁₃, R_{f} and n are each independently as defined herein, p is selected from 0, 1, 2, 3 or 4, and t is selected from 0, 1, 2, 3 or 4.

According to some embodiments, the compound represented by formula (I) is selected from the following structures: 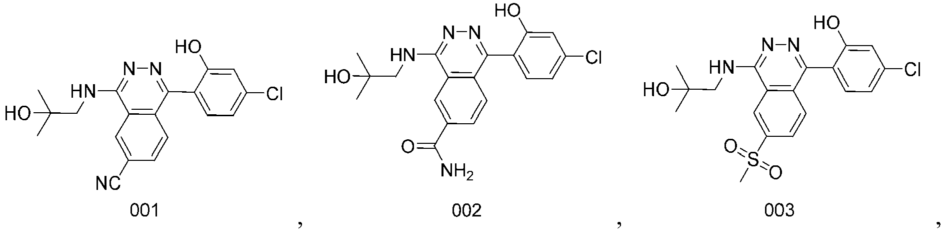 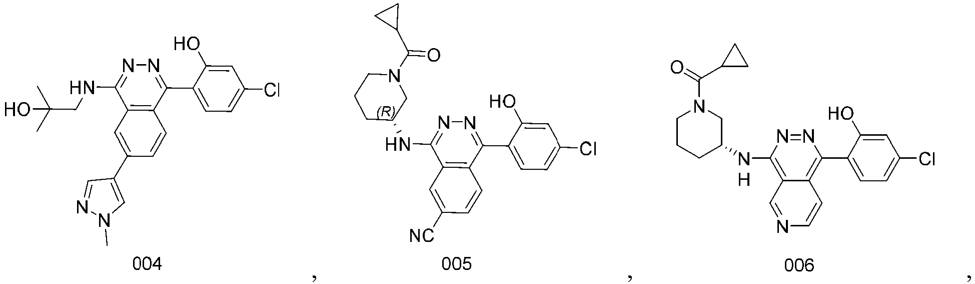 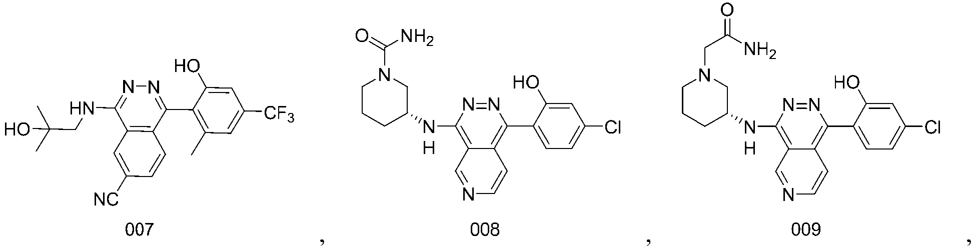 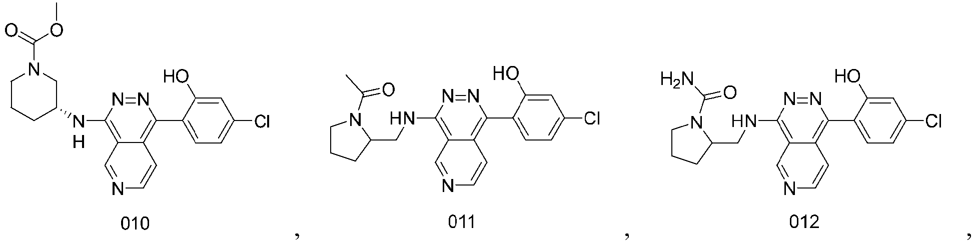 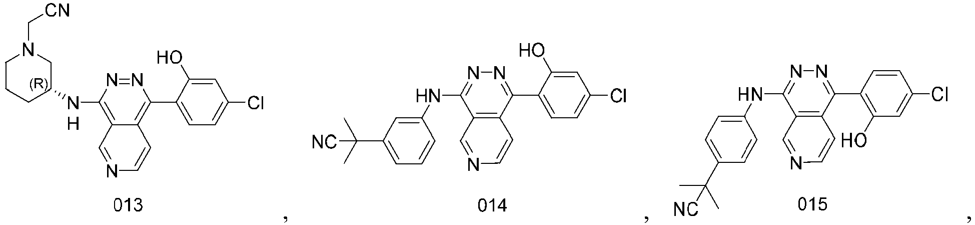 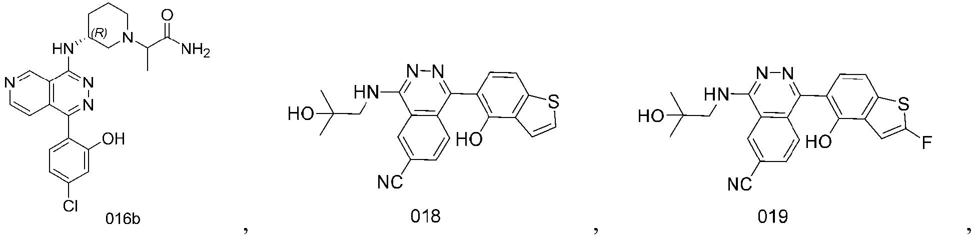 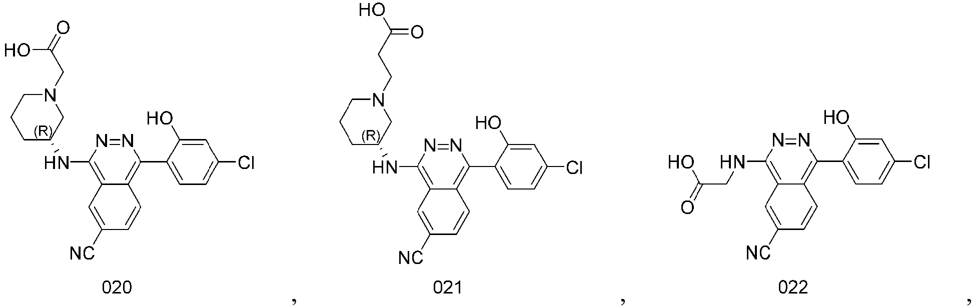 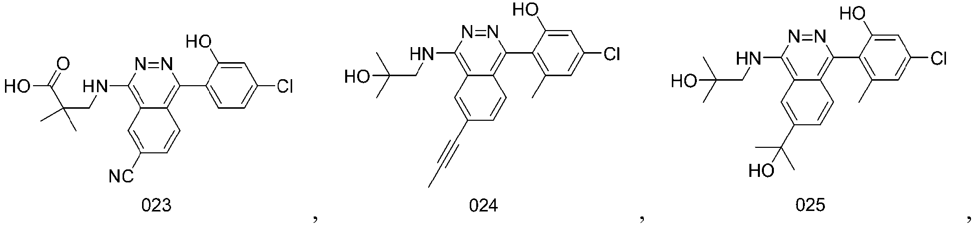 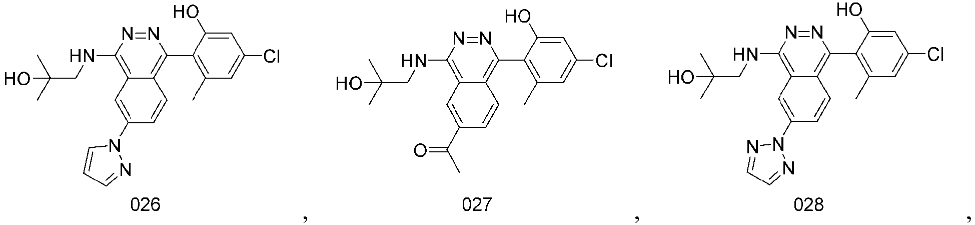 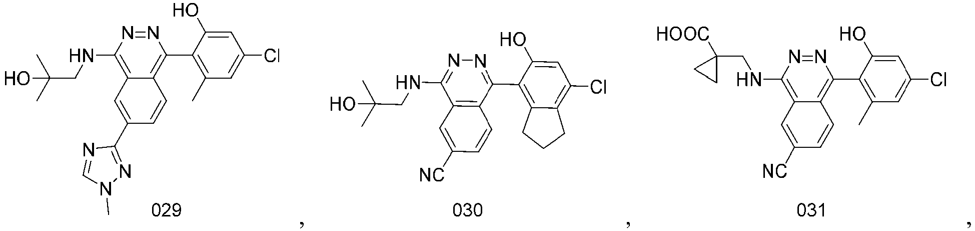 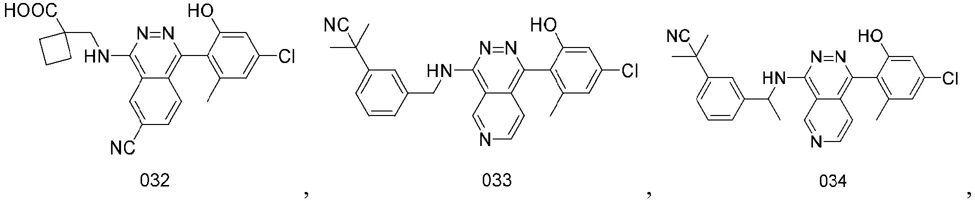 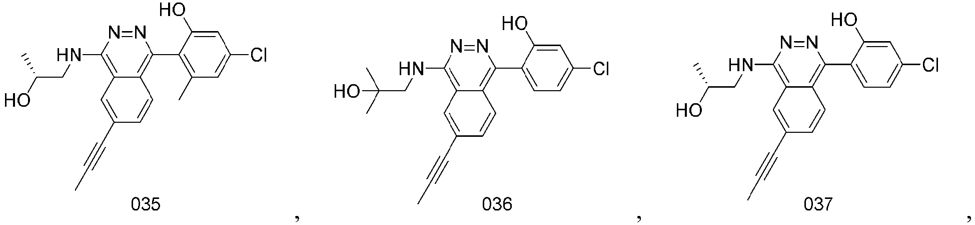 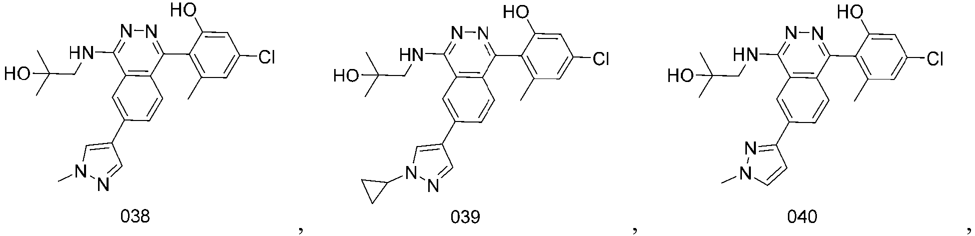 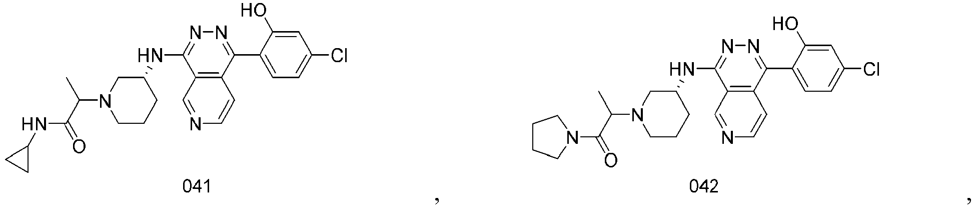 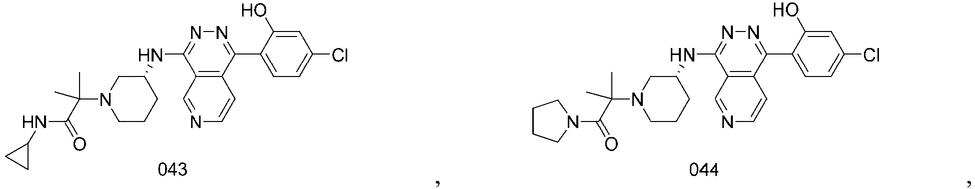 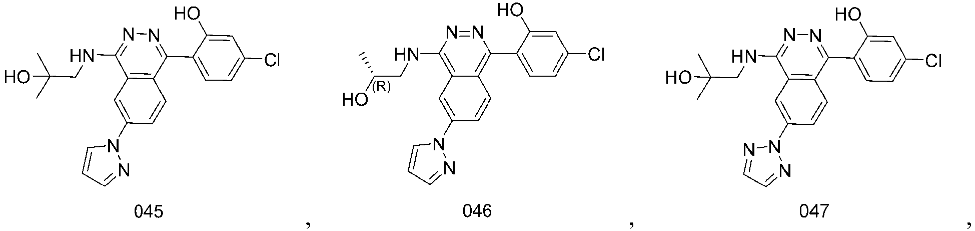 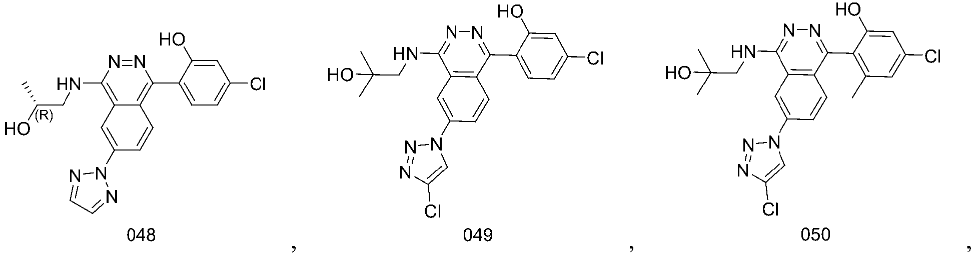 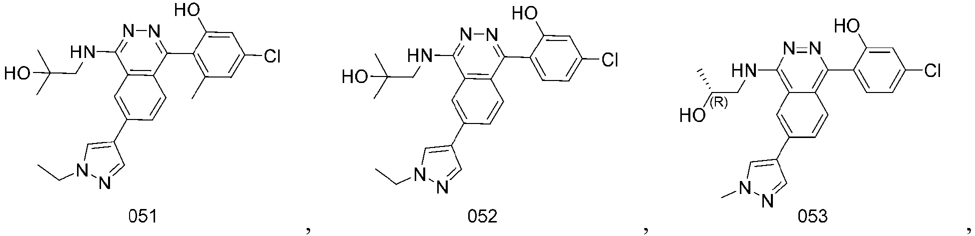 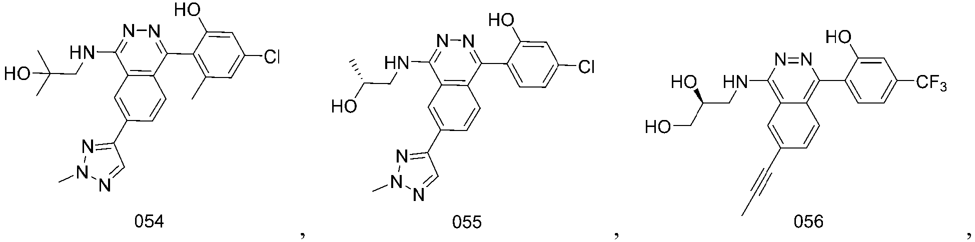 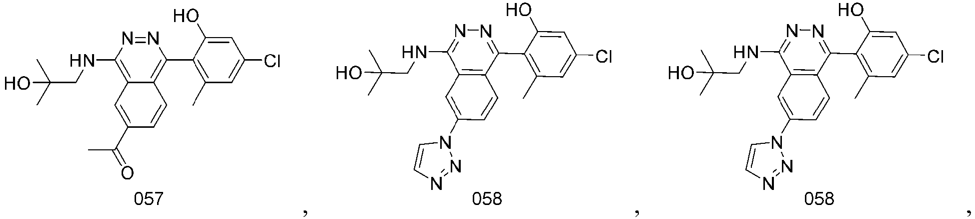 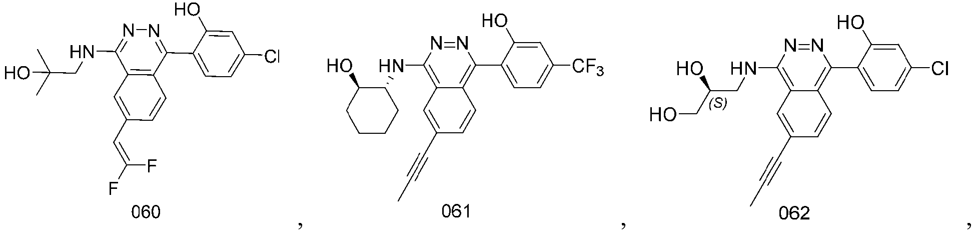 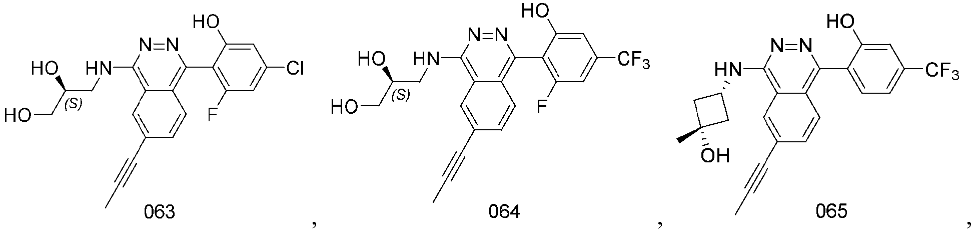 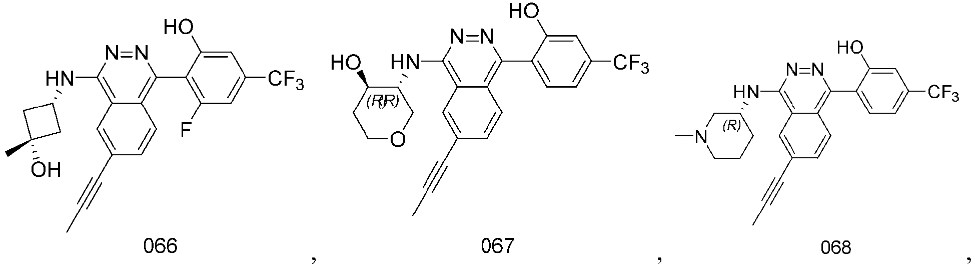 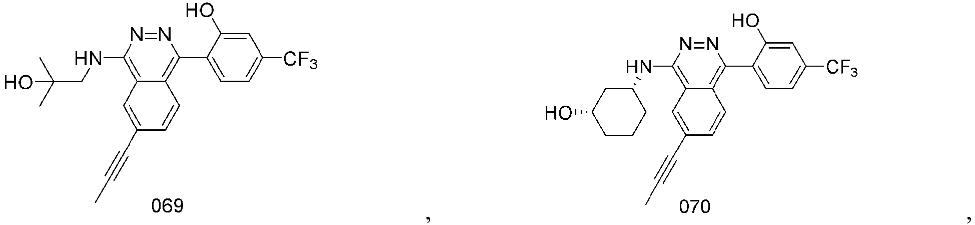 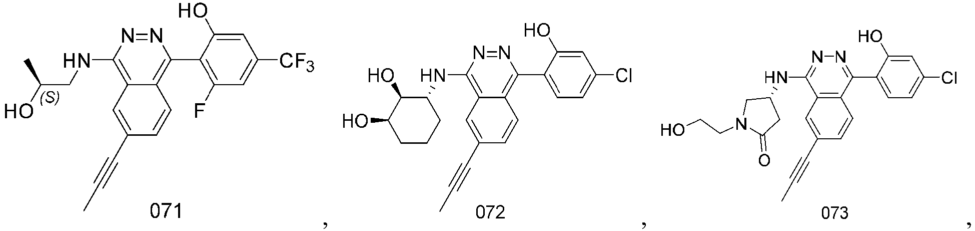 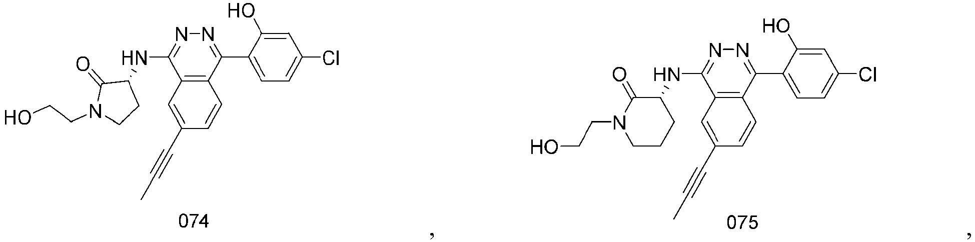 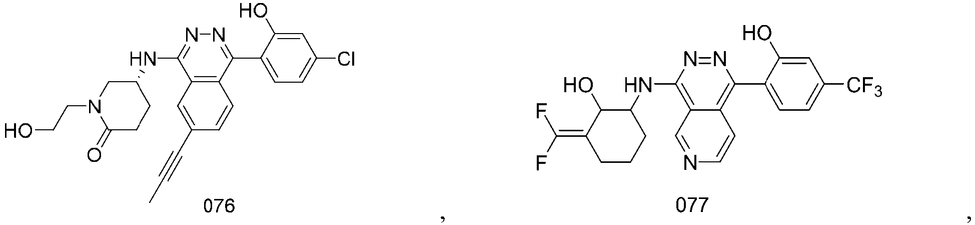 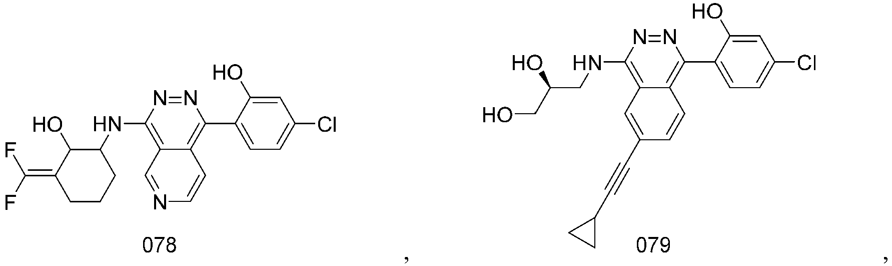 or

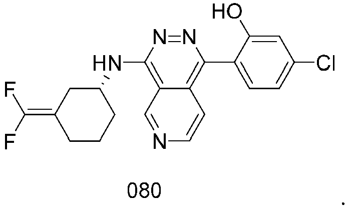

According to some embodiments, the compound represented by formula (I) is selected from the following structures: 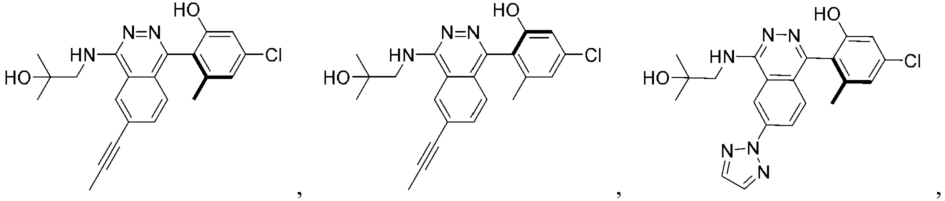 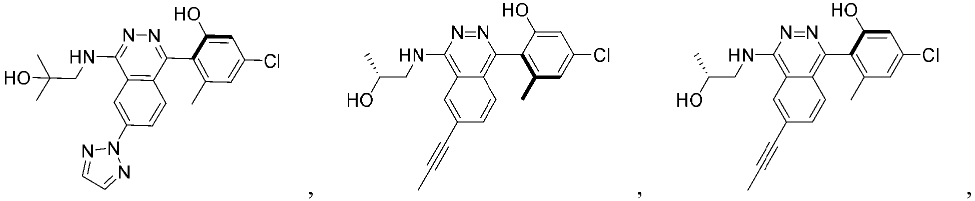 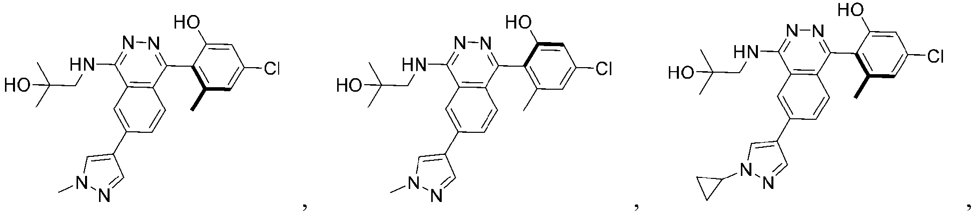 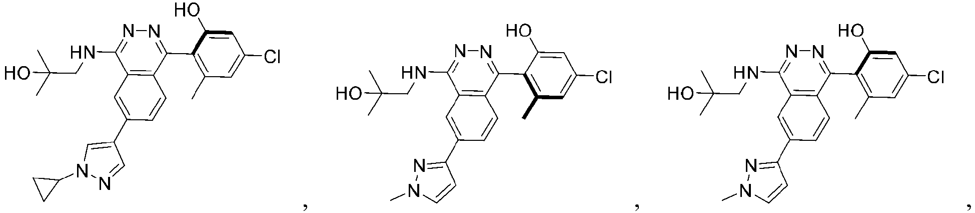 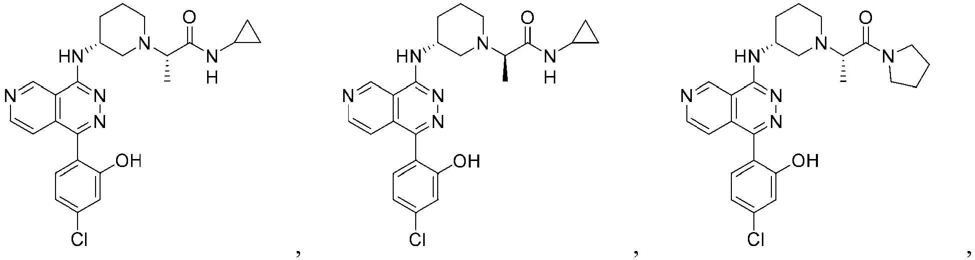 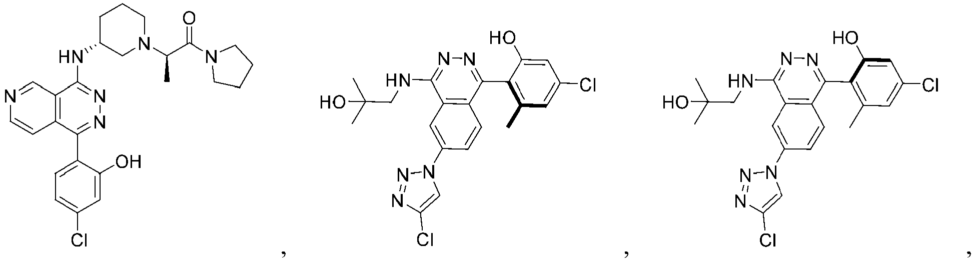 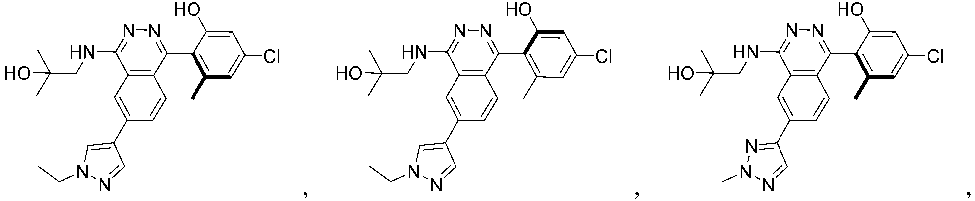 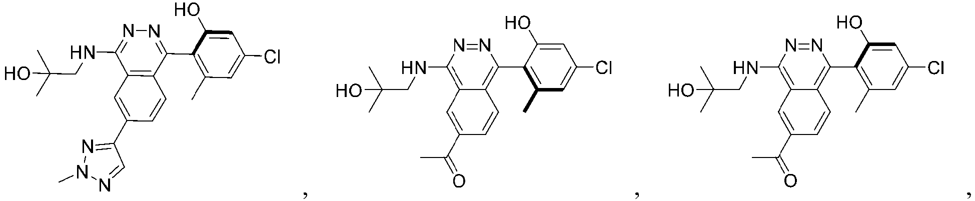 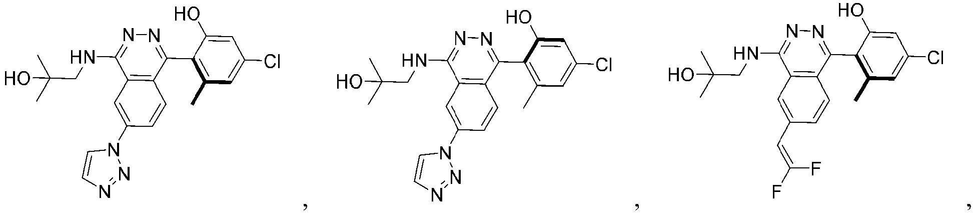 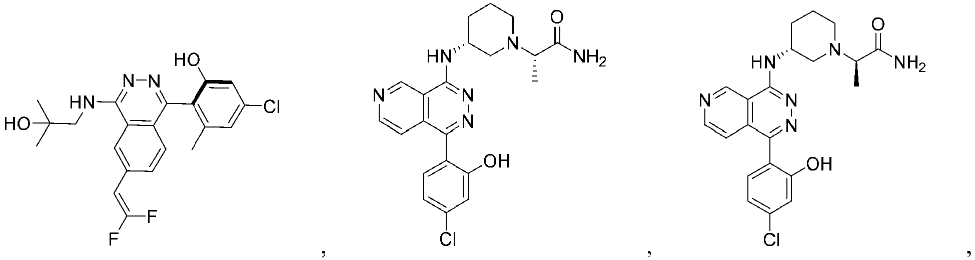 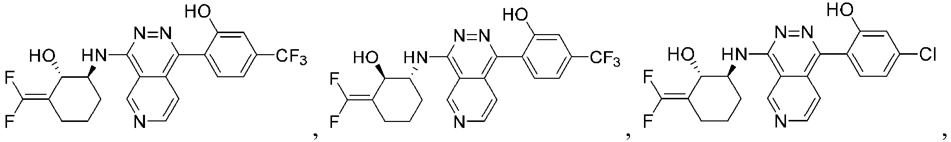 or

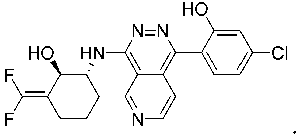

The present invention further provides a method for preparing the compound represented by formula (I), which comprises the following steps: 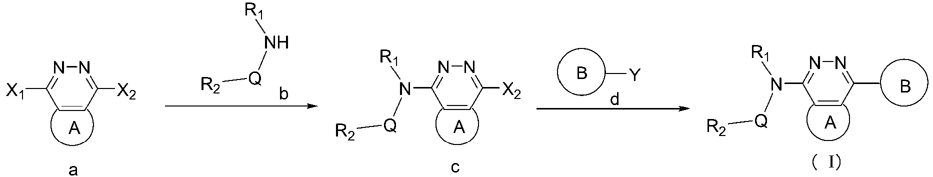
(1) allowing compound a to react with compound b to give compound c; and
(2) allowing compound c to react with compound d to give the compound represented by formula (I).

Ring A, ring B, R₁, R₂ and Q are each independently as defined herein; X₁ and X₂ are the same or different and are each independently selected from leaving groups, such as halogen, such as F, Cl, Br, or I; Y is selected from leaving groups, such as halogen, such as F, Cl, Br, I; or borono ( 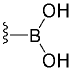 ).

The present invention further provides a pharmaceutical composition comprising a therapeutically effective amount of at least one of a compound represented by formula (I), a racemate, a stereoisomer, a tautomer, an isotope-labeled counterpart, a solvate, a polymorph, a pharmaceutically acceptable salt thereof or a prodrug thereof.

According to embodiments of the present invention, the pharmaceutical composition further comprises one or more pharmaceutically acceptable excipients.

According to embodiments of the present invention, the pharmaceutical composition may further comprise one or more additional therapeutic agents.

The present invention further provides a method for treating NLRP3-mediated diseases, which comprises administering to a patient a prophylactically or therapeutically effective amount of at least one of a compound represented by formula (I), a racemate, a stereoisomer, a tautomer, an isotope-labeled counterpart, a solvate, a polymorph, a pharmaceutically acceptable salt thereof or a prodrug thereof.

The present invention further provides a method for treating NLRP3-mediated diseases, which comprises administering to a patient a prophylactically or therapeutically effective amount of the aforementioned pharmaceutical composition. The NLRP3-mediated disease is selected from: autoinflammatory febrile syndromes such as cryopyrin-associated periodic syndrome (CAPS), sickle cell disease, systemic lupus erythematosus (SLE), chronic liver disease, nonalcoholic steatohepatitis (NASH), gout, gouty arthritis, pericarditis, type I and type II diabetes and related complications (e.g. nephropathy, retinopathy), neuroinflammation-related disorders (e.g. multiple sclerosis, brain infection, acute injury, neurodegenerative diseases, Alzheimer's disease), atherosclerosis and cardiovascular risk (e.g. hypertension), obesity, hidradenitis suppurativa, wound healing and scarring, and cancer (e.g. carcinoma of large intestine, lung cancer, myeloproliferative neoplasms, leukemia, myelofibrosis).

In some embodiment, the patient includes a mammal, preferably a human.

The present invention further provides at least one of a compound represented by formula (I), a racemate, a stereoisomer, a tautomer, an isotope-labeled counterpart, a solvate, a polymorph, a pharmaceutically acceptable salt thereof or a prodrug thereof, or a pharmaceutical composition thereof for use in treating NLRP3-mediated diseases.

The present invention further provides use of at least one of a compound represented by formula (I), a racemate, a stereoisomer, a tautomer, an isotope-labeled counterpart, a solvate, a polymorph, a pharmaceutically acceptable salt thereof or a prodrug thereof in the preparation of a drug.

According to embodiments of the present invention, the use may be use in the preparation of a drug for the treatment of NLRP3-mediated conditions and/or diseases, such as use in the preparation of an NLRP3 inhibitor drug.

According to an embodiment of the present invention, the diseases are, for example, autoinflammatory febrile syndromes such as cryopyrin-associated periodic syndrome (CAPS), sickle cell disease, systemic lupus erythematosus (SLE), chronic liver disease, nonalcoholic steatohepatitis (NASH), gout, gouty arthritis, pericarditis, type I and type II diabetes and related complications (e.g. nephropathy, retinopathy), neuroinflammation-related disorders (e.g. multiple sclerosis, brain infection, acute injury, neurodegenerative diseases, Alzheimer's disease), atherosclerosis and cardiovascular risk (e.g. hypertension), obesity, hidradenitis suppurativa, wound healing and scarring, and cancer (e.g. carcinoma of large intestine, lung cancer, myeloproliferative neoplasms, leukemia, myelofibrosis).

### Beneficial Effects

The compound of the present invention has a good inhibitory effect on NLRP3 inflammasome, and the compound obtained through structural optimization has excellent in vivo activity, good metabolic properties and safety. The compound can be used alone or in combination with other drugs to adjust an NLRP3 protein, so as to treat NLRP3-mediated conditions and/or diseases, and prepare a drug for preventing or treating such conditions or diseases.

### BRIEF DESCRIPTION OF THE DRAWINGS

FIG. 1: Schematic diagram of comparison of experimental results of rat ankle circumference measurement in an MSU-induced rat acute gouty arthritis model.
FIG. 2: Schematic diagram of comparison of detection results of inflammatory factors in an MSU-induced rat air pouch model.
FIG. 3: Schematic diagram of comparison of statistical results of H&E staining for detecting the thickness of the pericardial wall layer in a pericarditis model.
FIG. 4: Schematic diagram of comparison of ELISA results for detecting IL-1β production in pericardial effusion.
FIG. 5: Schematic diagram of comparison of body weight measurement results in a mouse NASH model experiment (Sem: semaglutide).
FIG. 6: Schematic diagram of comparison of mouse food intake measurement results in a mouse NASH model experiment (Sem: semaglutide).
FIG. 7: Schematic diagram of comparison of detection results of mouse serum ALT, AST, LDL, and HDL in a mouse NASH model experiment (Sem: semaglutide).

### Definition and Explanation of Terms

Unless otherwise indicated, the definitions of groups and terms recorded in the specification and claims of the present application, including their definitions as examples, exemplary definitions, preferred definitions, definitions recorded in tables, definitions of specific compounds in examples, etc., may be arbitrarily combined and associated with each other. Such combined and associated group definitions and compound structures should be understood to be within the scope of the specification and/or claims of the present application.

Unless otherwise stated, the numerical ranges recited in the specification and claims are equivalent to reciting at least each specific integer value therein. For example, the numerical range "1-14" is equivalent to recording each integer value in the numerical range "1-14", namely 1, 2, 3, 4, 5, 6, 7, 8, 9, 10, 11, 12, 13, 14.

The term "optional" (or "optionally") in the general formula definitions of the present application means a situation of being substituted with zero, one or more substituents, for example "optionally substituted with one, two or more R" means that it may not be substituted with R (unsubstituted) or may be selectively substituted with one, two or more R.

### "More" means three or more.

The term "C₁₋₁₂ alkyl" is understood to mean linear and branched alkyl groups having 1 to 12 carbon atoms, "C₁₋₈ alkyl" means linear and branched alkyl groups having 1, 2, 3, 4, 5, 6, 7, or 8 carbon atoms, and "C₁₋₆ alkyl" means linear and branched alkyl groups having 1, 2, 3, 4, 5, or 6 carbon atoms. The alkyl is, for example, methyl, ethyl, propyl, butyl, amyl, hexyl, isopropyl, isobutyl, sec-butyl, tert-butyl, isoamyl, 2-methylbutyl, 1-methylbutyl, 1-ethylpropyl, 1,2-dimethylpropyl, neoamyl, 1,1-dimethylpropyl, 4-methylamyl, 3-methylamyl, 2-methylamyl, 1-methylamyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or isomers thereof.

"C₂₋₁₂ alkenyl" is understood to mean preferably straight or branched monovalent hydrocarbon groups containing one or more double bonds and having 2, 3, 4, 5, 6, 7, 8, 9, 10, 11 or 12 carbon atoms, more preferably "C₂₋₈ alkenyl". "C₂₋₈ alkenyl" is understood to mean preferably straight or branched monovalent hydrocarbon groups containing one or more double bonds and having 2, 3, 4, 5, 6, 7 or 8 carbon atoms, for example, having 2, 3, 4, 5 or 6 carbon atoms (i.e. C₂₋₆ alkenyl), having 2 or 3 carbon atoms (i.e. C₂₋₃ alkenyl). It will be appreciated that where the alkenyl comprises more than one double bonds, the double bonds may be separated from each other or conjugated. The alkenyl is, for example, vinyl, allyl, (E)-2-methylvinyl, (Z)-2-methylvinyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)- pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methyl but-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, or 1-isopropylvinyl.

The "C₂₋₁₂ alkynyl" should be construed to represent preferably linear or branched monovalent hydrocarbon groups comprising one or more triple bonds and having 2, 3, 4, 5, 6, 7, 8, 9, 10, 11 or 12 carbon atoms, for example, having 2, 3, 4, 5, 6, 7, or 8 carbon atoms (i.e. "C₂₋₈ alkynyl"), having 2, 3, 4, 5, or 6 carbon atoms (i.e. "C₂₋₆ alkynyl"), or having 2 or 3 carbon atoms (i.e. "C₂₋₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "C₃₋₁₂ cycloalkyl" should be understood to mean saturated monovalent monocyclic, bicyclic (such as fused, bridged, or spiro) hydrocarbon ring or tricyclic alkanes having 3 to 12 carbon atoms, preferably "C₃₋₁₀ cycloalkyl", and more preferably "C₃₋₈ cycloalkyl". The term "C₃₋₁₂ cycloalkyl" should be understood to mean a saturated monovalent monocyclic, bicyclic (such as bridged or spiro) hydrocarbon ring or tricyclic alkane having 3, 4, 5, 6, 7, 8, 9, 10, 11 or 12 carbon atoms. The C₃₋₁₂ cycloalkyl may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group, such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonanyl, 2,6-diazaspiro[3,4]octanyl, or a tricyclic hydrocarbon group, such as adamantyl.

The term "C₃₋₁₂ cycloalkenyl" should be understood to mean a monovalent monocyclic, bicyclic (such as fused, bridged, or spiro) or tricyclic alkene containing a carbon-carbon double bond, and having 3 to 12 carbon atoms, preferably "C₃₋₁₀ cycloalkenyl", and more preferably "C₃₋₈ cycloalkenyl", which may have 3, 4, 5, 6, 7, 8, 9, 10, 11 or 12 carbon atoms. The C₃₋₁₂ cycloalkenyl may be a monocyclic hydrocarbon group, such as cyclopropenyl, cyclobutenyl, cyclopentenyl, cyclohexenyl, cycloheptenyl, cyclooctenyl, cyclononenyl or cyclodecenyl, or a bicyclic hydrocarbon group such as spiro[2.5]oct-5-enyl, spiro[3.5]non-6-enyl, or spiro[4.5]dec-7-enyl.

The term "C₆₋₁₄ aryl" should be understood to preferably mean a monovalent aromatic or partially aromatic monocyclic, bicyclic or tricyclic hydrocarbon ring having 6 to 14 carbon atoms, which may be a single aromatic ring or a fused polyaromatic ring, preferably "C₆₋₁₀ aryl". The term "C₆₋₁₄ aryl" should be understood as preferably representing a monovalent aromatic or partially aromatic monocyclic, bicyclic or tricyclic hydrocarbon ring ("C₆₋₁₄ aryl") having 6, 7, 8, 9, 10, 11, 12, 13, or 14 carbon atoms, in particular a ring having 6 carbon atoms ("C₆ aryl"), such as a phenyl; or biphenyl, or a ring having 9 carbon atoms ("C₉ aryl"), such as indanyl or indenyl, or a ring having 10 carbon atoms ("C₁₀ aryl"), such as tetrahydronaphthyl, dihydronaphthyl, or naphthyl, or a ring having 13 carbon atoms ("C₁₃ aryl"), such as fluoreny, or a ring having 14 carbon atoms ("C₁₄ aryl"), such as anthracenyl. When the C₆₋₂₀ aryl is substituted, it can be monosubstituted or polysubstituted. Furthermore, there is no limitation on the substitution site, and for example, substitution may be at the ortho, para or meta position.

The term "5- to 14-membered heteroaryl" should be understood to include monovalent monocyclic, bicyclic (such as fused, bridged, spiro) or tricyclic aromatic ring systems which have 5 to 14 ring atoms and comprise 1 to 5 heteroatoms independently selected from N, O and S, for example "5- to 10-membered heteroaryl". The term "5- to 14-membered heteroaryl" should be understood to include monovalent monocyclic, bicyclic or tricyclic aromatic ring systems, which have 5, 6, 7, 8, 9, 10, 11, 12, 13 or 14 ring atoms, in particular 5 or 6 or 9 or 10 carbon atoms, and comprise 1 to 5, preferably 1 to 3 heteroatoms that are each independently selected from N, O and S, and which, in addition, in each case may be benzo-fused. "Heteroaryl" also means a group in which a heteroaromatic ring is fused with one or more aryl, alicyclic, or heterocyclic rings, wherein the radical or site of attachment is on the heteroaromatic ring. Non-limiting examples include 1-, 2-, 3-, 5-, 6-, 7- or 8-indolizinyl, 1-, 3-, 4-, 5-, 6-, or 7-isoindolyl, 2-, 3-, 4-, 5-, 6- or 7-indolyl, 2-, 3-, 4-, 5-, 6-, or 7-indazolyl, 2-, 4-, 5-, 6-, 7- or 8-purinyl, 1-, 2-, 3-, 4-, 6-, 7-, 8- or 9-quinolizinyl, 2-, 3-, 4-, 5-, 6-, 7- or 8-quinolinyl, 1-, 3-, 4-, 5-, 6-, 7- or 8-isoquinolinyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6- or 7-pteridinyl, 1-, 2-, 3-, 4-, 5-, 6-, 7- or 8-4aH carbazolyl, 1-, 2-, 3-, 4-, 5-, 6-, 7- or 8-carbazolylcarbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-pyridyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl, 2-, 3-, 4-, 5-, 6- or 1-, 3-, 4-, 5-, 6-, 7-, 8-, 9- or 10-benzoisoquinolyl, 2-, 3-, 4- or thieno[2,3-b]furanyl, 2-, 3-, 5-, 6-, 7-, 8-, 9-, 10- or 11-7H-pyrazino[2,3-c]carbazolyl, 2-, 3-, 5-, 6- or 7-2H-furo[3,2-b]pyranyl, 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d]thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9 -furo[3,4-c]cinnolinyl, 1-, 2-, 3-, 4-, 5-, 6-, 8-, 9-, 10 or 11-4H-pyrido[2,3-c]carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4-, 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoxazinyl, 1-, 2-, 3-, 5-, 6-, 7-, 8-, 9-, 10- or 11-4H-pyrrolo[1,2-b][2]benzazapinyl. Typical fused heteroaryl groups include, but are not limited to, 2-, 3-, 4-, 5-, 6-, 7- or 8-quinolinyl, 1-, 3-, 4-, 5-, 6-, 7- or 8-isoquinolinyl, 2-, 3-, 4-, 5-, 6- or 7-indolyl, 2- 3-, 4-, 5-, 6- or 7-benzo[b]thienyl, 2-, 4-, 5-, 6- or 7-benzoxazolyl, 2-, 4-, 5-, 6- or 7-benzimidazolyl and 2-, 4-, 5-, 6- or 7-benzothiazolyl. When the 5-to 14-membered heteroaryl is linked to other groups to constitute the compound of the present invention, either the carbon atom on the 5- to 14-membered heteroaryl ring can be linked to other groups, or the heteroatom on the 5- to 14-membered heteroaryl ring can be linked to other groups. When the 5- to 14-membered heteroaryl is substituted, it can be monosubstituted or polysubstituted. Moreover, there is no restriction on the substitution sites, for example, hydrogen linked to a carbon atom on a heteroaryl ring may be substituted, or hydrogen linked to a heteroatom on a heteroaryl ring may be substituted.

Unless otherwise defined, the term "3- to 14-membered heterocyclyl" refers to a saturated or unsaturated non-aromatic ring or ring system, for example, a 4-, 5-, 6- or 7-membered monocyclic ring, a 7-, 8-, 9-, 10-, 11- or 12-membered bicyclic ring (e.g. fused, bridged, spiro) or a 10-, 11-, 12-, 13- or 14-membered tricyclic ring system, and contains at least one, for example 1, 2, 3, 4, 5 or more heteroatoms selected from O, S and N, wherein N and S may also be optionally oxidized to various oxidation states to form nitrogen oxides, -S(O)- or -S(O)₂-. Preferably, the heterocyclyl may be selected from "3- to 10-membered heterocyclyl". The term "3-10 membered heterocyclyl" means a saturated or unsaturated non-aromatic ring or ring system and contains at least one heteroatom selected from O, S and N. The heterocyclyl may be attached to the rest of the molecule via any of the carbon atoms or the nitrogen atom, if present. The heterocyclyl may include a fused or bridged ring and a spirocyclic ring. In particular, the heterocyclyl may include, but is not limited to: a 4-membered ring, such as azetidinyl, oxetanyl; a 5-membered ring, such as tetrahydrofuranyl, dioxolyl, pyrrolidinyl, imidazolidinyl, pyrazolidinyl, pyrrolinyl; or a 6-membered ring, such as tetrahydropyranyl, piperidinyl, morpholinyl, dithianyl, thiomorpholinyl, piperazinyl or trithianyl; or a 7-membered ring, such as diazepanyl. Optionally, the heterocyclyl may be benzo-fused. The heterocyclyl may be bicyclic, such as but not limited to a 5,5-membered ring, such as a hexahydrocyclopenta[c]pyrrol-2(1H)-yl ring, or a 5,6-membered bicyclic ring, such as a hexahydropyrrolo[1,2-a]pyrazin-2(1H)-yl ring. The heterocyclyl may be partially unsaturated, tht is, it may contain one or more double bonds, such as but not limited to dihydrofuranyl, dihydropyranyl, 2,5-dihydro-1H-pyrrolyl, 4H-[1,3,4]thiadiazinyl, 1,2,3,5-tetrahydrooxazolyl or 4H-[1,4]thiazinyl, or it may be benzo-fused, such as but not limited to dihydroisoquinolinyl. When the 3- to 14-membered heterocyclyl is linked to other groups to constitute the compound of the present invention, either the carbon atom on the 3- to 14-membered heterocyclyl can be linked to other groups, or the heterocyclic atom on the 3- to 14-membered heterocyclyl can be linked to other groups. For example, when the 3- to 14-membered heterocyclyl is selected from piperazinyl, the nitrogen atom on the piperazinyl may be linked to other groups. Alternatively, when the 3- to 14-membered heterocyclyl is selected from piperidinyl, the nitrogen atom on the piperidinyl ring and the carbon atom at the para position thereof may be linked to other groups.

The term "spiro ring" refers to a ring system in which two rings share one ring atom.

The term "fused ring" refers to a ring system in which two rings share 2 ring atoms.

The term "bridged ring" refers to a ring system in which two rings share 3 or more ring atoms.

The term "halogen" refers to fluorine, chlorine, bromine and iodine.

"Halo" means being substituted with one or more halogens.

Those skilled in the art will appreciate that the compound represented by formula (I) may exist in the form of various pharmaceutically acceptable salts. If the compounds have basic centers, they can form acid addition salts; if they have acidic centers, they can form base addition salts; if the compounds contain both acidic centers (e.g. carboxyl) and basic centers (e.g. amino), they can also form inner salts.

The compounds of the present invention may be present in the form of solvates, such as hydrates, wherein the compounds of the present invention comprise polar solvents, such as water, methanol or ethanol, as structural elements of the lattice of the compounds. The polar solvent, especially water, may be present in a stoichiometric or non-stoichiometric amount.

Depending on their molecular structure, the compounds of the present invention may be chiral and may therefore exist in various enantiomeric forms. These compounds may therefore exist in racemic or optically active forms. The compounds of the present invention encompass isomers in which the chiral carbons are each in R or S configuration, or mixtures and racemates thereof. The compounds of the present invention or their intermediates can be separated into enantiomeric compounds by chemical or physical methods known to those skilled in the art, or used in synthesis in this form. In the case of racemic amines, diastereomers are prepared from the mixture by reaction with an optically active resolving reagent. Examples of suitable resolving reagents are optically active acids, such as the R and S forms of tartaric acid, diacetyltartaric acid, dibenzoyltartaric acid, mandelic acid, malic acid, lactic acid, appropriate N-protected amino acids (e.g. N-benzoylproline or N-phenylsulfonylproline) or various optically active camphorsulfonic acids. Chromatographic enantiomeric resolution can also be advantageously carried out by means of optically active resolving reagents, such as dinitrobenzoylphenylglycine, cellulose triacetate or other carbohydrate derivatives or chirally derivatized methacrylate polymers immobilized on silica gel. Suitable eluents for this purpose are aqueous or alcoholic solvent mixtures, for example, hexane/isopropanol/acetonitrile.

The corresponding stable isomers can be separated according to known methods, such as by extraction, filtration or column chromatography.

The term "patient" refers to any animal, including mammals, preferably mice, rats, other rodents, rabbits, dogs, cats, pigs, cows, sheep, horses or primates, and most preferably humans.

The term "therapeutically effective amount" refers to that amount of an active compound or drug which causes a biological or medical response sought by a researcher, veterinarian, physician or other clinician in a tissue, system, animal, individual or human, and which comprises one or more of the following: (1) prevention of disease: for example, prevention of a disease, disorder or condition in an individual who is susceptible to the disease, disorder or condition but has not yet experienced or developed the pathology or symptoms of the disease. (2) Inhibition of disease: for example, inhibition of a disease, disorder or condition (i.e., prevention of further progression of pathology and/or symptoms) in an individual undergoing or exhibiting the pathology or symptoms of the disease, disorder or condition. (3) Alleviation of disease: for example, alleviation of a disease, disorder or condition (i.e., reversion of pathology and/or symptoms) in an individual undergoing or exhibiting the pathology or symptoms of the disease, disorder or condition.

### DETAILED DESCRIPTION OF THE INVENTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise stated, the raw materials and reagents used in the following examples are commercially available, or may be prepared in accordance with known methods.

The structures of the compounds are determined by nuclear magnetic resonance (NMR) or/and mass spectrometry (MS). NMR shifts (δ) are given in 10⁻⁶ (ppm). The NMR measurement is performed using a Bruker AVANCE-400 nuclear magnetic resonance spectrometer, with solvents of deuterated dimethyl sulfoxide (DMSO-*d₆*), deuterated chloroform (CDCl₃) and deuterated methanol (CD₃OD), and with an internal standard of tetramethylsilane (TMS).

MS is carried out using Agilent 1200/1290 DAD-6110/6120 Quadrupole MS LC-MS (Manufacturer: Agilent, MS model: 6110/6120 Quadrupole MS). waters ACQuity UPLC-QD/SQD (Manufacturer: waters, MS model: waters ACQuity Qda Detector/waters SQ Detector), THERMO Ultimate 3000- Q Exactive (Manufacturer: THERMO, MSmodel: THERMO Q Exactive)

High performance liquid chromatography (HPLC) analysis is performed using Agilent 1260II HPLC and Waters Acquity UPLC H-Class HPLC.

Chiral HPLC analysis is performed using Waters Acquity UPCC HPLC.

High performance liquid chromatography is performed using Waters MS-triggered Prep-LC with SQD2 detector, Waters MS triggered Prep-LC with Acquity QDA detector, Waters MS-triggered Prep-LC with QDA detector and GILSON Prep LC with UV detector.

Combiflash Rf200 (TELEDYNE ISCO) is used as the CombiFlash flash preparative instrument.

Yantai Huanghai HSGF254 or Qingdao GF254 silica gel plate is used as the silica gel plate for thin layer chromatography, the specification of the silica gel plate for thin layer chromatography (TLC) is 0.15 mm to 0.2 mm, and the specification for thin layer chromatography for separation and purification of products is 0.4 mm to 0.5 mm.

Yantai Huanghai silica gel of 200-300 mesh is generally used as the carrier for silica gel column chromatography.

The average kinase inhibition rate and IC₅₀ value are determined using NovoStar microplate reader (BMG, Germany).

Known starting materials of the present disclosure may be synthesized using or in accordance with methods known in the art, or may be purchased from ABCR GmbH & Co. KG, Acros Organics, Aldrich Chemical Company, Accela ChemBio Inc, Darui Chemicals and others.

Unless otherwise specified in the examples, all reactions can be carried out under an argon atmosphere or a nitrogen atmosphere.

Argon atmosphere or the nitrogen atmosphere means that the reaction flask is connected to an argon or nitrogen balloon with a volume of about 1L.

Hydrogen atmosphere means that the reaction flask is connected to a hydrogen balloon with a volume of about 1L.

The pressurized hydrogenation reaction is performed using a Parr 3916EKX hydrogenator and a Qinglan QL-500 hydrogen generator or an HC2-SS hydrogenator.

Vacuumizing and filling with hydrogen are usually repeatedly operated 3 times prior to the hydrogenation reaction.

CEM Discover-S 908860 microwave reactor is used for microwave reaction.

Unless otherwise specified in the examples, the solution refers to an aqueous solution.

Unless otherwise specified in the examples, the reaction temperature is room temperature (20°C to 30°C).

The reaction progress in the examples is monitored by thin layer chromatography (TLC), the developing agent used in the reaction, the column chromatography eluent system used for purifying the compound and the developing agent system of the thin layer chromatography include: A: dichloromethane/methanol system, and B: n-hexane/ethyl acetate system, the volume ratio of the solvents is adjusted according to the polarity of the compound, and a small amount of alkaline or acidic reagents such as triethylamine and acetic acid can also be added for adjustment.

### Example 1

### 1-(4-chloro-2-hydroxyphenyl)-4-((2-hydroxy-2-methylpropyl)amino)phthalazin-6-carbonitrile (001)

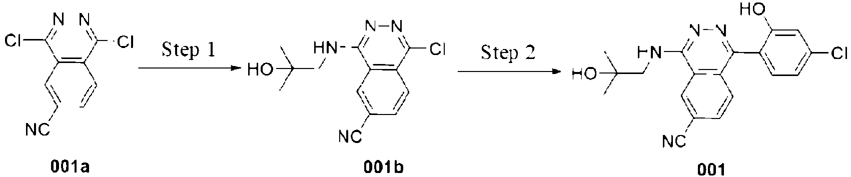

### Step 1 Preparation of 1-chloro-4-((2-hydroxy-2-methylpropyl)amino)phthalazin-6-carbonitrile (001b)

1,4-dichlorophthalazin-6-carbonitrile **001a** (200 mg, 0.89 mmol), 1-amino-2-methyl-2-propanol (159 mg, 1.78 mmol) were dissolved in *N*-methylpyrrolidone (5 mL), and *N,N-*diisopropylethylamine (346 mg, 2.68 mmol) was added thereto. The reaction system was stirred at 100°C for 2 h. After the reaction was completed, the resulting residue was purified by silica gel column chromatography with eluent system A to give the title compound **001b** (125 mg, yield: 50.6%).

MS m/z (ESI): 277.08 (M+1)⁺.

¹H NMR (400 MHz, DMSO-*d₆*) δ = 9.13 (s, 1H), 8.36 - 8.33 (m, 1H), 8.20 - 8.17(m, 1H), 7.70 (t, *J*=4.4 Hz, 1H), 4.76 (s, 1H), 3.61 (d, *J*=6.0 Hz, 2H), 1.19 (s, 6H).

### Step 2 Preparation of 1-(4-chloro-2-hydroxyphenyl)-4-((2-hydroxy-2-methylpropyl)amino)phthalazin-6-carbonitrile (001)

Compound **001b** (125 mg, 0.45 mmol), (4-chloro-2-hydroxyphenyl)boronic acid (116.8 mg, 0.68 mmol), and sodium carbonate (143.6 mg, 1.36 mmol) were dissolved in a mixed solvent of dioxane and water (5 mL, V/V = 10:1), and [1,1'-bis(diphenylphosphino)ferrocene]palladium (II) dichloride (99 mg, 0.14 mmol) was added thereto. The reaction system was stirred at 100°C for 4 h. After the reaction was completed, the mixture was extracted with dichloromethane, and the organic phase was collected, dried over anhydrous sodium sulfate, and concentrated. The crude product was purified by preparative high performance liquid chromatography (Waters MS triggered Prep-LC with Acquity QDA detector, chromatographic column: Welch C18 250*21.2 mm, 10 µm; mobile phase 1: water (containing 0.1% aqueous ammonia); mobile phase 2: acetonitrile; 20-min gradient, gradient ratio: acetonitrile phase 23%-33%, flow rate: 25 mL/min) to give the title compound **001** (16 mg, yield: 9.6%).

MS m/z (ESI): 369.1 (M+1)⁺.

¹H NMR (400 MHz, DMSO-*d₆*) δ = 9.08 (s, 1H), 8.12 (d, *J*=8.4 Hz, 1H), 7.60-7.60(m, 2H), 7.32 (d, *J*=7.6 Hz, 1H), 7.02 (s, 1H), 6.99 (s,1H), 5.03 (s, 1H), 3.67 (d, *J=5.6* Hz, 2H), 1.23 (s, 6H).

### Example 2

### 1-(4-chloro-2-hydroxyphenyl)-4-((2-hydroxy-2-methylpropyl)amino)phthalazin-6-formamide (002)

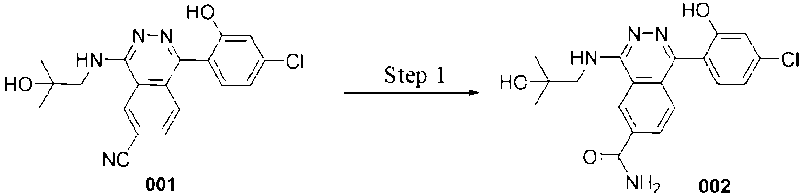

### Step 1 Preparation of 1-(4-chloro-2-hydroxyphenyl)-4-((2-hydroxy-2-methylpropyl)amino)phthalazin-6-formamide (002)

1-(4-chloro-2-hydroxyphenyl)-4-((2-hydroxy-2-methylpropyl)amino)phthalazin-6-carbonitrile **001** (20 mg, 0.05 mmol) was dissolved in ethanol (0.6 mL) and tetrahydrofuran (0.6 mL), to the mixture was added 1 mol/L sodium hydroxide (0.2 mL) solution, and the system was stirred at 25°C for 16 h. After the reaction was completed, the crude product was purified by preparative high performance liquid chromatography (Waters MS triggered Prep-LC with Acquity QDA detector, chromatographic column: Welch C18, 250 × 21.2 mm, 10 µm; mobile phase 1: water (containing 0.1% aqueous ammonia); mobile phase 2: acetonitrile; 15-min gradient, gradient ratio: acetonitrile phase 5%-40%, flow rate: 25 mL/min) to give the title compound **002** (5.26 mg, yield: 25%).

MS m/z (ESI): 387.1 (M+1)⁺.

¹H NMR (400 MHz, DMSO-*d*₆): δ 10.21 (s, 1H), 8.87 (s, 1H), 8.19 (d, *J*=7.2 Hz, 2H), 7.73 (s, 1H), 7.51 (d, *J*=8.4 Hz, 1H), 7.43 (t, *J*=5.2 Hz,1H), 7.32 (d, *J*=8.0 Hz, 1H), 7.01 (d, 8.4 Hz, 1H), 5.30 (s, 1H), 3.65 (d, *J*=7.0 Hz, 2H), 1.23 (s, 6H).

### Example 3

### (R)-1-(4-chloro-2-hydroxyphenyl)-4-((1-(cyclopropanecarbonyl)piperidin-3-yl)amino)phthalazin-6-carbonitrile (005)

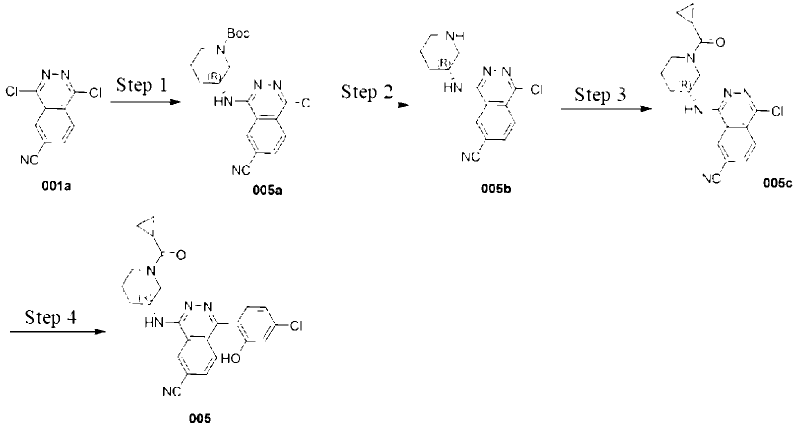

### Step 1 Preparation of tert-butyl (R)-3-((4-chloro-7-cyanophthalazin-1-yl)amino)piperidin-1-carboxylate (005a)

1,4-dichlorophthalazin-6-carbonitrile **001a** (200 mg, 0.89 mmol), tert-butyl (R)-3-aminopiperidin-1 carboxylate (216 mg, 1.07 mmol) were dissolved in N-methylpyrrolidone (5 mL), and *N*,*N*-diisopropylethylamine (346 mg, 2.68 mmol) was added thereto. The reaction system was stirred at 100°C for 2 h. After the reaction was completed, water was added to the reaction system, which was then extracted with ethyl acetate. The organic phase was collected and dried over anhydrous sodium sulfate. The concentrated residue was purified by silica gel column chromatography with eluent system B to give the title compound **005a** (120 mg, yield: 34.7%).

MS m/z (ESI): 388.1 (M+1)⁺.

¹H NMR (400 MHz, DMSO-*d*₆) δ 9.09 (s, 1H), 8.36 (dd, *J*=8.4 Hz, 1.2 Hz, 1H), 8.21 (d, *J*=8.4 Hz, 1H), 7.54 (s, 1H), 2.09-2.05 (m, 1H), 1.91-1.82(m, 1H), 1.67 (s, 1H), 1.52-1.44 (m, 2H), 1.38-1.31 (d, *J*=7.1, 4H), 1.23 (s, 9H).

### Step 2 Preparation of (R)-1-chloro-4-(piperidin-3-ylamino)phthalazin-6-carbonitrile (005b)

Compound **005a** (120 mg, 0.31 mmol) was dissolved in 2 M hydrochloric acid in ethyl acetate (5 mL), and the reaction was stirred at 20°C for 2 h. After the reaction was completed, the reaction mixture was directly concentrated under reduced pressure to give the title compound **005b** (90 mg, yield: 89.9%).

MS m/z (ESI): 288.1 (M+1)⁺.

### Step 3 Preparation of (R)-1-chloro-4-((1-(cyclopropanecarbonyl)piperidin-3-yl)amino)phthalazin-6-carbonitrile (005c)

Compound **005b** (80 mg, 0.21 mmol) was dissolved in dichloromethane (5 mL), and triethylamine (62.44 mg, 0.62 mmol) was added. After the reaction mixture was clarified, cyclopropanecarbonyl chloride (21.5 mg, 0.21 mmol) was added dropwise at 0°C, and the reaction system was stirred at 0°C for 30 min. After the reaction was completed, water was added to the reaction system for dilution, and the system was extracted with dichloromethane. The organic phase was collected, dried over anhydrous sodium sulfate, and concentrated under reduced pressure to give a crude product. The crude product was purified by silica gel column chromatography with eluent system B to give the title compound **005c** (50 mg, yield: 68.3%).

MS m/z (ESI): 356.1 (M+1)⁺.

### Step 4 Preparation of (R)-1-(4-chloro-2-hydroxyphenyl)-4-((1-(cyclopropanecarbonyl)piperidin-3-yl)amino)phthalazin-6-carbonitrile (005)

Compound **005c** (50 mg, 0.14 mmol), 2-hydroxy-4-chlorophenylboronic acid (29.06 mg, 0.17 mmol), and sodium carbonate (44.67 mg, 0.42 mmol) were dissolved in a mixed solvent of 1,4-dioxane and water (2.2 mL, V/V = 10:1), and [1,1'-bis(diphenylphosphino)ferrocene]palladium (II) dichloride (10.2 mg, 0.014 mmol) was added. The reaction system was allowed to react at 100°C for 4 h. After the reaction was completed, the reaction mixture was filtered, the filtrate was collected, and concentrated under reduced pressure to give a crude product. The crude product was purified by preparative high performance liquid chromatography (Waters MS triggered Prep-LC with Acquity QDA detector, chromatographic column: Welch C18, 250*21.2 mm, 10 µm; mobile phase 1: water (containing 0.1% aqueous ammonia); mobile phase 2: acetonitrile; 15-min gradient, gradient ratio: acetonitrile phase 30%-100%, flow rate: 25 mL/min) to give the title compound **005** (15.4 mg, yield: 24.5%).

MS m/z (ESI): 448.1 (M+1)⁺.

¹H NMR (400 MHz, DMSO-*d*₆) δ 10.32 (s, 1H), 9.04 (d, *J*=30.4 Hz, 1H), 8.13 (d, *J*=8.4 Hz, 1H), 7.59 (d, *J*=8.4 Hz, 1H), 7.49 (d, *J*=6.4 Hz, 1H), 7.32 (d, *J*=8.4 Hz,2H), 6.98 (s, 2H), 4.60 (d, *J*=10.4 Hz,1H), 4.38-4.19 (m, 2H), 4.01(d, *J*=12.4 Hz, 1H), 3.43-3.39 (m, 1H), 3.20-3.02 (m, 1H), 2.73-2.67 (m, 1H),2.19-2.13(m,1H),2.04-1.99 (m, 1H), 1.76-1.48(m, 1H), 0.87-0.66 (m, 2H), 0.49-0.39 (m, 2H).

### Example 4

### 4-((2-hydroxy-2-methylpropyl)amino)-1-(2-hydroxy-6-methyl-4-(trifluoromethyl)phenyl)phthalazin-6-carbonitrile (007)

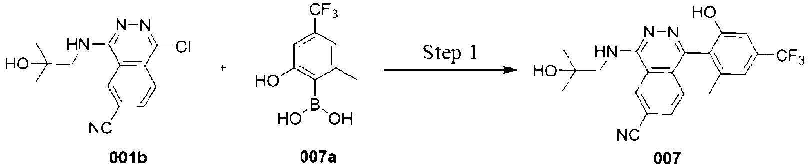

### Step 1 Preparation of 4-((2-hydroxy-2-methylpropyl)amino)-1-(2-hydroxy-6-methyl-4-(trifluoromethyl)phenyl)phthalazin-6-carbonitrile (007)

Compound **007a** (20 mg, 0.087 mmol, prepared by the method disclosed on pages 38-39 of the specification of patent application "US2020361899A1") was dissolved in a mixed solvent of 1,4-dioxane and water (2.2 mL, V/V = 10:1), then compound **001b** (20 mg, 0.072 mmol), sodium carbonate (23 mg, 0.22 mmol) and tetrakis(triphenylphosphine)palladium (8.35 mg, 0.0072 mmol) were added, and the reaction system was allowed to react at 100°C under nitrogen protection for 1 h. After the reaction was completed, the reaction mixture was filtered, the filtrate was collected, and concentrated under reduced pressure to give a crude product. The crude product was purified by preparative high performance liquid chromatography (Waters MS triggered Prep-LC with Acquity QDA detector, chromatographic column: Welch C18, 250*21.2 mm, 10 µm; mobile phase 1: water (containing 0.1% aqueous ammonia); mobile phase 2: acetonitrile; 15-min gradient, gradient ratio: acetonitrile phase 30%-100%, flow rate: 25 mL/min) to give the title compound 007 (7.84 mg, yield: 26%).

MS m/z (ESI): 417.1 (M+1)⁺.

¹H NMR (400 MHz, DMSO-*d*₆) δ 9.10 (s, 1H), 8.09 (d, *J*=8.4 Hz, 1H), 7.58 (t, *J=*5.6 Hz, 1H), 7.39 (d, *J*=8.4 Hz, 1H), 7.14 (s, 1H), 7.05 (s, 1H),5.01 (s, 1H),3.67 (ddd, *J*=44 Hz, 13.2 Hz, 5.6 Hz, 2H), 1.97 (m, 3H), 1.23 (s,6H).

### Example 5

### (R)-2-(3-((1-(4-chloro-2-hydroxyphenyl)pyrido[3,4-d]pyridazin-4-yl)amino)piperidin-1-yl)acetamide (009)

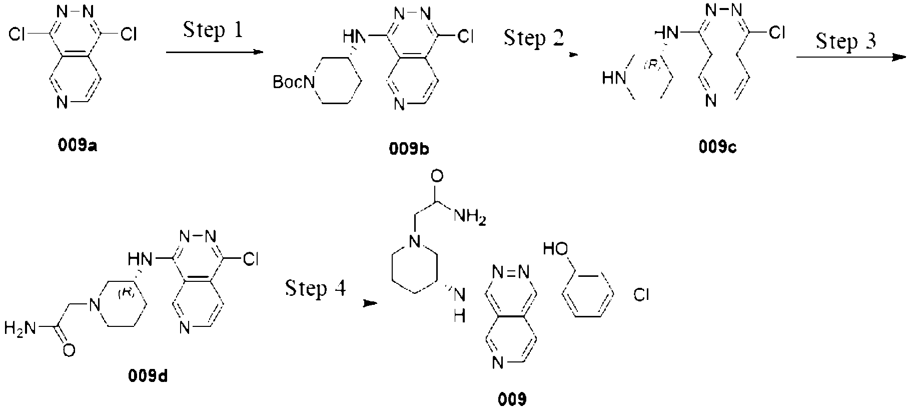

### Step 1 Preparation of tert-butyl (R)-3-((1-chloropyrido[3,4-d]pyridazin-4-yl)amino)piperidin-1-carboxylate (009b)

Compound **009a** (1 g, 5 mmol) was dissolved in *N*-methylpyrrolidone (10 mL), and tert-butyl (*R*)-3-aminopiperidin-1-carboxylate (1 g, 5 mmol) and *N,N*-diisopropylethylamine (1.29 g, 10 mmol) were added. The reaction mixture was allowed to react with stirring at 100°C for 1 h. After the reaction was completed, water was added to dilute the mixture, and the mixture was extracted with dichloromethane and washed with saturated brine. The organic phase was collected and the solvent was removed by rotary evaporation to give a crude product. The crude product was purified by silica gel column chromatography with eluent system B to give the title compound **009b** (1.7 g, yield: 93%).

MS m/z (ESI): 364.1 (M+1)⁺.

### Step 2 Preparation of (R)-1-chloro-N-(piperidin-3-yl)pyrido[3,4-d]pyridazin-4-amine (009c)

Compound **009b** (1.7 g, 4.7 mmol) was dissolved in 2 M hydrochloric acid in ethyl acetate (5 mL). The reaction mixture was allow to react at 25°C for 1 h. After the reaction was completed, the solvent was removed by rotary evaporation to give a crude product. The crude product was purified by preparative high performance liquid chromatography (Waters MS triggered Prep-LC with Acquity QDA detector, chromatographic column: Welch C18, 150*19 mm, 5 µm; mobile phase 1: water (containing 0.1% formic acid); mobile phase 2: acetonitrile; 5-min gradient, gradient ratio: acetonitrile phase 2%-10%, flow rate: 25 mL/min) to give the title compound **009c** (1.1 g, yield: 90%).

MS m/z (ESI): 264.1 (M+1)⁺.

### Step 3 Preparation of (R)-2-(3-((1-chloropyrido[3,4-d]pyridazin-4-yl)amino)piperidin-1-yl)acetamide (009d)

Compound **009c** (200 mg, 0.76 mmol) was dissolved in dichloromethane (5 mL), and triethylamine (230 mg, 2.28 mmol) and bromoacetamide (115 mg, 0.83 mmol) were added in sequence. The reaction was stirred at 25°C for 2 h. After the reaction was completed, the reaction mixture was directly dried by rotary evaporation, and the residue was purified by silica gel column chromatography with eluent system A to give the title compound **009d** (220 mg, yield: 90%).

MS m/z (ESI): 321.1 (M+1)⁺.

### Step 4 Preparation of (R)-2-(3-((1-(4-chloro-2-hydroxyphenyl)pyrido[3,4-d]pyridazin-4-yl)amino)piperidin-1-yl)acetamide (009)

Compound **009d** (220 mg, 0.69 mmol) was dissolved in a mixed solvent of 1,4-dioxane and water (1.1 mL, V/V = 10:1). (4-chloro-2-hydroxyphenyl)boric acid (118 mg, 0.69 mmol), [1,1'-bis(diphenylphosphino)ferrocene]palladium (II) dichloride (100 mg, 0.14 mmol) and sodium carbonate (145 mg, 1.37 mmol) were added. The reaction mixture was allowed to react under nitrogen protection at 100°C for 2 h. After the reaction was completed, the reaction mixture was filtered, the filtrate was collected, and concentrated under reduced pressure. The crude product was purified by preparative high performance liquid chromatography (Waters MS-triggered Prep-LC with SQD2 detector, chromatographic column: Oxbridge C18 150 × 19 mm, 5 µm; mobile phase 1: water (containing 0.1% TFA); mobile phase 2: acetonitrile; 15-min gradient, gradient ratio: acetonitrile phase 18%-28%, flow rate: 20 mL/min) to give the title compound **009** (34.66 mg, yield: 12%).

MS m/z (ESI): 413.1(M+1)⁺.

¹H NMR (400 MHz, DMSO-*d*₆) δ 10.38 (s, 1H), 10.07 (s, 1H), 9.81 (s, 1H), 8.93 (s, *J*=5.6 Hz,1H), 7.96 (s, 1H), 7.69 (s, 1H), 7.37-7.34 (m, 2H), 7.07-7.05 (m, 2H), 4.81 (s, 2H), 3.99 (s, 2H), 3.84-2.99 (m, 3H), 2.15-1.73 (m,4H).

### Example 6

### (R)-(3-((1-(4-chloro-2-hydroxyphenyl)pyrido[3,4-d]pyridazin-4-yl)amino)piperidin-1-yl)(cyclopropyl)methanone (006)

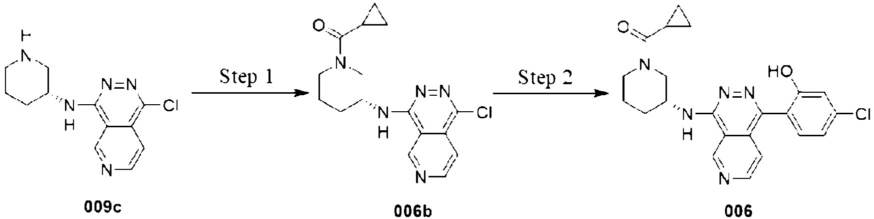

### Step 1 Preparation of (R)-(3-((1-chloropyrido[3,4-d]pyridazin-4-yl)amino)piperidin-1-yl)(cyclopropyl)methanone (006b)

(R)-1-chloro-N-(piperidin-3-yl)pyrido[3,4-d]pyridazin-4-amine **009c** (200 mg, 0.76 mmol) was dissolved in dichloromethane (10 mL), triethylamine (153 mg, 1.52 mmol) was added, and cyclopropanecarbonyl chloride (95 mg, 0.91 mmol) was added at 0°C. The reaction mixture was stirred to react at 25°C for 2 h. After the reaction was completed, the reaction mixture was directly dried by rotary evaporation, and the resulting residue was purified by silica gel column chromatography with eluent system A to give the title compound **006b** (175 mg, yield: 63%).

MS m/z (ESI): 332.1 (M+1)⁺.

### Step 2 Preparation of (R)-(3-((1-(4-chloro-2-hydroxyphenyl)pyrido[3,4-d]pyridazin-4-yl)amino)piperidin-1-yl)(cyclopropyl)methanone (006)

Compound **006b** (175 mg, 0.53 mmol) was dissolved in a mixed solvent of dioxane and water (11 mL, V/V = 10:1). (4-chloro-2-hydroxyphenyl)boric acid (91 mg, 0.53 mmol), sodium carbonate (112 mg, 1.05 mmol) and [1,1'-bis(diphenylphosphino)ferrocene]palladium (II) dichloride (39 mg, 0.05 mmol) were added. The reaction mixture was allowed to react with stirring at 100°C for 4 h. After the reaction was completed, the reaction mixture was filtered, and the filtrate was concentrated under reduced pressure. The crude product was purified by preparative high performance liquid chromatography (Waters MS triggered Prep-LC with Acquity QDA detector, chromatographic column: Welch C18, 250*21.2 mm, 10 µm; mobile phase 1: water (containing 0.1% aqueous ammonia); mobile phase 2: acetonitrile; 15-min gradient, gradient ratio: acetonitrile phase 17%-37%, flow rate: 25 mL/min) to give the title compound **006** (15.54 mg, yield: 6.9%).

MS m/z (ESI): 424.1 (M+1)⁺.

¹H NMR (400 MHz, DMSO-*d*₆) *δ* 9.78 (d, *J =* 20.4 Hz, 1H), 8.86 (d, *J =* 5.6 Hz, 1H), 7.70 (s, 1H), 7.32 (t, *J* = 7.2 Hz, 2H), 7.00 (d, *J* = 7.2 Hz, 2H), 4.43-4.33 (m, 1H), 4.33 - 4.04 (m, 2H), 3.21 - 3.14 (m, 1H), 2.95-2.72 (m, 1H), 2.17 (s, 1H), 1.87 - 1.50 (m, 4H), 0.76 - 0.45 (m, 4H).

### Example 7

### (R)-3-((1-(4-chloro-2-hydroxyphenyl)pyrido[3,4-d]pyridazin-4-yl)amino)piperidin-1-formamide (008)

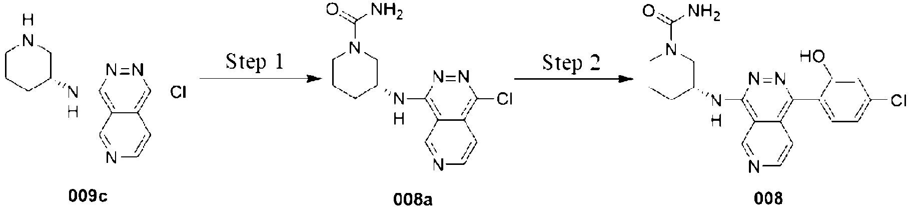

### Step 1 Preparation of (R)-3-((1-chloropyrido[3,4-d]pyridazin-4-yl)amino)piperidin-1-formamide (008a)

Compound **009c** (150 mg, 0.57 mmol) was dissolved in dichloromethane, triethylamine (173 mg, 1.71 mmol) was added, and then phenyl carbamate (78 mg, 0.59 mmol) was slowly added dropwise. The reaction mixture was allowed to react at room temperature for 1 h. After the reaction was completed, the mixture was dried by rotary evaporation to remove the solvent, thus giving the crude product. The resulting residue was purified by silica gel column chromatography with eluent system B to give the title compound **008a** (170 mg, yield: 97%).

MS m/z (ESI): 307.1 (M+1)⁺.

### Step 2 Preparation of (R)-3-((1-(4-chloro-2-hydroxyphenyl)pyrido[3,4-d]pyridazin-4-yl)amino)piperidin-1-formamide (008)

Compound **008a** (100 mg, 0.33 mmol) was dissolved in 1,4-dioxane and water (6 mL, V/V = 5:1), and (4-chloro-2-hydroxyphenyl)boronic acid (67 mg, 0.39 mmol), [1,1'-bis(diphenylphosphino)ferrocene]palladium (II) dichloride (48 mg, 0.07 mmol) and sodium carbonate (69 mg, 0.65 mmol) were added. The reaction mixture was allowed to react with stirring under nitrogen protection at 100°C for 2 h. After the reaction was completed, the reaction mixture was filtered, and the filtrate was concentrated under reduced pressure. The crude product was purified by preparative high performance liquid chromatography (Waters MS triggered Prep-LC with Acquity QDA detector, chromatographic column: Welch C18, 250 * 21.2 mm, 10 µm; mobile phase 1: water (containing 0.1% aqueous ammonia); mobile phase 2: acetonitrile; 15-min gradient, gradient ratio: acetonitrile phase 12%-27%, flow rate: 25 mL/min) to give the title compound 008 (4.83 mg, yield: 4%).

MS m/z (ESI): 307.1 (M+1)⁺.

¹H NMR (400 MHz, DMSO-*d*₆) *δ* 9.74 (s, 1H), 8.84 (d, *J* = 5.6 Hz, 1H), 7.67 (d, *J* = 7.2 Hz, 1H), 7.34 (s, 1H), 7.29 (d, *J =* 8.0 Hz, 1H), 6.91 (s, 2H), 5.99 (s, 2H), 4.30 (d, *J =* 6.8 Hz, 1H), 4.20 (d, *J* = 12.4 Hz, 1H), 3.88 (d, *J* = 13.2 Hz, 1H), 2.77 (dd, *J* = 12.4, 10.0 Hz, 2H), 2.13 (d, *J* = 8.8 Hz, 1H), 1.75 (d, *J =* 3.6 Hz, 1H), 1.65 (dd, *J =* 23.6, 12.0 Hz, 1H), 1.51 (t, *J =* 12.0 Hz, 1H).

### Example 8

### Methyl (R)-3-((1-(4-chloro-2-hydroxyphenyl)pyrido[3,4-d]pyridazin-4-yl)amino)piperidin-1-carboxylate (010)

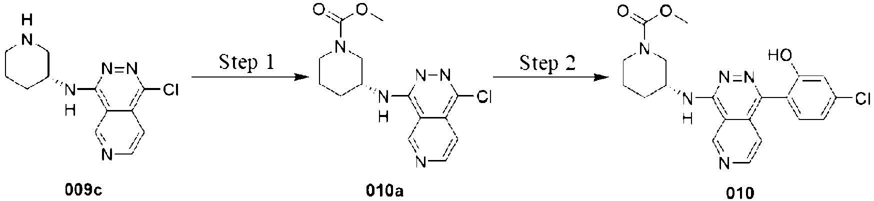

### Step 1 Preparation of methyl (R)-3-((1-chloropyrido[3,4-d]pyridazin-4-yl)amino)piperidin-1-carboxylate (010a)

Compound **009c** (200 mg, 0.76 mmol) was dissolved in dichloromethane (5 mL), triethylamine (115.4 mg, 1.14 mmol) was added at 0°C, and the mixture was stirred to react for 10 min. Then, methyl chloroformate (57 mg, 0.61 mmol) was added at 0°C, and the mixture was further stirred to react at 0°C for 40 min. After the reaction was completed, the reaction mixture was concentrated, and the resulting residue was purified by silica gel column chromatography with eluent system A to give the title compound **010a** (153 mg, yield: 62.5%).

MS m/z (ESI): 322.1 (M+1)⁺.

### Step 2 Preparation of methyl (R)-3-((1-(4-chloro-2-hydroxyphenyl)pyrido[3,4-d]pyridazin-4-yl)amino)piperidin-1-carboxylate (010)

Compound **010a** (153 mg, 0.48 mmol), 2-hydroxy-4-chlorophenylboronic acid (123 mg, 0.71 mmol), and sodium carbonate (151.6 mg, 1.43 mmol) were dissolved in a mixed solvent of 1,4-dioxane and water (5 mL, V/V = 4:1), and 1,1'-bis(diphenylphosphino)ferrocene palladium dichloride (104.6 mg, 0.14 mmol) was added, and the reaction was carried out at 100°C under nitrogen protection for 4 h. After the reaction was completed, the reaction mixture was filtered and concentrated under reduced pressure. The crude product was purified by preparative high performance liquid chromatography (Waters MS triggered Prep-LC with Acquity QDA detector, chromatographic column: Welch C18, 250*21.2 mm, 10 µm; mobile phase 1: water (containing 0.1% formic acid); mobile phase 2: acetonitrile; 20-min gradient, gradient ratio: acetonitrile phase 25%-55%, flow rate: 30 mL/min) to give the title compound **010** (6.35 mg, yield: 3.2%).

MS m/z (ESI): 414.1 (M+1)⁺.

¹H NMR (400 MHz, DMSO-*d*₆): *δ* 10.30 (s, 1H), 9.78 (s, 1H), 8.86 (d, *J*=5.6 Hz, 1H), 7.70 (d, *J*=7.2 Hz, 1H), 7.36(d, *J*=8.4 Hz, 1H), 7.31 (d, *J*=5.6 Hz, 1H), 7.05 (s, 2H), 4.35-4.27 (m, 2H), 3.90 (d, *J*=13.2 Hz, 1H), 3.58 (s, 3H), 2.94-2.89 (m, 2H), 2.14 -2.11(m, 1H), 1.86-1.83 (m, 1H), 1.74-1.66 (m, 1H), 1.58-1.48 (m, 1H).

### Example 9

### 2-(((1-(4-chloro-2-hydroxyphenyl)pyrido[3,4-d]pyridazin-4-yl)amino)methyl)pyrrolidin-1-formamide (012)

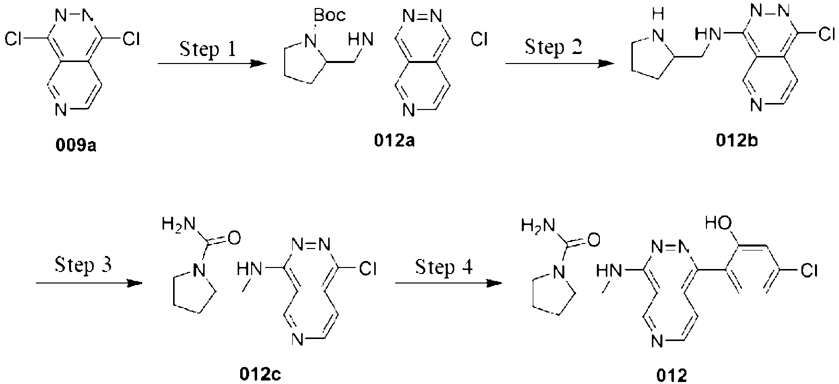

### Step 1 Preparation of tert-butyl 2-(((1-chloropyrido[3,4-d]pyridazin-4-yl)amino)methyl)pyrrolidin-1-carboxylate (012a)

Compound **009a** (200 mg, 1.00 mmol) was dissolved in *N*-methylpyrrolidone (5 mL), and tert-butyl [2-(aminomethyl)pyrrolidin-1-yl]carboxylate (211 mg, 1.00 mmol) and *N,N-*diisopropylethylamine (388 mg, 3.00 mmol) were added. The reaction mixture was allowed to react at 100°C for 2 h. After the reaction was completed, the reaction mixture was directly dried by rotary evaporation, and the resulting residue was purified by silica gel column chromatography with eluent system B to give the title compound **012a** (230 mg, yield: 63%).

MS m/z (ESI): 364.1 (M+1)⁺.

### Step 2 1-chloro-N-(pyrrolidin-2-ylmethyl)pyrido[3,4-d]pyridazin-4-amine (012b)

4 M hydrochloric acid in dioxane (15 mL) was added to compound **012a** (100 mg, 0.27 mmol), and the mixture was stirred to react at 25°C for 2 h. After the reaction was completed, the reaction mixture was directly dried by rotary evaporation to give the title compound **012b** (75 mg, yield: 97%).

MS m/z (ESI): 264.1 (M+1)⁺.

### Step 3 2-(((1-chloropyrido[3,4-d]pyridazin-4-yl)amino)methyl)pyrrolidin-1-formamide (012c)

Compound **012b** (700 mg, 0.76 mmol) was dissolved in dichloromethane (10 mL), and phenyl carbamate (104 mg, 0.76 mmol) and triethylamine (306.97 mg, 3.03 mmol) were added. The reaction mixture was allowed to react with stirring at 25°C for 4 h. After the reaction was completed, the reaction mixture was directly dried by rotary evaporation, and the resulting residue was purified by silica gel column chromatography with eluent system A to give the title compound **012c** (200 mg, yield: 86.0%).

MS m/z (ESI): 307.0 (M+1)⁺.

¹H NMR (400 MHz, DMSO-*d*₆) *δ* 9.70 (s, 1H), 9.23 (s, 1H), 9.12-9.02 (m, 2H), 6.31 (s, 2H), 4.13 (s, 1H), 3.73-3.47 (m, 2H), 1.91 (d, *J*=5.9, 6H).

### Step 4 2-(((1-(4-chloro-2-hydroxyphenyl)pyrido[3,4-d]pyridazin-4-yl)amino)methyl)pyrrolidin-1-formamide (012)

Compound **012c** (100 mg, 0.326 mmol) and sodium carbonate (103 mg, 0.978 mmol) were dissolved in a mixed solvent of 1,4-dioxane and water (11 mL, V/V = 10:1). (4-chloro-2-hydroxyphenyl)boric acid (62 mg, 0.36 mmol) and [1,1'-bis(diphenylphosphino)ferrocene]palladium (II) dichloride (48 mg, 0.065 mmol) were added. The reaction system was allowed to react with stirring at 100°C for 14 h. After the reaction was completed, the mixture was filtered with celite, and the filtrate was concentrated under reduced pressure. The crude product was purified by preparative high performance liquid chromatography (Waters MS triggered Prep-LC with Acquity QDA detector, chromatographic column: Welch C18, 250*21.2 mm, 10 µm; mobile phase 1: water (containing 0.1% aqueous ammonia); mobile phase 2: acetonitrile; 15-min gradient, gradient ratio: acetonitrile phase 15%-100%, flow rate: 25 mL/min) to give the title compound **012** (1.75 mg, a mixture of two enantiomers, yield: 1.35%).

MS m/z (ESI): 399.0 (M+1)⁺.

¹H NMR (400 MHz, DMSO-*d*₆) *δ* 9.60 (s, 1H), 8.86 (d, *J*=5.2 Hz, 1H), 8.78 (s, 1H), 7.31 (d, J=6.4 Hz, 2H), 6.97 (s, 2H), 6.40 (s, 2H), 4.15 (s, 1H), 3.74 (s, 1H),3.22 (m, 2H), 1.94-1.91 (m, 4H).

### Example 10

### 1-(2-(1-(4-chloro-2-hydroxyphenyl)pyrido[3,4-d]pyridazin-4-ylamino)methyl)pyrrolidin-1-yl)ethan-1-one (011)

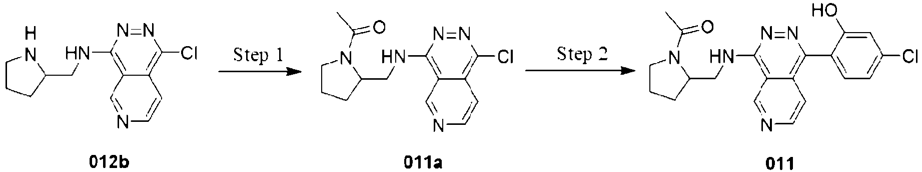

### Step 1-(2-((1-chloropyrido[3,4-d]pyridazin-4-ylamino)methyl)pyrrolidin-1-yl)ethan-1-one (011a)

Compound **012b** (200 mg, 0.76 mmol) was dissolved in dichloromethane (8 mL) and triethylamine (383.7 mg, 3.79 mmol) was added. The reaction was stirred at 0°C for 5 min, and then a solution of acetyl chloride (30 mg, 0.38 mmol) in dichloromethane (2 mL) was slowly added dropwise. The reaction mixture was allowed to react with stirring at 0°C for 30 min. After the reaction was completed, the reaction mixture was quenched with water and extracted with dichloromethane. The organic layer was collected and dried over anhydrous sodium sulfate. The resulting residue was purified by silica gel column chromatography with eluent system A to give the title compound **011a** (120 mg, yield: 51.9%).

MS m/z (ESI): 306 (M+1)⁺.

### Step 2 Preparation of 1-(2-(1-(4-chloro-2-hydroxyphenyl)pyrido[3,4-d]pyridazin-4-ylamino)methyl)pyrrolidin-1-yl)ethan-1-one (011)

Compound **011a** (120 mg, 0.39 mmol) was dissolved in a mixed solvent of 1,4-dioxane and water (11 mL, V/V = 10:1). (4-chloro-2-hydroxyphenyl)boric acid (81 mg, 0.47 mmol), sodium carbonate (125 mg, 1.18 mmol) and [1,1'-bis(diphenylphosphino)ferrocene]palladium (II) dichloride (57 mg, 0.078 mmol) were added. The reaction mixture was allowed to react with stirring at 100°C for 2 h. After the reaction was completed, the reaction mixture was filtered, the filtrate was concentrated under reduced pressure to give a crude product. The crude product was purified by preparative high performance liquid chromatography (Waters MS triggered Prep-LC with Acquity QDA detector, chromatographic column: Xtimate C18, 250*30mm, 10 µm; mobile phase 1: water (containing 0.05% aqueous ammonia); mobile phase 2: acetonitrile; 15-min gradient, gradient ratio: acetonitrile phase 12%-35%, flow rate: 25 mL/min) to give the title compound **011** (5.7 mg, a mixture of two enantiomers, yield: 3.7%).

MS m/z (ESI): 398 (M+1)⁺.

¹H NMR (400 MHz, DMSO-*d*₆) δ 9.69 (s,1H), 8.84 (t, *J*=5.6 Hz, 1H), 8.26 (s, 1H), 7.34-7.29 (m, 2H), 6.95 (s, 2H), 4.45-4.34 (m, 1H), 3.84-3.70 (m, 2H), 3.58-3.50(m,2H), 2.20 (s, 1.5 H), 2.08-2.06 (m, 1H), 2.02 (s,1.5 Hz), 1.93-1.89 (m, 3H).

### Example 11

### 2-(3-((1-(4-chloro-2-hydroxyphenyl)pyrido[3,4-d]pyridazin-4-yl)amino)phenyl)-2-methylpropionitrile (014)

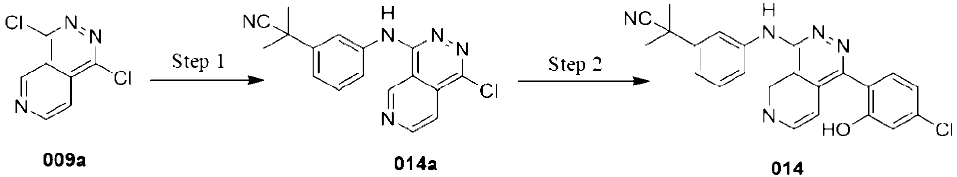

### Step 1 2-(3-((1-chloropyrido[3,4-d]pyridazin-4-yl)amino)phenyl)-2-methylpropionitrile (014a)

Compound **009a** (200.0 mg, 1.2 mmol) and 2-(3-aminophenyl)-2-methylpropionitrile (250 mg, 1.2 mmol) were dissolved in N-methylpyrrolidone (5 mL), and *N*,*N*-diisopropylethylamine (484 mg, 3.7 mmol) was added thereto. The reaction system was allowed to react with stirring at 100°C for 2 h. After the reaction was completed, the mixture was extracted with ethyl acetate, and the organic phase was collected and dried over anhydrous sodium sulfate. The resulting residue was purified by silica gel column chromatography with eluent system B, to give the title compound **014a** (200 mg, yield: 39%).

MS m/z (ESI): 324.1 (M+1)⁺.

### Step 2 Preparation of 2-(3-((1-(4-chloro-2-hydroxyphenyl)pyrido[3,4-d]pyridazin-4-yl)amino)phenyl)-2-methylpropionitrile (014)

Compound **014a** (100.0 mg, 0.3 mmol) and (4-chloro-2-hydroxyphenyl)boronic acid (80 mg, 0.4 mmol) were dissolved in a mixed solvent of 1,4-dioxane and water (11 mL, V/V = 1:10), and 1,1-bis(diphenylphosphino)ferrocene palladium dichloride (113.0 mg, 0.15 mmol) and sodium carbonate (98 mg, 0.9 mmol) were added thereto. The reaction system was allowed to react with stirring at 90°C under nitrogen for 6 h. After the reaction was completed, the solvent was removed by rotary evaporation to give a crude product. The crude product was separated and purified by preparative high performance liquid chromatography (Waters MS-triggered Prep-LC with QDA detector, chromatographic column: WELCH Xtimate C18, 21.2 × 250mm, 10µm; mobile phase 1: water (containing 0.1% formic acid); mobile phase 2: acetonitrile; 18-min gradient, gradient ratio: acetonitrile phase 5%-100%; flow rate: 30 mL/min) to give the title compound **014** (4.2 mg, yield: 3.2%).

MS m/z (ESI): 416.1 (M+1)⁺.

¹H NMR (400 MHz, DMSO-*d*₆) *δ* 10.28 (s, 1H), 10.02 (s, 1H), 9.79 (s, 1H), 8.95 (d, *J* = 5.6 Hz, 1H), 8.10 (dd, *J =* 4.0, 2.0 Hz, 2H), 7.49-7.45 (m, 1H), 7.42-7.39 (m, 2H), 7.24 (d, *J =* 8.4 Hz, 1H), 7.07-7.05 (m, 2H), 1.74 (s, 6H).

### Example 12

### 2-(4-((1-(4-chloro-2-hydroxyphenyl)pyrido[3,4-d]pyridazin-4-yl)amino)phenyl)-2-methylpropionitrile (015)

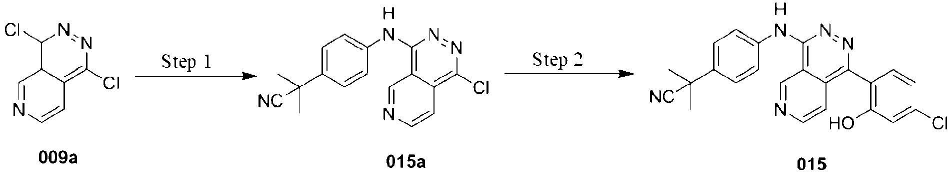

### Step 1 Preparation of 2-(4-((1-chloropyrido[3,4-d]pyridazin-4-yl)amino)phenyl)-2-methylpropionitrile (015a)

1,4-dichloropyrido[3,4-*d*]pyridazine **009a** (200 mg, 1.00 mmol) was dissolved in *N-*methylpyrrolidone (5 mL), and 2-(4-aminophenyl)-2-methylpropionitrile (160 mg, 1.00 mmol) and *N,N*-diisopropylethylamine (388 mg, 3.00 mmol) were added. The reaction mixture was stirred at 100°C for 2 h. After the reaction was completed, the reaction mixture was directly dried by rotary evaporation, and the resulting residue was purified by silica gel column chromatography with eluent system A to give the title compound **015a** (200 mg, yield: 59%).

MS m/z (ESI): 324.1 (M+1)⁺.

### Step 2 Preparation of 2-(4-((1-(4-chloro-2-hydroxyphenyl)pyrido[3,4-d]pyridazin-4-yl)amino)phenyl)-2-methylpropionitrile (015)

Compound **015a** (100 mg, 0.31 mmol) was dissolved in a mixed solvent of dioxane and water (11 mL, V/V = 10:1). (4-chloro-2-hydroxyphenyl)boric acid (53 mg, 0.31 mmol), sodium carbonate (65 mg, 0.62 mmol) and [1,1'-bis(diphenylphosphino)ferrocene]palladium (II) dichloride (23 mg, 0.03 mmol) were added. The reaction mixture was stirred at 100°C for 2 h. After the reaction was completed, the reaction mixture was directly dried by rotary evaporation, directly concentrated, and then purified by preparative high performance liquid chromatography (Waters MS triggered Prep-LC with Acquity QDA detector, chromatographic column: Welch C18 250*21.2 mm, 10 µm; mobile phase 1: water (containing 0.1% aqueous ammonia); mobile phase 2: acetonitrile; 15-min gradient, gradient proportion: acetonitrile phase 37%-47%, flow rate: 25 mL/min) to give the title compound **015** (2.54 mg, yield: 1.9%).

MS m/z (ESI): 416.1 (M+1)⁺.

¹H NMR (400 MHz, DMSO-*d*₆) *δ* 9.89 (s, 1H), 9.64 (s, 1H), 8.83 (d, *J =* 5.6 Hz, 1H), 8.00 (d, *J =* 8.4 Hz, 2H), 7.57 (d, *J =* 4.0 Hz, 1H), 7.51 (d, *J =* 8.8 Hz,2H), 7.15 - 7.13 (m, 1H), 6.48-6.35 (d, 2H), 1.71 (s, 6H).

### Example 13

### 5-chloro-2-(4-((2-hydroxy-2-methylpropyl)amino)-6-(1-propyn-1-yl)phthalazin-1-yl)-3-methylphenol (024)

### (R)-5-chloro-2-(4-((2-hydroxy-2-methylpropyl)amino)-6-(prop-1-yn-1-yl)phthalazin-1-yl)-3-methylphenol

### (S)-5-chloro-2-(4-((2-hydroxy-2-methylpropyl)amino)-6-(prop-1-yn-1-yl)phthalazin-1-yl)-3-methylphenol

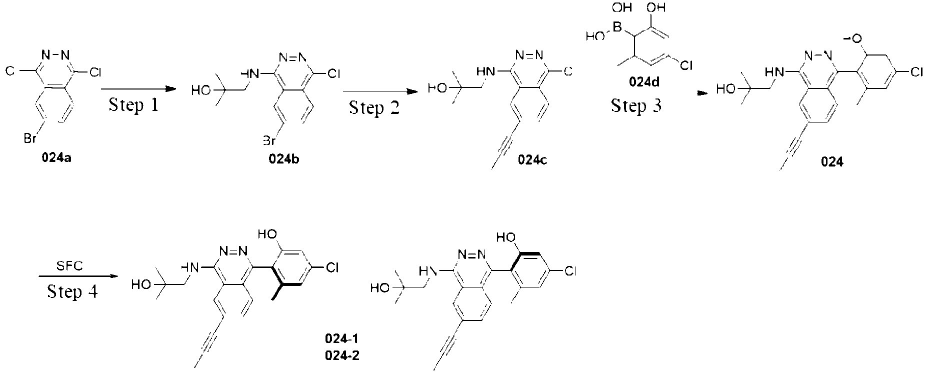

### Step 1 Preparation of 1-((7-bromo-4-chlorophthalazin-1-yl)amino)-2-methylpropan-2-ol (024b)

6-bromo-1,4-dichlorophthalazine **024a** (1 g, 3.6 mmol), and 1-amino-2-methyl-2-propanol (350 mg, 3.96 mmol) were dissolved in *N*-methylpyrrolidone (15 mL), and *N,N-*diisopropylethylamine (1.4 g, 10.8 mmol) was added thereto. The reaction system was allowed to react at 100°C for 2 h. After the reaction was completed, the reaction mixture was washed with saturated brine, extracted with ethyl acetate (3x30 mL), and the organic phases were combined, dried over anhydrous sodium sulfate, filtered and concentrated to give a crude product. The resulting residue was purified by silica gel column chromatography with eluent system B to give the title compound **024b** (500 mg, yield: 41.7%).

MS m/z(ESI): 330.0(M+1).

¹H NMR (400 MHz, DMSO-*d*₆) δ 8.82 (d, 1H), 8.16 (dd, 1H), 7.98 (d, 1H), 7.59 (t, 1H), 4.83 (s, 1H), 3.60 (d, 2H), 1.19 (s, 6H).

### Step 2 Preparation of 1-((4-chloro-7-(propyn-1-yl)phthalazin-1-yl)amino)-2-methylpropan-2-ol (024c)

Compound **024b** (100 mg, 0.30 mmol) was dissolved in N,N-dimethylacetamide (11 mL), cesium carbonate (147.8 mg, 0.45 mmol), cuprous iodide (23 mg, 0.12 mmol), 1-(trimethylsilyl)propyne (33.9 mg, 0.30 mmol) and bis(triphenylphosphine) palladium dichloride (42.5 mg, 0.061 mmol) were added, and 0.05 mL of water was added thereto. The reaction system was allowed to react under nitrogen protection at 100°C for 1 h. After the reaction was completed, the reaction mixture was washed with saturated brine, and extracted with ethyl acetate (3x30mL). The organic phases were combined, dried over anhydrous sodium sulfate, filtered and concentrated to give a crude product. The resulting residue was purified by silica gel column chromatography with eluent system B, to give the title compound **024c** (60 mg, yield: 60.9%).

MS m/z(ESI): 290.1(M+1).

### Step 3 Preparation of 5-chloro-2-(4-((2-hydroxy-2-methylpropyl)amino)-6-(1-propyn-1-yl)phthalazin-1-yl)-3-methylphenol (024)

Compound **024c** (50 mg, 0.17 mmol) was dissolved in a mixed solvent of 1,4-dioxane and water (3.3 mL, V/V = 10:1), followed by the addition of (4-chloro-2-hydroxy-6-methylphenyl)boronic acid **024d** (35 mg, 0.19 mmol), potassium carbonate (72 mg, 0.52 mmol) and 1,1'-bis(diphenylphosphino)ferrocene palladium dichloride (12.5 mg, 0.017 mmol), and the reaction was carried out at 100°C under nitrogen protection for 2 h. After the reaction was completed, the reaction mixture was filtered, the filtrate was collected, and concentrated under reduced pressure to give a crude product. The crude product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 27%-100%, flow rate: 20 mL/min) to give the title compound **024** (15.2 mg, yield: 20.8%, a pair of axial chiral isomers).

MS m/z(ESI): 396.1(M+1).

¹H NMR (400 MHz, DMSO-*d*₆) δ 9.83 (s, 1H), 8.51 (s, 1H), 7.73 (d, 1H), 7.39 (s, 1H), 7.23 (d, 1H), 6.91 (s, 1H), 6.85 (s, 1H),3.68-3.54 (m, 2H), 2.13 (s, 3H), 1.88 (s, 3H), 1.21 (s, 6H).

### Step 4 (R)-5-chloro-2-(4-((2-hydroxy-2-methylpropyl)amino)-6-(prop-1-yn-1-yl)phthalazin-1-yl)-3-methylphenol

### (S)-5-chloro-2-(4-((2-hydroxy-2-methylpropyl)amino)-6-(prop-1-yn-1-yl)phthalazin-1-yl)-3-methylphenol

Compound **024** (20 mg) was purified by preparative SFC chromatography (SFC 150 column: Daicel CHIRALCEL AZ, 250mm× 30 mm ID, 10 µm; mobile phase 1: carbon dioxide; mobile phase 2: ethanol (0.2% aqueous ammonia); 6-min gradient, gradient ratio carbon dioxide: ethanol phase = 60/40, flow rate: 2 mL/min) to give the title compound **024-1** (8 mg, shorter retention time, yield: 40%) and the title compound **024-2** (9 mg, longer retention time, yield: 45%).

### Mono-configurational isomer (shorter retention time)

MS m/z(ESI): 396.1(M+1).

¹H NMR (400 MHz, CD₃OD) δ 8.29 (s, 1H), 7.70 (dd, 1H), 7.34 (d, 1H), 6.87 (d, 1H), 6.79 (d, 1H), 3.68 (d, 2H), 2.08 (s, 3H), 1.93 (s, 3H), 1.29 (s, 6H).

### Mono-configurational isomer (longer retention time)

MS m/z(ESI): 396.1(M+1).

¹H NMR (400 MHz, CD₃OD) δ 8.31 (s, 1H), 7.72 (d, 1H), 7.36 (d, 1H), 6.88 (s, 1H), 6.82 (d, 1H), 3.70 (d, 2H), 2.10 (s, 3H), 1.96 (s, 3H), 1.30 (s, 6H).

### Example 14

### 5-chloro-2-(4-(R)-2-hydroxypropyl)amino)-6-(prop-1-yn-1-yl)phthalazin-1-yl)-3-methylphenol (035)

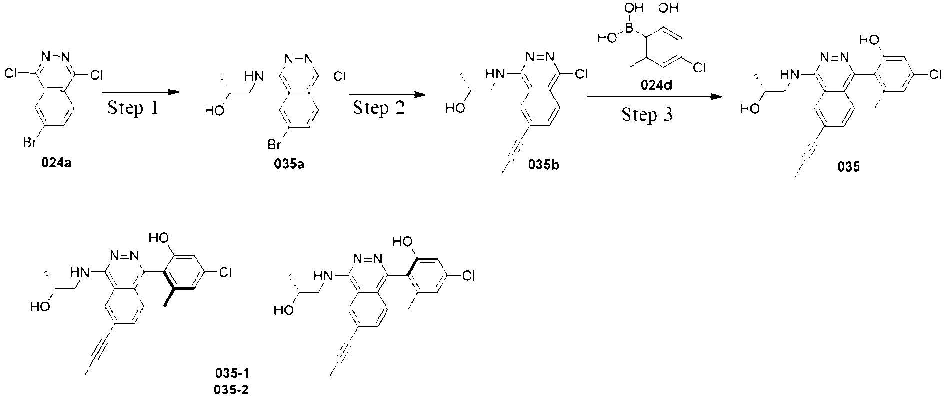

The synthetic route of Example 13 was used. The raw material 1-amino-2-methyl-2-propanol in step 1 was replaced with (*R*)-(-)-1-amino-2-propanol (500 mg, 6.65 mmol, Shanghai Haohong Bio-pharmaceutical Technology Co., Ltd.). The reaction product from step 3 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 25%-100%, flow rate: 20 mL/min) to give the title product **035** (13 mg, yield: 24.7%, a pair of axial chiral isomers).

MS m/z (ESI): 382.1 (M+1).

¹H NMR (400 MHz, CD₃OD) δ 8.32 (s, 1H), 7.73 (d, 1H), 7.36 (d, 1H), 6.90 (s, 1H), 6.82 (s, 1H), 4.59 (s, 1H), 4.19 (d, 1H), 3.77-3.66 (m, 1H), 3.57 (td, 1H), 2.10 (s, 3H), 1.97 (s, 3H), 1.28 (d, 3H).

### Example 15

### 5-chloro-2-(4-((2-hydroxy-2-methylpropyl)amino)-6-(prop-1-yn-1-yl)phthalazin-1-yl)phenol (036)

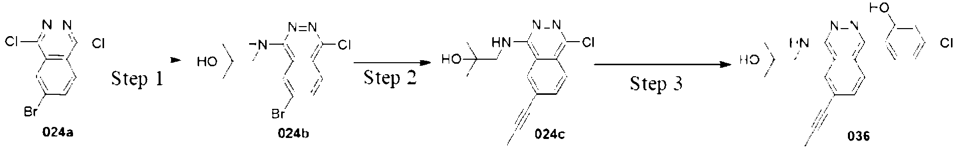

The synthetic route of Example 13 was used. The raw material (4-chloro-2-hydroxy-6-methylphenyl)boronic acid **024d** in step 3 was replaced with (4-chloro-2-hydroxyphenyl)boronic acid (50 mg, 0.29 mmol). The reaction product from step 3 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 23%-100%, flow rate: 20 mL/min) to give the title product **036** (10 mg, yield: 23.6%).

MS m/z (ESI): 382.1 (M+1).

¹H NMR (400 MHz, CD₃OD) δ 8.31 (s, 1H), 7.76 (dd, 1H), 7.57 (d, 1H), 7.31 (d, 1H), 7.02-6.99 (m, 2H), 3.70 (s, 2H), 2.11 (s, 3H), 1.31 (s, 6H).

### Example 16

### (R)-5-chloro-2-(4-((2-hydroxypropyl)amino)-6-(prop-1-yn-1-yl)phthalazin-1-yl)phenol (037)

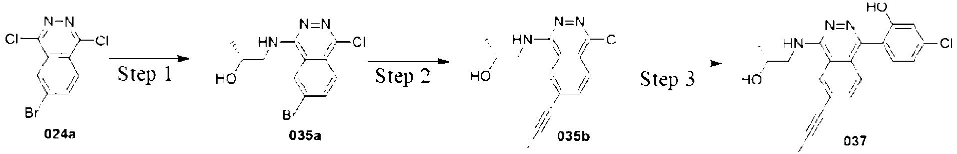

The synthetic route of Example 14 was used. The raw material (4-chloro-2-hydroxy-6-methylphenyl)boronic acid **024d** in step 3 was replaced with (4-chloro-2-hydroxyphenyl)boronic acid (50 mg, 0.29 mmol). The reaction product from step 3 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 22%-100%, flow rate: 20 mL/min) to give the title product **037** (15 mg, yield: 28.6%).

MS m/z (ESI): 368.1 (M+1).

¹H NMR (400 MHz, CD₃OD) δ 8.33 (s, 1H), 7.74 (d, 1H), 7.58(d,1H), 7.38 (d, 1H), 6.91 (s, 1H), 6.83 (s, 1H), 4.60 (s, 1H), 4.20 (d, 1H), 3.79-3.67 (m, 1H), 3.58 (td, 1H), 2.11 (s, 3H), 1.29 (d, 3H).

### Example 17

### 5-chloro-2-(4-((2-hydroxy-2-methylpropyl)amino)-6-(1-methyl-1H-pyrazol-4-yl)phthalazin-1-yl)phenol (004)

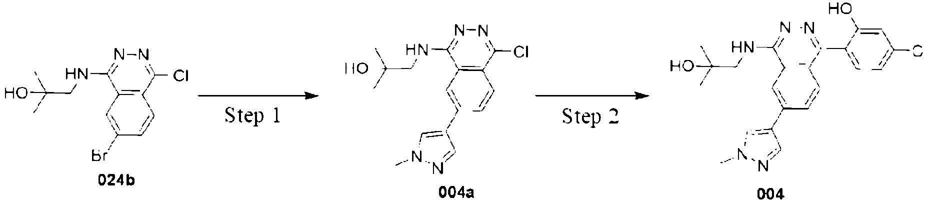

### Step 1 Preparation of 1-((4-chloro-7-(1-methyl-1H-pyrazol-4-yl)phthalazin-1-yl)amino)-2-methylpropan-2-ol (004a)

Compound **024b** (100 mg, 0.30 mmol) was dissolved in a mixed solvent of 1,4-dioxane and water (6 mL, V/V = 5:1), followed by the addition of 1-methyl-1*H*-pyrazole-4-boronic acid pinacol ester (63 mg, 0.3 mmol), potassium carbonate (84 mg, 0.61 mmol) and 1,1'-bis(diphenylphosphino)ferrocene palladium dichloride (44 mg, 0.06 mmol), and the reaction was carried out at 80°C under nitrogen protection for 2 h. After the reaction was completed, the mixture was cooled to room temperature, filtered and concentrated under reduced pressure to give a crude product. The resulting residue was purified by silica gel column chromatography with eluent system B to give the title compound **004a** (60 mg, yield: 47.8%).

MS m/z (ESI): 332.1 (M+1)+.

### Step 2 Preparation of 5-chloro-2-(4-((2-hydroxy-2-methylpropyl)amino)-6-(1-methyl-1H-pyrazol-4-yl)phthalazin-1-yl)phenol (004)

Compound **004a** (30 mg, 0.091 mmol) was dissolved in a mixed solvent of 1,4-dioxane and water (3.3 mL, V/V = 10:1), followed by the addition of (4-chloro-2-hydroxyphenyl)boronic acid (19 mg, 0.11 mmol), sodium carbonate (29 mg,0.27 mmol) and 1,1'-bis(diphenylphosphino)ferrocene palladium dichloride (7 mg,0.0091 mmol), and the reaction was carried out at 100°C under nitrogen protection for 2 h. After the reaction was completed, the reaction mixture was filtered, the filtrate was collected, and concentrated under reduced pressure to give a crude product. The crude product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 21%-100%, flow rate: 20 mL/min) to give the title compound **004** (8.7 mg, yield: 22.8%).

MS m/z (ESI): 424.1 (M+1).

¹H NMR (400 MHz, DMSO-*d₆*) δ 8.56 (s, 1H), 8.34 (s, 1H), 8.26 (s, 1H), 8.10 (s, 1H), 8.01 (d, 1H), 7.48 (s, 1H), 7.46 (d, 1H), 7.32 (d, 1H),7.04 (d, 1H), 7.02-6.99 (m, 1H), 3.92 (s, 3H), 3.66 (s, 2H), 1.23 (s, 6H).

### Example 18

### 5-chloro-2-(4-((2-hydroxy-2-methylpropyl)amino)-6-(l-methyl-1H-pyrazol-4-yl)phthalazin-1-yl)3-methylbenzene (038)

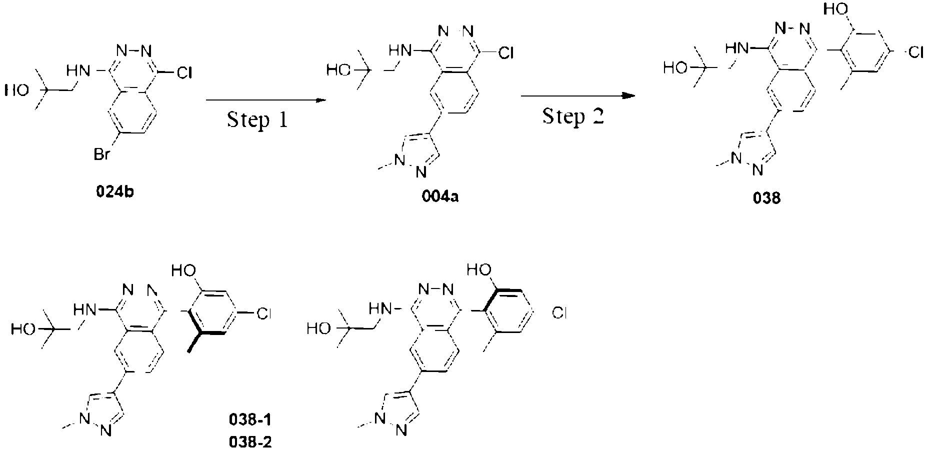

### Step 1 Preparation of 1-(4-chloro-7-(1-methylpyrazol-4-yl)phthalazin-1-amino)-2-methyl-2-propanol (004a)

Compound **024b** (100 mg, 0.30 mmol) was dissolved in a mixed solvent of dioxane and water (5 mL, V /V = 5:1), and 1-methylpyrazole-4-boronic acid pinacol ester (57 mg, 0.27 mmol), potassium carbonate (84 mg, 0.61 mmol) and 1,1'-bis(diphenylphosphino)ferrocene palladium dichloride (22 mg, 0.03 mmol) were added. The reaction was carried out at 80°C under nitrogen protection for 1 h. After the reaction was completed, the organic phase was collected and concentrated by rotary evaporation. The resulting residue was purified by silica gel column chromatography with eluent system B to give the title compound **004a** (60 mg, yield: 57%).

MS m/z (ESI): 332.1 (M+1)+.

### Step 2 Preparation of 5-chloro-2-(4-((2-hydroxy-2-methylpropyl)amino)-6-(1-methyl-1H-pyrazol-4-yl)phthalazin-1-yl)3-methylbenzene (038)

Compound **004a** (50 mg, 0.15 mmol) was dissolved in a mixed solvent of 1,4-dioxane and water (5.5 mL, V/V = 10:1), and (4-chloro-2-hydroxy-6-methylphenyl)boric acid (57 mg, 0.27 mmol), potassium carbonate (62 mg, 0.45 mmol) and 1,1'-bis(diphenylphosphino)ferrocene palladium dichloride (11 mg, 0.015 mmol) were added. The reaction was carried out at 100°C under nitrogen protection for 1 h. After the reaction was completed, the system was filtered with diatomaceous earth, and the filtrate was concentrated under reduced pressure. The crude product was purified by preparative high performance liquid chromatography (Waters MS triggered Prep-LC with Acquity QDA detector, chromatographic column: Xbridge 5µm C18 150 x 19 mm; mobile phase 1: water (containing 0.1% formic acid); mobile phase 2: acetonitrile; 13-min gradient, gradient ratio: acetonitrile phase 24%-100%, flow rate: 25 mL/min) to give the title product **038** (11.8 mg, yield: 17.7%, a pair of axial chiral isomers).

MS m/z (ESI): 438.1 (M+1).

¹H NMR (400 MHz, DMSO-*d₆*) δ 8.57 (s, 1H),8.32 (s, 1H),8.31(s, 1H) 8.08 (s, 1H), 7.96 (d, 1H), 7.44 (s, 1H), 7.23 (d, 1H),6.89 (d, 2H), 3.92 (s, 3H),3.72(d, 2H), 1.89 (s, 3H), 1.23 (s, 6H).

### Example 19

### 5-chloro-2-(6-(1-cyclopropyl-1H-pyrazol-4-yl)-4-((2-hydroxy-2-methylpropyl)amino)phthalazin-1-yl)-3-methylphenol (039)

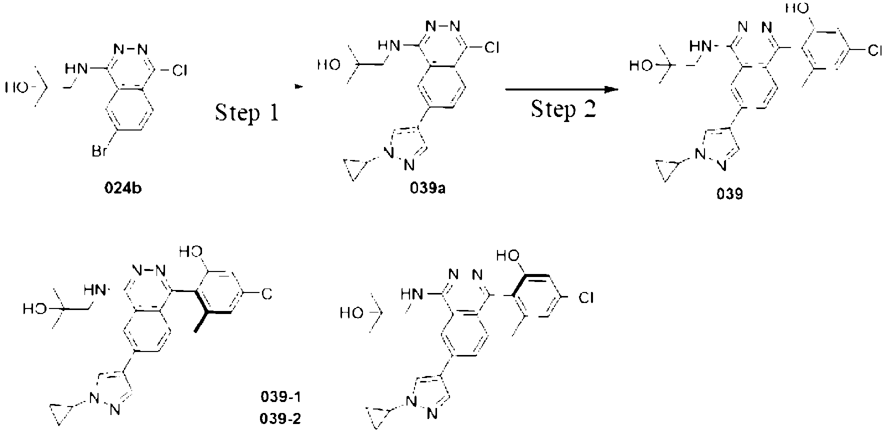

The synthetic route of Example 18 was adopted, and the raw material 1-methylpyrazole-4-boric acid pinacol ester in step 1 was replaced with 1-cyclopropylpyrazole-4-boric acid pinacol ester (60 mg, 0.26 mmol). The reaction product from step 2 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 20%-100%, flow rate: 20 mL/min) to give the title product **039** (9 mg, yield: 15.6%, a pair of axial chiral isomers).

MS m/z(ESI): 464.2(M+1).

¹H NMR (400 MHz, CD₃OD) δ 8.52 (s, 1H), 8.30(s,1H),8.08 (s, 1H), 8.04 (d, 1H), 7.43 (d, 1H), 6.90 (s, 1H), 6.83 (d, 1H), 3.75 (d, 2H), 2.03-2.00 (m, 1H), 1.98 (s, 3H), 1.34 (s, 6H), 1.19-1.15 (m, 2H), 1.13-1.08 (m, 2H).

### Example 20

### 5-chloro-2-(4-((2-hydroxy-2-methylpropyl)amino)-6-(1-methyl-1H-pyrazol-3-yl)phthalazin-1-yl)-3-methylphenol (040)

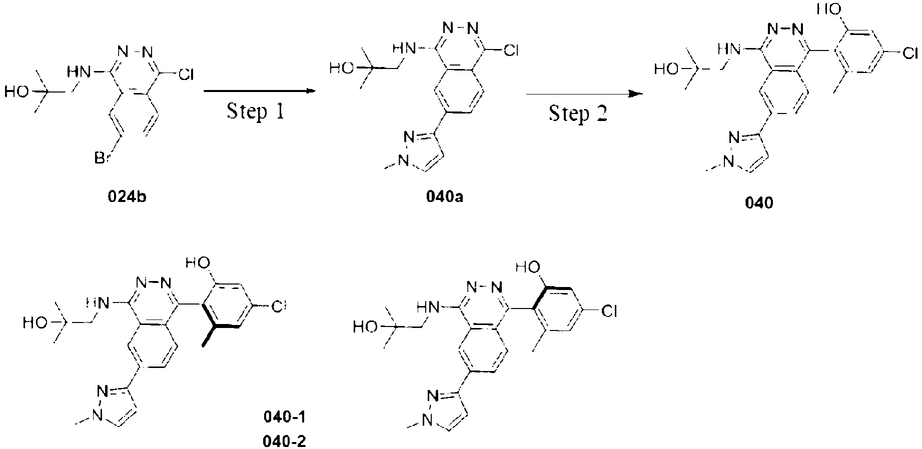

The synthetic route of Example 18 was adopted, and the raw material 1-methylpyrazole-4-boric acid pinacol ester in step 1 was replaced with 1-methylpyrazole-3-boric acid pinacol ester (60 mg, 0.29 mmol). The reaction product from step 2 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 22%-100%, flow rate: 20 mL/min) to give the title product **040** (10.3 mg, yield: 19.6%, a pair of axial chiral isomers).

MS m/z (ESI): 438.1 (M+1).

¹H NMR (400 MHz, DMSO-*d₆*) δ 8.68 (s, 1H), 8.19 (s, 1H), 8.16 (s, 1H), 7.81 (d, 1H), 7.53 (s, 1H), 7.26 (d, 1H), 6.91 (d, 1H), 6.88 (d, 1H), 6.84 (d, 1H), 3.91 (s, 3H), 3.72-3.50 (m, 2H), 1.86 (s, 3H), 1.20 (s, 6H).

### Example 21

### 4-((2-hydroxy-2-methylpropyl)amino)-1-(4-hydroxybenzo[b]thiophen-5-yl)phthalazine-6-carbonitrile (018)

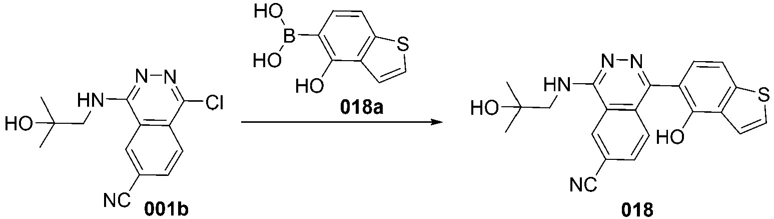

### Preparation of 4-((2-hydroxy-2-methylpropyl)amino)-1-(4-hydroxybenzothiophen-5-yl)phthalazine-6-carbonitrile (018)

Compound **001b** (86 mg. 0.44 mmol) was dissolved in a mixed solvent of 1,4-dioxane and water (3 mL, V/V = 5:1), and (4-hydroxybenzo[*b*]thiophene-5-yl)boronic acid **018a** (123 mg, 0.44 mmol, prepared by the method of Example 26 in the specification of patent application "WO2022166890A1"), 1,1-bis(diphenylphosphino)ferrocene palladium chloride (64 mg, 0.087 mmol) and sodium carbonate (11 mg, 1.3 mmol) were added, and nitrogen was replaced three times. The reaction mixture was stirred to react at 100°C for 3 h. The reaction mixture was directly concentrated under reduced pressure. The crude product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 26%-100%, flow rate: 20 mL/min) to give the title compound **018** (2.4 mg, yield: 0.02%).

MS m/z (ESI): 391.1 (M+1).

1H NMR (400 MHz, DMSO-*d₆*) δ 9.10 (s, 1H), 8.37 (s, 1H), 8.12 (d, 1H), 7.71 (s, 2H), 7.63 (m, 3H), 7.31 (d, 1H), 3.69 (d, 2H),1.24 (s, 6H).

### Example 22

### 1-(2-fluoro-4-hydroxybenzo[b]thiophen-5-yl)-4-((2-hydroxy-2-methylpropyl)amino)phthalazine-6-carbonitrile (019)

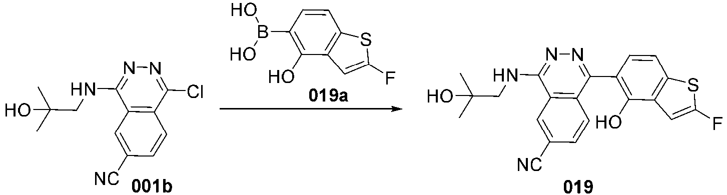

The synthetic route of Example 21 was used. The raw material (4-hydroxybenzo[*b*]thiophene-5-yl)boronic acid **018a** in step 1 was replaced with (2-fluoro-4-hydroxybenzo[*b*]thiophen-5-yl)boronic acid **019a** (60 mg, 0.28 mmol, prepared by the method of Example 44 in the specification of patent application "WO2022166890A1"). The final product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 13-min gradient, gradient ratio: acetonitrile phase 22%-100%, flow rate: 20 mL/min) to give the title product **019** (2.3 mg, yield: 2.1%).

MS m/z (ESI): 409.1 (M+1).

¹H NMR (400 MHz, DMSO-*d₆*) δ 10.06 (s, 1H), 9.34 (s, 1H), 8.31 (d,1H), 7.75 (d, 1H), 7.60 (d, 1H), 7.33-7.31 (m, 2H), 3.71 (d, 2H), 1.28 (s, 6H).

### Example 23

### (S)- 2-((R)-3-((1-(4-chloro-2-hydroxyphenyl)pyrido[3,4-d]pyridazin-4-yl)amino)piperidin-1-yl)propionamide

### (R)- 2-((R)-3-((1-(4-chloro-2-hydroxyphenyl)pyrido[3,4-d]pyridazin-4-yl)amino)piperidin-1-yl)propionamide

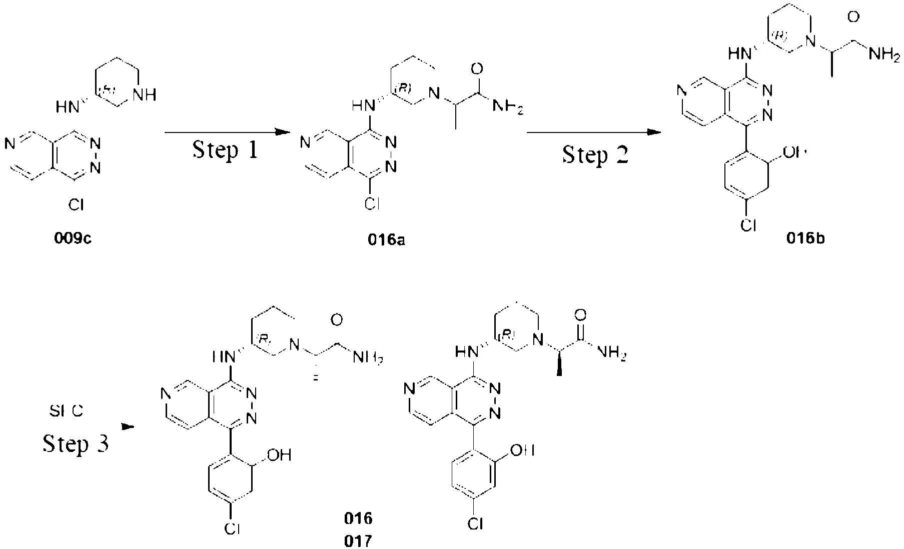

### Step 1 Preparation of 2-((R)-3-((1-chloropyrido[3,4-d]pyridazin-4-yl)amino)piperidin-1-1-yl)propionamide (016a)

Compound **009c** (430 mg, 1.63 mmol) was dissolved in *N,N*-dimethylformamide (5 mL), and then 2-bromopropionamide (322 mg, 2.12 mmol), triethylamine (495 mg, 4.89 mmol) and sodium iodide (245 mg, 1.63 mmol) were added in sequence. The reaction mixture was allowed to react at 50°C for 12 h. After the reaction was completed, the mixture was dried by rotary evaporation to remove the solvent to give a crude product. For the crude product, the resulting residue was purified by silica gel column chromatography with eluent system A to give the title compound **016a** (400 mg, yield: 73%).

MS m/z (ESI): 335.1 (M+1).

### Step 2 Preparation of 2-((R)-3-((1-(4-chloro-2-hydroxyphenyl)pyrido[3,4-d]pyridazin-4-yl)amino)piperidin-1-yl)propionamide (016b)

Compound **016a** (40 mg, 0.12 mmol) was dissolved in a mixed solvent of 1,4-dioxane and water (3.6 mL, V/V = 5:1), and 2-hydroxy-4-chlorophenylboronic acid (25 mg, 0.14 mmol), sodium carbonate (38 mg, 0.36 mmol) and 1,1'-bis(diphenylphosphino)ferrocene palladium chloride (17 mg, 0.02 mmol) were added. The reaction mixture was allowed to react with stirring under nitrogen protection at 100°C for 4 h. After the reaction was completed, the reaction mixture was filtered and the filtrate was concentrated under reduced pressure to give a crude product. For the crude product, the resulting residue was purified by silica gel column chromatography with eluent system A to give the title compound **016b** (5.2 mg, yield: 10.1%).

MS m/z (ESI): 427.1 (M+1).

### Step 3 Preparation of (S)- 2-((R)-3-((1-(4-chloro-2-hydroxyphenyl)pyrido[3,4-d]pyridazin-4-yl)amino)piperidin-1-yl)propionamide (016)

### Preparation of (R)- 2-((R)-3-((1-(4-chloro-2-hydroxyphenyl)pyrido[3,4-d]pyridazin-4-yl)amino)piperidin-1-yl)propionamide (017)

Crude compound **016b** (20 mg) was purified by preparative SFC chromatography (SFC 150 column: Daicel CHIRALCEL AZ, 250mm× 30 mm I.D., 10µm; mobile phase 1: carbon dioxide; mobile phase 2: ethanol (0.2% aqueous ammonia); 6-min gradient, gradient ratio: carbon dioxide: ethanol phase = 65/35, flow rate: 2 mL/min) to give the title compound **016** (2.43 mg, shorter retention time, yield: 12%) and the title compound **017** (2.32 mg, longer retention time, yield: 11.6%).

### Mono-configurational compound 016 (shorter retention time)

MS m/z (ESI): 427.1 (M+1).

¹H NMR (400 MHz, DMSO-*d₆*) δ 10.29 (s, 1H), 9.78 (s, 1H), 8.84 (d, 1H), 7.61 (d, 1H), 7.35-7.33 (m, 2H), 7.29(d, 1H),7.04-7.02 (m, 3H), 4.51 (s, 1H), 3.15-3.07 (m, 2H), 2.68 (d, 1H), 2.35 (t, 1H), 2.26-2.21(m, 1H), 2.02-1.98(m, 1H), 1.82-1.77(m, 1H), 1.65-1.48 (m, 2H),1.11 (d, 3H).

### Mono-configurational compound 017 (longer retention time)

MS m/z (ESI): 427.1 (M+1).

¹H NMR (400 MHz, DMSO-*d₆*) δ 10.37 (s, 1H), 9.78 (s, 1H), 8.85 (d, 1H), 7.61 (d,, 1H), 7.35-7.33 (d,2H), 7.29(d, 1H),7.04-7.01 (m, 3H), 4.51 (s, 1H), 3.12-3.06 (m, 2H), 2.73-2.67(m, 1H), 2.33-2.23 (m,2H), 2.01-1.97(m, 1H), 1.82-1.78(m, 1H),1.63-1.51(m, 2H), 1.11 (d, 3H).

### Example 24

### 5-chloro-2-(4-((2-hydroxy-2-methylpropyl)amino)-6-(1H-pyrazol-1-yl)phthalazin-1-yl)-3-methylphenol (026)

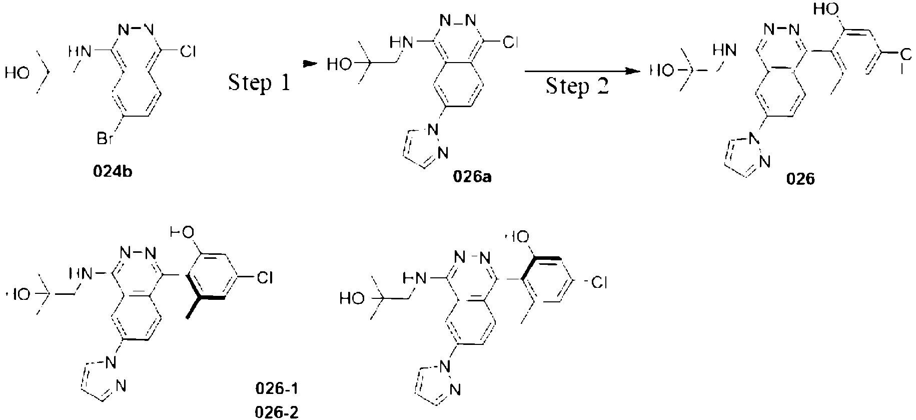

### Step 1 Preparation of 1-((4-chloro-7-(1H-pyrazol-1-yl)phthalazin-1-yl)amino)-2-methylpropan-2-ol (026a)

Compound **024b** (30 mg, 0.09 mmol) was dissolved in *N,N*-dimethylformamide (5 mL), and then pyrazole (6 mg, 0.09 mmol), cuprous oxide (2.6 mg, 0.02 mmol), cesium carbonate (88.66 mg, 0.27 mmol), and ferrous triacetylacetonate (4.61 mg, 0.02 mmol) were added, and nitrogen gas was blown into the reaction flask for protection. The reaction mixture was allowed to react at 110°C under nitrogen protection for 16 h. The reaction mixture was filtered, and the filtrate was concentrated under reduced pressure. For the crude product, the resulting residue was purified by silica gel column chromatography with eluent system A to give the title compound **026a** (30 mg, yield: 86.8%).

MS m/z (ESI): 318.1 (M+1).

### Step 2 Preparation of 5-chloro-2-(4-((2-hydroxy-2-methylpropyl)amino)-6-(1H-pyrazol-1-yl)phthalazin-1-yl)-3-methylphenol (026)

Compound **026a** (30 mg, 0.09 mmol) and (4-chloro-2-hydroxy-6-methylphenyl)boric acid (19.36 mg, 0.10 mmol) were dissolved in a mixed solvent of 1,4-dioxane and water (3.3 mL, V/V = 10:1), potassium carbonate (39.14 mg 0.28 mmol) was added, and 1,1'-bis(diphenylphosphino)ferrocene palladium dichloride (13.7 mg, 0.02 mmol) was added thereto, and the reaction system was stirred to react at 100°C under nitrogen protection for 2 h. After the reaction was completed, the reaction mixture was filtered, the filtrate was collected, and concentrated under reduced pressure to give a crude product. The crude product was purified by preparative high performance liquid chromatography (GILSON Prep LC with UV detector, chromatographic column: Xtimate 10 µm C18 250 x 30 mm; mobile phase 1: water (containing 0.1% formic acid); mobile phase 2: acetonitrile; 15-min gradient, gradient ratio: acetonitrile phase 22%-95%, flow rate: 50 mL/min) to give the title compound **026** (5 mg, yield: 12.5%, a pair of axial chiral isomers).

MS m/z (ESI): 424.1 (M+1).

¹H NMR (400 MHz, DMSO-*d₆*) δ 9.90 (s, 1H), 8.78 (d, 1H), 8.69 (d, 1H), 8.31 (dd, 1H), 7.88 (d, 1H), 7.51 (t, 1H), 7.41 (d, 1H), 6.93 (d, 1H), 6.88 (d, 1H), 6.69 (t,1H), 3.71 (m, 2H), 1.91 (s, 3H), 1.24 (s, 6H).

### Example 25

### 2-((R)-3-((1-(4-chloro-2-hydroxyphenyl)pyrido[3,4-d]pyridazin-4-yl)amino)piperidin-1-yl)-N-cyclopropylpropionamide (041)

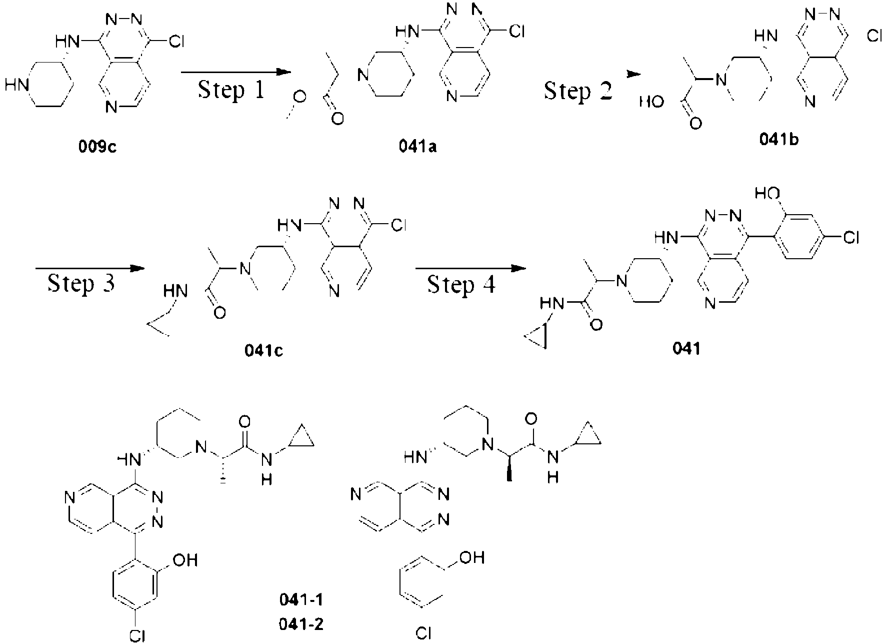

### Step 1 Preparation of methyl 2-((R)-3-((1-chloropyrido[3,4-d]pyridazin-4-yl)amino)piperidin-1-yl)propionate (041a)

Compound **009c** (180 mg, 0.60 mmol) and methyl 2-bromopropionate (100 mg, 0.60 mmol) were dissolved in ultra-dry acetonitrile (10 mL), potassium carbonate (166 mg, 1.2 mmol) was added, and the reaction system was allowed to react with stirring at 80°C for 16 h, then extracted with ethyl acetate (10 mLx3), and washed with saturated brine. The organic phase was collected, and dried by rotary evaporation to remove the solvent to give a crude product. For the crude product, the resulting residue was purified by silica gel column chromatography with eluent system B, to give the title compound **041a** (180 mg, yield: 86%).

MS m/z(ESI): 350.2(M+1).

### Step 2 Preparation of 2-((R)-3-((1-chloropyrido[3,4-d]pyridazin-4-yl)amino)piperidin-1-yl)propionic acid (041b)

Compound **041a** (180 mg, 0.50 mmol) was dissolved in a mixed solvent of tetrahydrofuran and water (2 mL, V/V = 1:1), and lithium hydroxide (37 mg, 1.50 mmol) was added. The reaction mixture was allowed to react with stirring at room temperature for 1 h, and then dried by rotary evaporation to remove the solvent to give a crude product, compound **041b** (150 mg), which was directly used for the next step without purification.

MS m/z (ESI): 336.1 (M+1).

### Step 3 Preparation of 2-((R)-3-((1-chloropyrido[3,4-d]pyridazin-4-yl)amino)piperidin-1-yl)-N-cyclopropylpropionamide (041c)

Compound **041b** (170 mg, 0.50 mmol) and cyclopropylamine (29 mg, 0.50 mmol) were dissolved in tetrahydrofuran solution (5 mL), 2-(7-azobenzotriazole)-*N*,*N*,*N*',*N*'-tetramethyluronium hexafluorophosphate (231 mg, 0.6 mmol) and *N,N*-diisopropylethylamine (131 mg, 1.00 mmol) were added, and the mixture was stirred at room temperature for 1 h. After the reaction was completed, the solvent was then removed by rotary evaporation to give a crude product. For the crude product, the resulting residue was purified by silica gel column chromatography with eluent system A, to give the title compound **041c** (180 mg, yield: 95%).

MS m/z (ESI): 375.2 (M+1).

### Step 4 Preparation of 2-((R)-3-((1-(4-chloro-2-hydroxyphenyl)pyrido[3,4-d]pyridazin-4-yl)amino)piperidin-1-yl)-N-cyclopropylpropionamide (041)

Compound **041c** (180 mg, 0.48 mmol) was dissolved in a mixed solvent of dioxane and water (5.5 mL, V/V = 10:1), (4-chloro-2-hydroxyphenyl)boric acid (91 mg, 0.53 mmol), [1,1'-bis(diphenylphosphino)ferrocene]palladium (II) dichloride (70 mg, 0.10 mmol) and sodium carbonate (112 mg, 0.96 mmol) were added, and the reaction system was stirred to react at 100°C for 1 h. After the reaction was completed, the reaction mixture was filtered, the filtrate was concentrated under reduced pressure. The crude product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 10-min gradient, gradient ratio: acetonitrile phase 18%-28%, flow rate: 20 mL/min) to give the title compound **041** (5.34 mg, yield: 2%, mixture of a pair of diastereomers).

MS m/z (ESI): 467.2 (M+1).

¹H NMR (400 MHz, DMSO-*d₆*) δ 9.78 (d, 1H), 8.85 (d, 1H), 8.29 (s, 1H), 7.82 (dd, 1H), 7.58 (d, 1H), 7.33 (dd, 1H), 7.29 (d, 1H), 7.03 (d, 2H), 4.50-4.48 (m, 1H), 3.06-3.00 (m, 2H), 2.68-2.65(m, 1H), 2.36-2.29 (m, 2H), 2.24-2.17 (m, 1H), 2.00-1.94 (m, 1H), 1.87-1.70 (m, 1H), 1.66-1.49 (m, 2H), 1.09 (t, 3H), 0.59-0.50 (m, 2H), 0.44-0.33 (m, 2H).

### Example 26

### 2-(R)-3-((1-(4-chloro-2-hydroxyphenyl)pyrido[3,4-d]pyridazin-4-yl)amino)piperidin-1-yl)-1-(pyrrolidin-1-yl)propan-1-one (042)

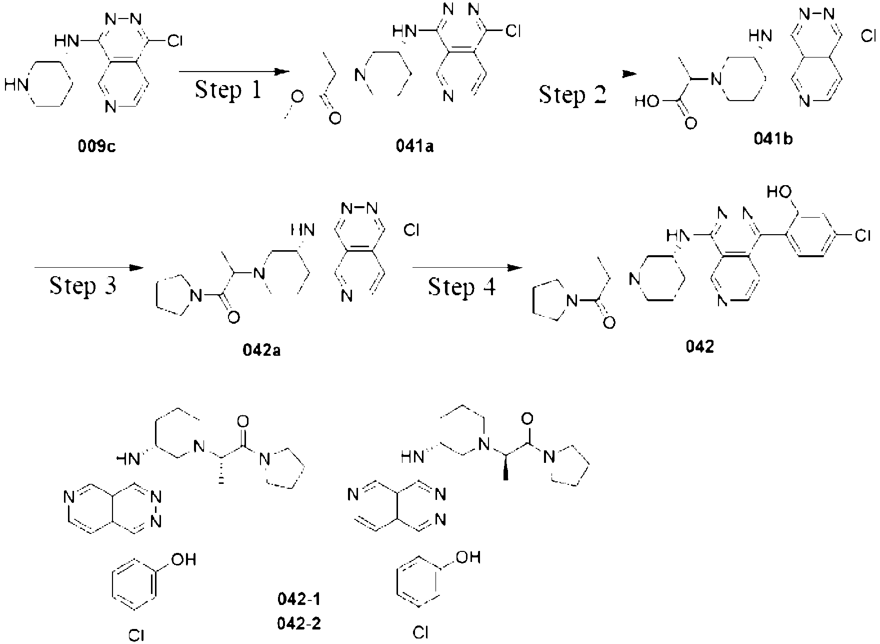

The synthetic route of Example 25 was adopted, and the raw material cyclopropylamine in step 3 was replaced with tetrahydropyrrole (60 mg, 0.84 mmol). The final product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 20%-40%, flow rate: 20 mL/min) to give the title product **042** (5 mg, yield: 11.7%).

MS m/z (ESI): 481.2 (M+1).

¹H NMR (400 MHz, CD₃OD) δ 9.71 (d, 1H), 8.84 (d, 1H), 7.49 (d, 1H), 7.36 (dd, 1H), 7.03 (d, 2H), 4.62-4.58 (m, 1H), 3.87-3.79 (m, 1H), 3.65-3.59 (m, 1H), 3.58-3.52 (m, 1H), 3.50-3.46 (m, 1H), 3.45-3.39 (m, 2H), 2.86-2.69 (m, 1H), 2.68-2.60 (m, 1H), 2.60-2.50 (m, 1H), 1.97-1.65 (m, 8H), 1.26 (dd, 3H).

### Example 27

### (R)-2-(3-((1-(4-chloro-2-hydroxyphenyl)pyrido[3,4-d]pyridazin-4-yl)amino)piperidin-1-yl)-N-cyclopropyl-2-methylpropionamide (043)

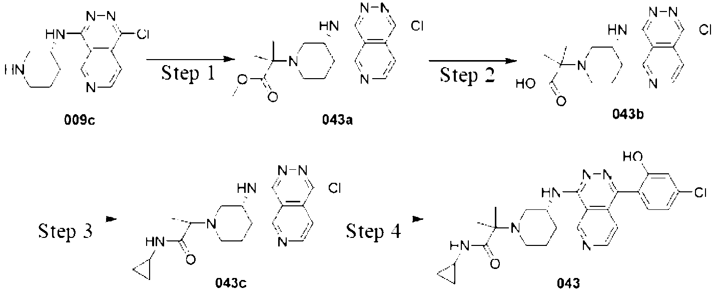

The synthetic route of Example 25 was adopted, and the raw material methyl 2-bromopropionate in step 1 was replaced with ethyl 2-bromo-2-methylpropionate (300 mg, 1.54 mmol). The final product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 25%-45%, flow rate: 20 mL/min) to give the title product **043** (8 mg, yield: 12.1%).

MS m/z (ESI): 481.2 (M+1).

¹H NMR (400 MHz, DMSO-*d₆*) δ 10.27 (s, 1H), 9.80 (s, 1H), 8.85 (d, 1H), 7.69 (d, 1H), 7.54 (d, 1H), 7.35-7.33 (m, 1H), 7.29 (d, 1H), 7.04-7.02 (m, 2H), 4.57-4.53 (m, 1H), 2.89-2.93 (m, 1H), 2.33-2.22 (m, 3H), 2.02-1.95 (m, 1H), 1.84-1.82 (m, 1H), 1.66-1.55 (m, 2H), 1.47-1.27 (m, 1H), 1.08 (d, 6H), 0.54-0.47 (m, 2H), 0.44-0.34(m, 2H).

### Example 28

### 1-(1-(4-chloro-2-hydroxyphenyl)-4-((2-hydroxy-2-methylpropyl)amino)phthalazin-6-yl)ethan-1-one (057)

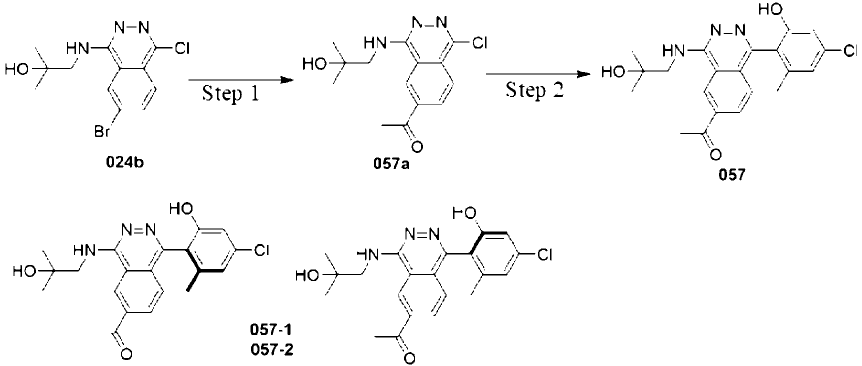

### Step 1 Preparation of 1-(1-chloro-4-((2-hydroxy-2-methylpropyl)amino)phthalazin-6-yl)ethan-1-one (057a)

Compound **024b** (100 mg, 0.30 mmol) was dissolved in ultra-dry dioxane (10 mL), and bis(triphenylphosphine) palladium dichloride (42 mg, 0.06 mmol) and tributyl(1-ethoxyvinyl)stannane (109 mg, 0.30 mmol) were added. The mixture was stirred to react at 90°C for 30 min under nitrogen protection. After the reaction was completed, aqueous potassium fluoride solution (10 mL) was added dropwise to quench the reaction, and the mixture was stirred at room temperature for 10 min. The mixture was then extracted with ethyl acetate (15 mLx3). The organic phase was collected and the solvent was removed by rotary evaporation to give a crude product. The crude product was dissolved in tetrahydrofuran (6 mL), concentrated hydrochloric acid (0.1 mL, 12 mol/L) was added, and the mixture was stirred at room temperature for 5 min. After the reaction was completed, the pH of the system was adjusted to neutral using saturated sodium bicarbonate solution, and then the reaction mixture was extracted with ethyl acetate (10 mLx3), and washed with saturated brine, the organic phase was collected, and the solvent was removed by rotary evaporation to give a crude product. For the crude product, the resulting residue was purified by silica gel column chromatography with eluent system B, to give the title compound **057a** (40 mg, yield: 45%).

MS m/z(ESI): 294.0(M+1).

### Step 2 Preparation of 1-(1-(4-chloro-2-hydroxy-6-methylphenyl)-4-((2-hydroxy-2-methylpropyl)amino)phthalazin-6-yl)ethan-1-one (057)

Compound **057a** (40 mg, 0.13 mmol)was dissolved in a mixed solvent of dioxane and water (2.2 mL, V/V = 1:1). (4-chloro-2-hydroxy-6-methylphenyl)boric acid (30 mg, 0.16 mmol), 1,1'-bis(diphenylphosphino)ferrocene palladium dichloride (20 mg, 0.03 mmol) and potassium carbonate (9 mg, 0.27 mmol) were added. The reaction system was stirred to react under nitrogen protection at 100°C for 2 h. After the reaction was completed, the reaction mixture was filtered, the filtrate was concentrated under reduced pressure to give a crude product. The crude product was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 10-min gradient, gradient ratio: acetonitrile phase 32%-100%, flow rate: 20 mL/min) to give the title compound **057** (1.53 mg, yield: 3%).

MS m/z (ESI): 400.1 (M+1).

¹H NMR (400 MHz, CD₃OD) δ 8.97 (d, 1H), 8.33 (dd, 1H), 7.54 (d, 1H), 6.99-6.78 (m, 2H), 3.83-3.69 (m, 2H), 2.77 (s, 3H), 1.97 (s, 3H), 1.34 (s, 6H).

### Example 29

### 5-chloro-2-(6-(1-ethyl-1H-pyrazol-4-yl)-4-((2-hydroxy-2-methylpropyl)amino)phthalazin-1-yl)-3-methylphenol (051)

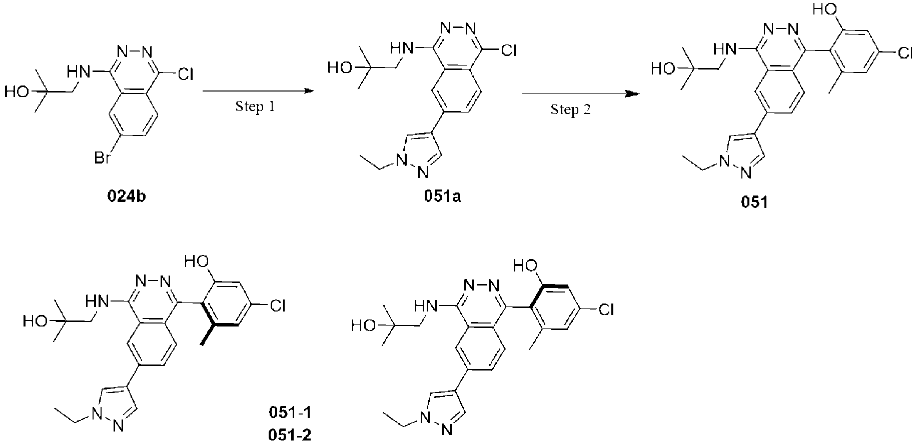

The synthetic route of Example 18 was adopted, and the raw material 1-methylpyrazole-4-boric acid pinacol ester in step 1 was replaced with 1-ethylpyrazole-4-boric acid pinacol ester (50 mg, 0.23 mmol). The reaction product from step 2 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 18%-100%, flow rate: 20 mL/min) to give the title product **051** (2.5 mg, yield: 12.6%, a pair of axial chiral isomers).

MS m/z (ESI): 452.1 (M+1).

¹H NMR (400 MHz, CD₃OD) δ 8.54 (d, 1H), 8.26 (s, 1H), 8.10 (s, 1H), 8.05 (dd, 1H), 7.45 (d, 1H), 6.91 (s, 1H), 6.84 (d, 1H), 4.26 (q, 2H), 3.74 (d, 2H), 1.99 (s, 3H), 1.52 (t, 3H), 1.35 (s, 6H).

### Example 30

### 5-chloro-2-(4-((2-hydroxy-2-methylpropyl)amino)-6-(2H-1,2,3-triazol-2-yl)phthalazin-1-yl)-3-methylphenol (028)

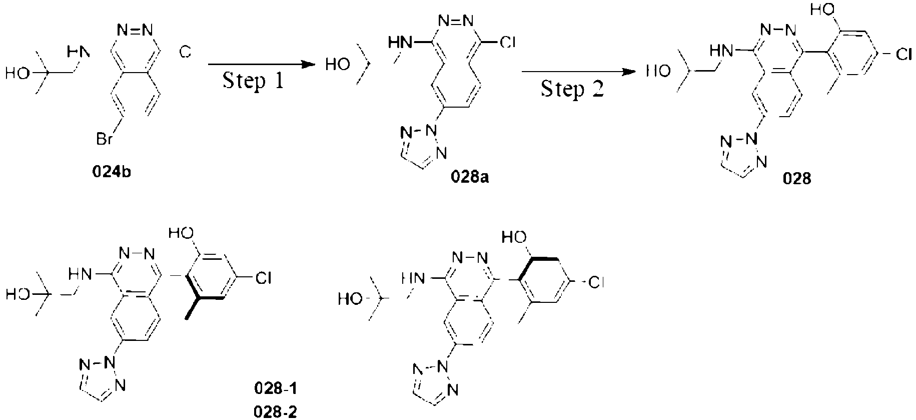

The synthetic route of Example 24 was adopted, and the pyrazole as the raw material in step 1 was replaced with 1,2,3-triazole (20 mg, 0.29 mmol). The reaction product from step 2 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 25%-100%, flow rate: 20 mL/min) to give the title product **028** (2 mg, yield: 10.6%, a pair of axial chiral isomers).

MS m/z (ESI): 425.1 (M+1).

¹H NMR (400 MHz, CD₃OD) δ 9.02 (d, 1H), 8.57(dd, 1H), 8.05 (s, 2H), 7.64 (d, 1H), 6.92 (d, 1H), 6.85 (d, 1H), 3.76 (d, 2H), 2.01 (s, 3H), 1.34 (s, 6H).

### Example 31

### 5-chloro-2-(4-((2-hydroxy-2-methylpropyl)amino)-6-(1H-1,2,3-triazol-1-yl)phthalazin-1-yl)-3-methylphenol (058)

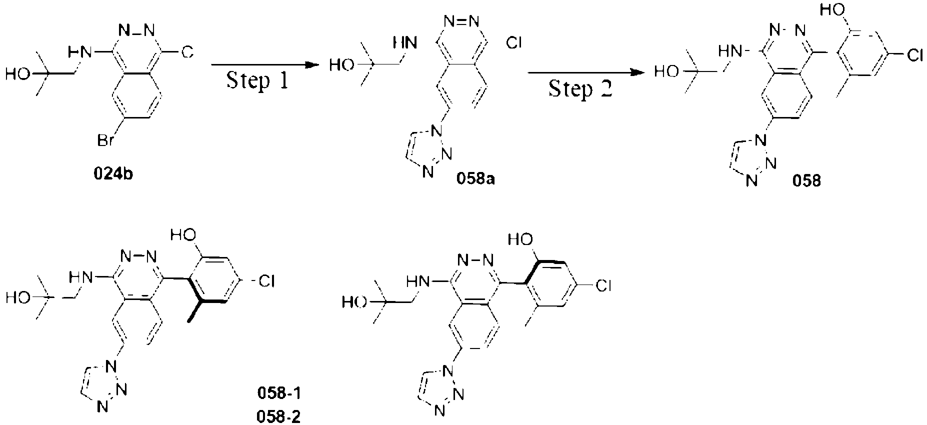

The synthetic route of Example 24 was adopted, and the pyrazole as the raw material in step 1 was replaced with 1,2,3-triazole (20 mg, 0.29 mmol). The reaction product from step 2 was purified by preparative high performance liquid chromatography (Waters MS-triggered Prep-LC with SQD2 detector, chromatographic column: Xbridge 5µm C18 150 x 19 mm; mobile phase 1: water (containing 0.1% formic acid); mobile phase 2: acetonitrile; 15-min gradient, gradient ratio: acetonitrile phase 25%-100%, flow rate: 20 mL/min) to give the title product **058** (3 mg, yield: 15.9%, a pair of axial chiral isomers).

MS m/z (ESI): 425.1 (M+1).

¹H NMR (400 MHz, CD₃OD) δ 9.28 (d, 1H), 8.79 (d, 1H), 8.60 (dd, 1H), 8.04 (d, 1H), 7.87 (d, 1H), 6.99 (d, 1H), 6.89 (d, 1H), 3.77 (d, 2H), 2.10 (s, 3H), 1.42 (s, 6H).

### Example 32

### (S)-3-((4-(4-chloro-2-hydroxyphenyl)-7-(prop-1-yn-1-yl)phthalazin-1-yl)amino)propan-1,2-diol (062)

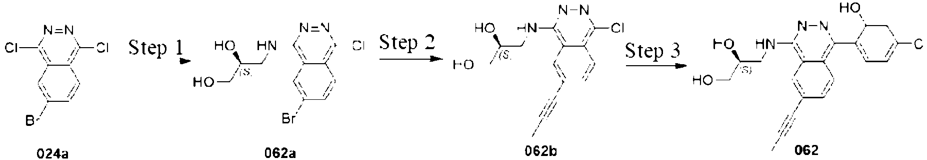

### Step 1 Preparation of (S)-3-((7-bromo-4-chlorophthalazin-1-yl)amino)propan-1,2-diol (062a)

Compound **024a** (4 g, 0.014 mol) and (S)-3-aminopropan-1,2-diol (1.31 g, 0.014 mmol) were dissolved in ethanol (25 mL), and the mixture was heated to 80°C to react for 4 h. After the reaction was completed, the reaction mixture was concentrated, and the resulting residue was purified by silica gel column chromatography with eluent system A to give the title compound **062a** (1.2 g, yield: 25%).

MS m/z (ESI): 332.1 (M+1)+.

### Step 2 Preparation of (S)-3-((4-chloro-7-(propyl-1-yn-1-yl)phthalazin-1-yl)amino)propan-1,2-diol (062b)

Compound **062a** (600 mg, 1.98 mmol), 1-(trimethylsilyl)propyne (178 mg, 1.59 mmol), cuprous iodide (151 mg, 0.79 mmol), cesium carbonate (646 mg, 1.98 mmol), and bis(triphenylphosphine) palladium dichloride (278 mg, 0.39 mmol) were dissolved in a mixed solvent of *N*,*N-*dimethylacetamide and water (22 mL, V/V = 10:1), and the mixture was heated to 100°C to react for 4 h. After the reaction was completed, to the reaction mixture was added saturated ammonium chloride solution, the mixture was extracted with ethyl acetate (10 mL x 3), filtered and concentrated to give a crude product, and the resulting residue was purified by silica gel column chromatography with eluent system A to give the title compound **062b** (70 mg, yield: 13.5%).

MS m/z (ESI): 292.1 (M+1).

### Step 3 Preparation of (S) -3-((4-(4-chloro-2-hydroxyphenyl)-7-(prop-1-yn-1-yl)phthalazin-1-yl)amino)propan-1,2-diol (062)

Compound **062b** (50 mg, 0.17 mmol) was dissolved in a mixed solvent of dioxane and water (2.2 mL, V/V = 10:1), followed by the addition of (4-chloro-2-hydroxyphenyl)boronic acid (30 mg, 0.17 mmol), 1,1'-bis(diphenylphosphino)ferrocene palladium dichloride (12 mg, 0.02 mmol) and potassium carbonate (47 mg, 0.34 mmol), and the reaction mixture was stirred to react at 100°C under nitrogen protection for 1 h. After the reaction was completed, the reaction mixture was filtered, and the filtrate was concentrated under reduced pressure. The crude product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% NH₃); mobile phase 2: acetonitrile; 15-min gradient, gradient ratio: acetonitrile phase 15%-100%, flow rate: 25 mL/min) to give the title compound **062** (4.4 mg, yield: 6.7%).

MS m/z (ESI): 384.1 (M+1)⁺.

¹H NMR (400 MHz, CD₃OD) δ 8.25 (s, 1H), 7.72 (dd, 1H), 7.55 (d, 1H), 7.29 (d, 1H), 7.01 (d, 1H), 6.99 (s, 2H), 4.02-3.95 (m, 1H), 3.85-3.79 (m, 1H), 3.75-3.69 (m, 1H), 3.61 (d, 2H), 2.10 (s, 3H).

### Example 33

### (2S)-3-((4-(4-chloro-2-fluoro-6-hydroxyphenyl)-7-(prop-1-yn-1-yl)phthalazin-1-yl)amino)propan-1,2-diol (063)

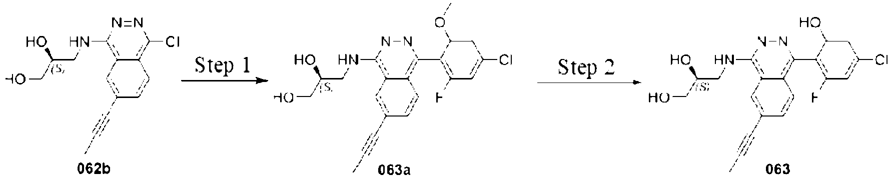

### Step 1 Preparation of (2S)-3-((4-(4-chloro-2-fluoro-6-methoxyphenyl)-7-(prop-1-yn-1-yl)phthalazin-1-yl)amino)propan-1,2-diol (063a)

Compound **062b** (80 mg, 0.27 mmol), 2-(4-chloro-2-fluoro-6-methoxyphenyl)-4,4,5,5-tetramethyl-1,3,2-dioxaborolane (86.4 mg, 0.30 mmol, prepared by the synthesis method disclosed in patent "WO2023275366A1"), chloro(2-dicyclohexylphosphino-2',4',6'-triisopropyl-1,1'-biphenyl)[2-(2'-amino-1,1'-biphenyl)]palladium(II) (21.6 mg, 0.027 mmol) and potassium carbonate (113.7 mg, 0.82 mmol) were dissolved in a mixed solvent of 1,4-dioxane and water (1.1 mL, V/V=10:1), and the mixture was heated to 120°C to react under nitrogen protection for 1 h. After the reaction was completed, the reaction mixture was filtered and concentrated, and the resulting residue was purified by silica gel column chromatography with eluent system A to give the title compound **063a** (20 mg, yield: 18%).

MS m/z (ESI): 416.1 (M+1).

### Step 2 Preparation of (2S)-3-((4-(4-chloro-2-fluoro-6-hydroxyphenyl)-7-(prop-1-yn-1-yl)phthalazin-1-yl)amino)propan-1,2-diol (063)

Compound **063a** (20 mg, 0.048 mmol) and lithium iodide (64.4 mg, 0.471 mmol) were dissolved in 2,4,6-trimethylpyridine (0.5 mL), and the reaction mixture was stirred to react at 130°C for 1 h. After the reaction was completed, the crude product was purified by preparative high performance liquid chromatography (Waters MS triggered Prep-LC with Acquity QDA detector, chromatographic column Welch 10 µm C18 250 x 21.2 mm; mobile phase 1: water (containing 0.1% ammonia); mobile phase 2: acetonitrile; 15-min gradient, gradient ratio: acetonitrile phase 15%-100%, flow rate: 25 mL/min) to give the title compound **063** (1.02 mg, yield: 5.3%).

MS m/z (ESI): 402.1 (M+1).

¹H NMR (400 MHz, CD₃OD) δ 8.13 (s, 1H), 7.60 (dd, 1H), 7.48 (d, 1H), 6.46 (d, 1H), 6.25 (dd, 1H),5.25 (t, 1H), 3.91-3.85 (m, 1H), 3.75-3.65 (m, 2H), 3.52 (d, 2H), 2.01 (s, 3H).

### Example 34

### (2S)-3-((4-(2-fluoro-6-hydroxy-4-(trifluoromethyl)phenyl)-7-(prop-1-yn-1-yl)phthalazin-1-yl)amino)propan-1,2-diol (064)

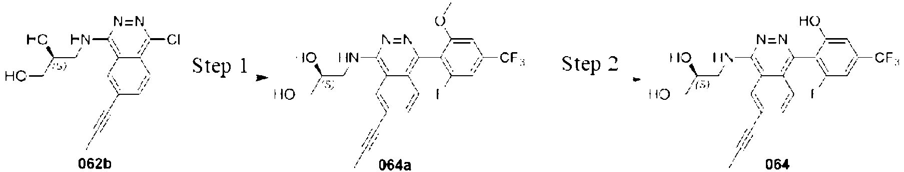

### Step 1 Preparation of (2S)-3-((4-(2-fluoro-6-methoxy-4-(trifluoromethyl)phenyl)-7-(prop-1-yn-1-yl)phthalazin-1-yl)amino)propan-1,2-diol (064a)

Compound **062b** (160 mg, 0.55 mmol), 2-(2-fluoro-6-methoxy-4-(trifluoromethyl)phenyl)-4,4,5,5-tetramethyl-1,3,2-dioxaborolane (211 mg, 0.66 mmol, prepared by the synthesis method disclosed in patent "WO2023275366A1"), chloro(2-dicyclohexylphosphino-2',4',6'-triisopropyl-1,1'-biphenyl)[2-(2'-amino-1,1'-biphenyl)]palladium(II) (43 mg, 0.055 mmol) and potassium carbonate (227 mg, 1.645 mmol) were dissolved in a mixed solvent of 1,4-dioxane and water (3.3 mL, V/V=10:1), and the reaction system was heated to 100°C to react under nitrogen protection for 1 h. After the reaction was completed, the reaction mixture was filtered and concentrated, and the resulting residue was purified by silica gel column chromatography with eluent system A to give the title compound **064a** (70 mg, yield: 28%).

MS m/z (ESI): 450.1 (M+1).

### Step 2 Preparation of (2S)-3-((4-(2-fluoro-6-hydroxy-4-(trifluoromethyl)phenyl)-7-(prop-1-yn-1-yl)phthalazin-1-yl)amino)propan-1,2-diol (064)

Compound **064a** (70 mg, 0.16 mmol) and lithium iodide (209 mg, 1.56 mmol) were dissolved in 2,4,6-trimethylpyridine (0.5 mL), and the reaction mixture was stirred to react at 130°C for 1 h. After the reaction was completed, the crude product was purified by preparative high performance liquid chromatography (Waters MS-triggered Prep-LC with QDA detector, chromatographic column Welch 10 µm C18 250 x 21.2 mm; mobile phase 1: water (containing 0.1% ammonia); mobile phase 2: acetonitrile; 15-min gradient, gradient ratio: acetonitrile phase 25%-100%, flow rate: 25 mL/min) to give the title compound **064** (20 mg, yield: 15%).

MS m/z (ESI): 436.1 (M+1).

¹H NMR (400 MHz, CD₃OD) δ 8.36 (s, 1H), 7.79 (dd, 1H), 7.45 (d, 1H), 7.11 (d, 2H), 4.06-3.99 (m, 1H), 3.85 (dd, 1H), 3.75 (dd, 1H), 3.63 (d, 2H), 2.11 (s, 3H).

### Example 35

### (S)-3-((4-(2-hydroxy-4-(trifluoromethyl)phenyl)-7-(prop-1-yn-1-yl)phthalazin-1-yl)amino)propan-1,2-diol (056)

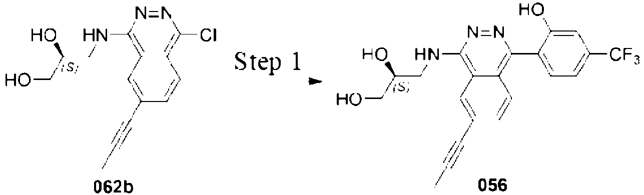

### Step 1 Preparation of (S)-3-((4-(2-hydroxy-4-(trifluoromethyl)phenyl)-7-(prop-1-yn-1-yl)phthalazin-1-yl)amino)propan-1,2-diol (056)

Compound **062b** (70 mg, 0.24 mmol), 1,1-bis(diphenylphosphino)ferrocene palladium dichloride (17.6 mg, 0.024 mmol), (4-trifluoromethyl-2-hydroxyphenyl)boric acid (54.6 mg, 0.26 mmol) and sodium carbonate (50.9 mg, 0.48 mmol) were dissolved in a mixed solvent of 1,4-dioxane and water (6 mL, V/V=5:1). Under nitrogen protection, the reaction system was heated to 90°C to react for 1 h. After the reaction was completed, the reaction mixture was filtered and concentrated. The crude product was purified by preparative high performance liquid chromatography (Waters MS-triggered Prep-LC with QDA detector, chromatographic column: Xbridge 5 µm C18 150 x 19 mm; mobile phase 1: water (containing 0.1% formic acid); mobile phase 2: acetonitrile; 15-min gradient, gradient ratio: acetonitrile phase 25%-100%, flow rate: 25 mL/min) to give the title compound **056** (11.4 mg, yield: 11.4%).

MS m/z (ESI): 418.1 (M+1).

¹H NMR (400 MHz, DMSO-*d₆*) δ 8.48 (s, 1H), 8.29 (d, 1H), 7.73 (d, 1H), 7.67 (s, 1H), 7.51 (d, 1H), 7.38 (d, 1H), 7.28 (d, 2H), 3.85 (dd, 1H), 3.70 (d, 1H), 3.54 (d, 1H), 3.42 (s, 2H), 2.13 (s, 3H).

### Example 36

### 2-(4-(((1S, 3S)-3-hydroxy-3-methylcyclobutyl)amino)-6-(prop-1-yn-1-yl)phthalazin-1-yl)-5-(trifluoromethyl)phenol (065)

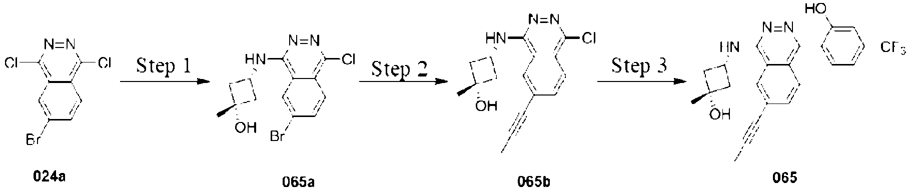

### Step 1 (1S,3S)-3-((7-bromo-4-chlorophthalazin-1-yl)amino)-1-methylcyclobutan-1-ol (065a)

Compound **024a** (1 g, 3.6 mmol) was dissolved in *N*-methylpyrrolidone (5 mL), and (1*S*,3*S*)-3-amino-1-methylcyclobutan-1-ol (0.36 g, 3.6 mmol) and *N,N*-diisopropylethylamine (2.33 g, 18 mmol) were added. The reaction was stirred at 100°C for 3 h. After the reaction was completed, the reaction mixture was washed three times with saturated brine and ethyl acetate. The organic phase was collected and concentrated by rotary evaporation. The resulting residue was purified by silica gel column chromatography with eluent system A to give the title compound **065a** (200 mg, yield: 17%).

MS m/z (ESI): 342.0 (M+1).

¹H NMR (400 MHz, DMSO-*d₆*) δ 8.80 (d, 1H), 8.14 (dd, 1H), 7.98 (d, 1H), 7.85 (d, 1H), 5.01 (s, 1H), 4.16-4.07 (m, 1H), 2.45 (ddd, 2H), 2.20-2.07 (m, 2H), 1.32 (s, 3H).

### Step 2 Preparation of (1S,3S)-3-((4-chloro-7-(prop-1-yn-1-yl)phthalazin-1-yl)amino)-1-methylcyclobutan-1-ol (065b)

Compound **065a** (50 mg, 0.15 mmol) was dissolved in a mixed solvent of *N,N-*dimethylacetamide, methanol and water (6 mL, V/V/V = 1:1:1), and 1-(trimethylsilyl)propyne ( 13.1 mg 0.12 mmol), bis(triphenylphosphine) palladium dichloride (10 mg, 0.015 mmol), copper iodide (2.8 mg, 0.015 mmol) and cesium carbonate (71 mg, 0.22 mmol) were added. Under nitrogen protection, the reaction system was stirred to react at 80°C for 2 h. After the reaction was completed, the reaction mixture was concentrated, and the resulting residue was purified by silica gel column chromatography with eluent system A to give the title compound **065b** (25 mg, yield: 57%).

MS m/z (ESI): 302.1 (M+1).

### Step 3 Preparation of 2-(4-((1S,3S)-3-hydroxy-3-methylcyclobutyl)amino)-6-(prop-1-yn-1-yl)phthalazin-1-yl)-5-(trifluoromethyl)phenol (065)

Compound **065b** (40 mg, 0.13 mmol) was dissolved in a mixed solvent of dioxane and water (5.5 mL, V/V = 10:1), and (4-trifluoromethyl-2-hydroxyphenyl)boric acid (21.8 mg, 0.11 mmol), sodium carbonate (42 mg, 0.40 mmol) and 1,1'-bis(diphenylphosphino)ferrocene palladium dichloride (9.7 mg, 0.013 mmol) were added. The reaction was stirred at 100°C under nitrogen protection for 1 h. After the reaction was completed, the system was filtered with diatomaceous earth, and the filtrate was concentrated under reduced pressure. The crude product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% formic acid); mobile phase 2: acetonitrile; 15-min gradient, gradient ratio: acetonitrile phase 25%-100%, flow rate: 25 mL/min) to give the title compound **065** (7 mg, yield: 12.4%).

MS m/z (ESI): 428.1 (M+1).

¹H NMR (400 MHz, DMSO-*d₆*) δ 8.56 (s, 1H), 7.75-7.65 (m, 2H), 7.50 (d, 1H), 7.37 (d, 1H), 7.32-7.24 (m, 2H), 5.00 (s, 1H), 4.23 (dd, 1H), 2.48-2.42 (m, 2H), 2.18 (d, 2H), 2.13 (s, 3H), 1.34 (s, 3H).

### Example 37

### 3-fluoro-2-(4-(((1S,3S)-3-hydroxy-3-methylcyclobutyl)amino)-6-(prop-1-yn-1-yl)phthalazin-1-yl)-5-(trifluoromethyl)phenol (066)

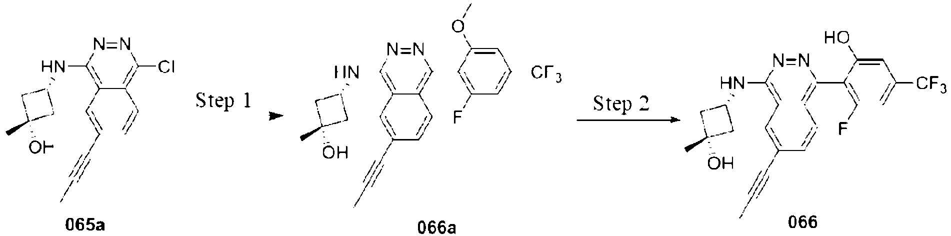

The synthetic route of Example 34 was adopted, and the raw material **062b** in step 1 was replaced with **065a** (100 mg, 0.33 mmol). The final product was purified by preparative high performance liquid chromatography (Waters MS-triggered Prep-LC with SQD2 detector, chromatographic column: Xbridge 5 µm C18 150 x 19 mm; mobile phase 1: water (containing 0.1% formic acid); mobile phase 2: acetonitrile; 15-min gradient, gradient ratio: acetonitrile phase 20%-100%, flow rate: 20 mL/min) to give the title product **066** (13.8 mg, yield: 18%).

MS m/z (ESI): 446.1 (M+1).

¹H NMR (400 MHz, CD₃OD) δ 8.55 (s, 1H), 7.85 (dd, 1H), 7.50 (d, 1H), 7.13 (d, 1H), 7.11 (s, 1H), 4.26-4.16 (m, 1H), 2.75-2.66 (m, 2H), 2.32 (dd, 2H), 2.12 (s, 3H), 1.45 (s, 3H).

### Example 38

### 2-(4-(((1R,2R)-2-hydroxycyclohexyl)amino)-6-(prop-1-yn-1-yl)phthalazin-1-yl)-5-(trifluoromethyl)phenol (061)

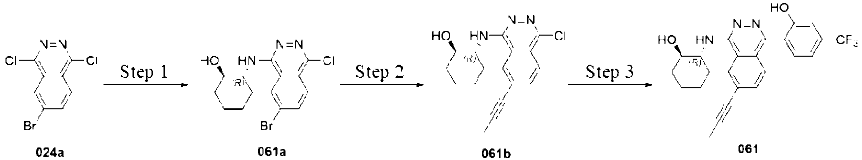

The synthetic route of Example 36 was used. The raw material (1*S*,3*S*)-3-amino-1-methylcyclobutan-1-ol in step 1 was replaced with(1*R*,2*R*)-2-aminocyclohexan-1-ol (1.3 g, 10.8 mmol). The final product was purified by preparative high performance liquid chromatography (Waters MS-triggered Prep-LC with SQD2 detector, chromatographic column: Xbridge 5 µm C18 150 x 19 mm; mobile phase 1: water (containing 0.1% formic acid); mobile phase 2: acetonitrile; 15-min gradient, gradient ratio: acetonitrile phase 29%-100%, flow rate: 20 mL/min) to give the title product **061** (10 mg, yield: 8%).

MS m/z (ESI): 442.1 (M+1).

¹H NMR (400 MHz, DMSO-*d₆*) δ 8.53 (s, 1H), 8.25 (s, 1H), 7.70 (d, 1H), 7.49 (d, 1H), 7.36 (d, 1H), 7.31-7.20 (m, 3H), 2.69-2.64 (m, 1H), 2.35-2.31 (m, 1H), 2.12 (s, 3H), 2.01-1.94 (m, 2H), 1.75-1.64 (m, 2H), 1.34-1.26 (m,, 4H).

### Example 39

### (3R,4R)-3-((4-(2-hydroxy-4-(trifluoromethyl)phenyl)-7-(prop-1-yn-1-yl)phthalazin-1-yl)amino)tetrahydro-2H-pyran-4-ol (067)

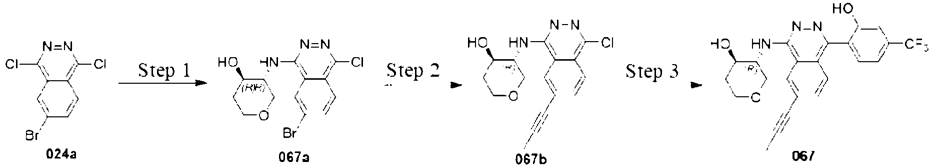

The synthetic route of Example 36 was used. The raw material (1*S*,3*S*)-3-amino-1-methylcyclobutan-1-ol in step 1 was replaced with (3*R*,4*R*)-3-aminotetrahydro-2*H*-pyran-4-ol (460 mg, 3.96 mmol). The final product was purified by preparative high performance liquid chromatography (Waters MS-triggered Prep-LC with SQD2 detector, chromatographic column: Xbridge 5 µm C18 150 x 19 mm; mobile phase 1: water (containing 0.1% formic acid); mobile phase 2: acetonitrile; 15-min gradient, gradient ratio: acetonitrile phase 28%-38%, flow rate: 20 mL/min) to give the title product **067** (1.73 mg, yield: 4%).

MS m/z (ESI): 444.2 (M+1)⁺.

¹H NMR (400 MHz, CD₃OD) δ 8.36 (s, 1H), 7.71 (d, 1H), 7.50 (d, 2H), 7.27 (d, 1H), 7.22 (s, 1H), 4.40-4.33 (m, 1H), 4.24(dd, 1H), 4.00-3.90 (m, 2H), 3.56-3.48 (m, 2H), 2.09 (s, 3H), 1.81-1.66 (m, 2H).

### Example 40

### (R)-2-(4-((1-methylpiperidin-3-yl)amino)-6-(prop-1-yn-1-yl)phthalazin-1-yl)-5-(trifluoromethyl)phenol (068)

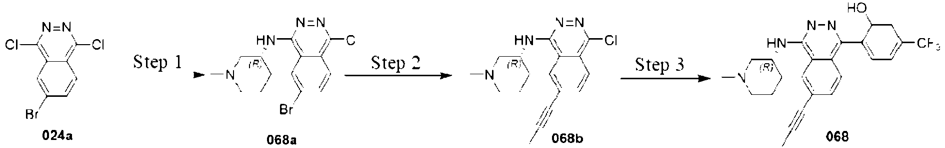

The synthetic route of Example 36 was adopted. The raw material (1*S*,3*S*)-3-amino-1-methylcyclobutan-1-ol in step 1 was replaced with (R)-1-methylpiperidin-3-amine (0.41 g, 3.6 mmol). The final product was purified by preparative high performance liquid chromatography (Waters MS triggered Prep-LC with Acquity QDA detector, chromatographic column: Xtimate C18 150 x19mm; mobile phase 1: water (containing 0.1% formic acid); mobile phase 2: acetonitrile; 15-min gradient, gradient ratio: acetonitrile phase 25%-100%, flow rate: 25 mL/min) to give the title product **068** (6 mg, yield: 7.1%).

MS m/z (ESI): 441.1 (M+1).

¹H NMR (400 MHz, DMSO-*d₆*) δ 8.53 (s, 1H), 8.35 (s, 1H), 7.72 (d, 1H), 7.51 (d, 1H), 7.37 (d, 1H), 7.29-7.27 (m, 2H), 7.22 (d,1H), 4.42-4.38(m, 1H), 2.73-2.67 (m, 2H), 2.21 (s, 3H), 2.13 (s, 3H), 1.99-1.96(m, 1H), 1.93-1.87 (m, 2H), 1.77-1.74 (m, 1H), 1.64-1.57 (m, 1H), 1.49-1.41 (m, 1H).

### Example 41

### 2-(4-((2-hydroxy-2-methylpropyl)amino)-6-(prop-1-yn-1-yl)phthalazin-1-yl)-5-(trifluoromethyl)phenol (069)

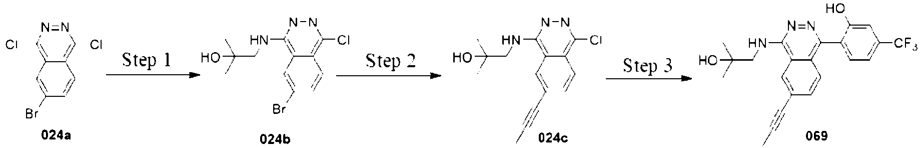

The synthetic route of Example 36 was adopted. The raw material (1*S*,3*S*)-3-amino-1-methylcyclobutan-1-ol in step 1 was replaced with 1-amino-2-methyl-2-propanol (175 mg, 2.0 mmol). The final product was purified by preparative high performance liquid chromatography (Waters MS-triggered Prep-LC with SQD2 detector, chromatographic column Xbridge 5µm C18 150 x 19 mm; mobile phase 1: water (containing 0.1% trifluoroacetic acid); mobile phase 2: acetonitrile; 15 min gradient, gradient ratio: acetonitrile phase 42%-100%, flow rate: 20 mL/min) to give the title compound **069** (2.1 mg, yield: 2.8%).

MS m/z (ESI): 416.1 (M+1).

¹H NMR (400 MHz, DMSO-*d₆*) δ 10.75 (s, 1H), 8.85 (s, 1H), 7.96 (d, 1H), 7.55 (t, 2H), 7.35 (d, 2H), 3.66 (d, 2H), 2.17 (s, 3H), 1.27 (s, 6H).

### Example 42

### 2-(4-((1R, 3S)-3-hydroxycyclohexyl)amino)-6-(prop-1-yn-1-yl)phthalazin-1-yl)-5-(trifluoromethyl)phenol (070)

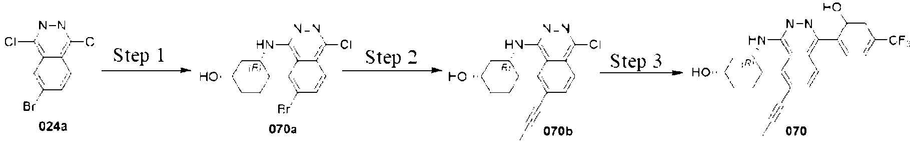

The synthetic route of Example 36 was adopted. The raw material (1*S*,3*S*)-3-amino-1-methylcyclobutan-1-ol in step 1 was replaced with (1*S*,3*R*)-3-aminocyclohexan-1-ol (0.55 g, 3.6 mmol). The final product was purified by preparative high performance liquid chromatography (Waters MS-triggered Prep-LC with QDA detector, chromatographic column: Xbridge 5 µm C18 150 x 19 mm; mobile phase 1: water (containing 0.1% formic acid); mobile phase 2: acetonitrile; 15-min gradient, gradient ratio: acetonitrile phase 30%-100%, flow rate: 25 mL/min) to give the title compound **070** (12.4 mg, yield: 10%).

MS m/z (ESI): 442.1 (M+1).

¹H NMR (400 MHz, CD₃OD) δ 8.37 (s, 1H), 7.75 (dd, 1H), 7.55 (d, 1H), 7.53 (d, 1H), 7.31 (d, 1H), 7.26 (s,1H), 4.35-4.29 (m, 1H), 3.81-3.75 (m, 1H), 2.43 (d, 1H), 2.12 (s, 3H), 2.00 (d, 1H), 1.95-1.84 (m, 1H), 1.56-1.40 (m, 3H), 1.40-1.23 (m, 2H).

### Example 43

### 3-fluoro-2-(4-(((S)-2-hydroxypropyl)amino)-6-(prop-1-yn-1-yl)phthalazin-1-yl)-5-(trifluoromethyl)phenol (071)

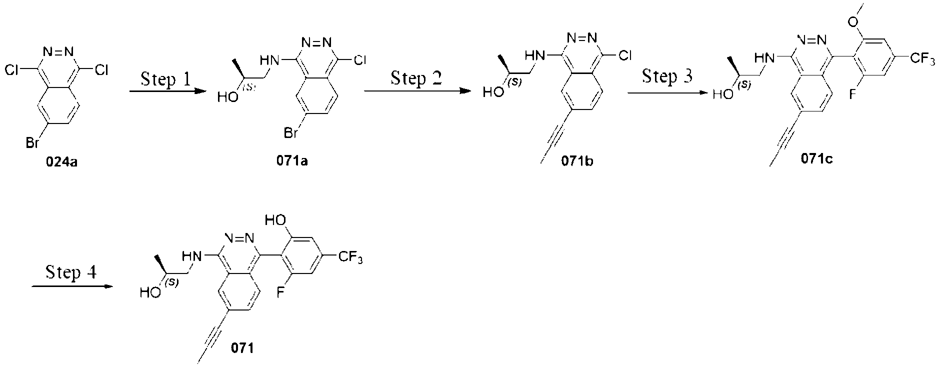

### Step 1 Preparation of (S)-1-(7-bromo-4-chlorophthalazin-1-yl)amino)propan-2-ol (071a)

Compound **024a** (2.5 g, 9 mmol) and (*S*)-1-amino-2-propanol (0.68 g, 9 mmol) were dissolved in *N*-methyl-2-pyrrolidone (25 mL), *N,N*-diisopropylethylamine (1.2 g, 9 mmol) was added, and the mixture was heated to 100°C to react for 1 h. After the reaction was completed, the reaction mixture was cooled to room temperature, diluted with water, extracted with ethyl acetate (150 mL x 3), and the organic solvent was concentrated to give a crude product. For the crude product, the resulting residue was purified by silica gel column chromatography with eluent system B to give the title compound **071a** (1.3 g, yield: 45%).

MS m/z (ESI): 316.1 (M+1).

The synthetic route of Example 34 was asopted for subsequent synthetic route. The raw material **062b** in step 1 was replaced with **071a** (500 mg, 1.59 mmol). The final product was purified by preparative high performance liquid chromatography (Waters MS-triggered Prep-LC with QDA detector, chromatographic column Xbridge 5 µm C18 150 x 19 mm; mobile phase 1: water (containing 0.1% formic acid); mobile phase 2: acetonitrile; 15-min gradient, gradient ratio: acetonitrile phase 25%-100%, flow rate: 25 mL/min) to give the title product **071** (2.05 mg, yield: 5%).

MS m/z (ESI): 420.1 (M+1).

¹H NMR (400 MHz, CD₃OD) δ 8.23 (s, 1H), 7.65 (d, 1H), 7.31 (d, 1H), 6.99-6.98 (m, 2H), 4.10 (dd, 1H), 3.64 (dd, 1H), 3.49 (dd, 1H), 2.01 (s, 3H), 1.18 (d, 3H).

### Example 44

### (R)-4-((4-(4-chloro-2-hydroxyphenyl)-7-(prop-1-yn-1-yl)phthalazin-1-yl)amino)-1-(2-hydroxyethyl)pyrrolidin-2-one (073)

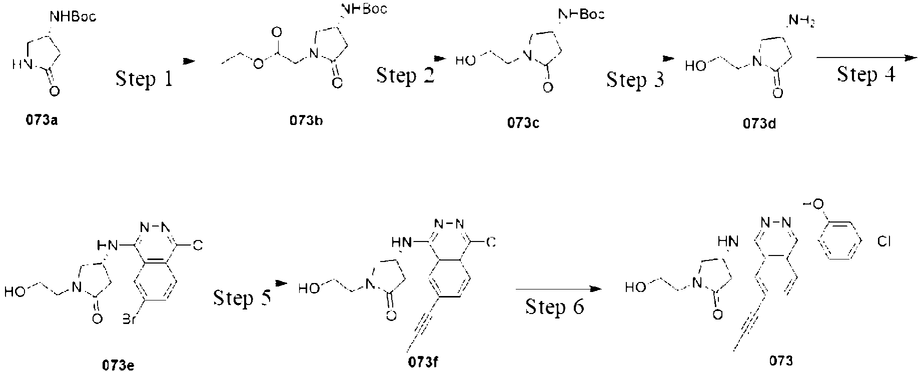

### Step 1 Preparation of ethyl (R)-2-(4-((tert-butyloxycarbonyl)amino)-2-oxopyrrolidin-1-yl)acetate (073b)

Compound (*R*)-tert-butyl (5-oxopyrrolidin-3-yl)carbamate **073a** (600 mg, 3.00 mmol) was dissolved in tetrahydrofuran (40 mL), and NaH (180 mg, 7.49 mmol) was slowly added at 0°C. The reaction mixture was stirred at 0°C for 1 h, then ethyl bromoacetate (600 mg, 3.60 mmol) was added and the mixture was allowed to react at room temperature for 3 h. After the reaction was completed, ammonium chloride solution (5 mL) was added to quench the reaction, and the mixture was extracted with ethyl acetate. The organic phase was collected, dried and concentrated to give a crude product. The crude product was purified by silica gel column chromatography purification system B to give the title compound **073b** (850 mg, 89%) as a white solid.

MS m/z(ESI): 287.3(M+1).

### Step 2 Preparation of tert-butyl (R)-(1-(2-hydroxyethyl)-5-oxopyrrolidin-3-yl)carbamate (073c)

Compound **073b** (850 mg, 2.85 mmol) was dissolved in tert-butanol/methanol (16.8 mL, V/V = 10:1), sodium borohydride (297 mg, 7.85 mmol) was added, and the mixture was stirred to react at 80°C for 2 h. After the reaction was completed, ice water was added to quench the reaction, the reaction mixture was concentrated under reduced pressure, and extracted with ethyl acetate. The organic phase was collected, dried, and concentrated to give a crude product. The crude product was purified by silica gel column chromatography purification system B to give the title compound **073c** (720 mg, 85.20%).

MS m/z(ESI): 245.2(M+1)

### Step 3 Preparation of (R)-4-amino-1-(2-hydroxyethyl)pyrrolidin-2-one (073d)

Compound **073c** (720 mg, 2.95 mmol) was dissolved in 4 M hydrochloric acid in dioxane (10 mL), stirred at room temperature for 2 h, and after the reaction was completed, the mixture was concentrated under reduced pressure to give a crude product **073d** (620 mg, 87.55%).

MS m/z (ESI): 145.1 (M+1).

### Step 4 Preparation of (R)-4-((7-bromo-4-chlorophthalic acid-1-yl)amino)-1-(2-hydroxyethyl)pyrrolidin-2-one (073e)

Compound **073d** (620 mg, 4.31 mmol) was dissolved in *N*-methylpyrrolidone (10 mL), and compound **024a** (1.2 g, 4.32 mmol) and *N,N*-diisopropylethylamine (3 mL) were added. The reaction was stirred at 100°C for 2 h. After the reaction was completed, the mixture was concentrated under reduced pressure to give a crude product. The crude product was separated and purified by silica gel column chromatography system A to give the title compound **073e** (500 mg, 37.60%).

MS m/z(ESI): 386.6(M+1).

### Step 5 Preparation of (R)-4-((4-chloro-7-(prop-1-yn-1-yl)phthalazin-1-yl)amino)-1-(2-hydroxyethyl)pyrrolidin-2-one (073f)

Compound **073e** (200 mg, 0.051 mmol) was dissolved in *N*,*N-*dimethylacetamide (3 mL), and copper iodide (30 mg, 0.52 mmol), bis(triphenylphosphine) palladium dichloride (55 mg, 0.078 mmol), 1-(trimethylsilyl)propyne (18 mg, 0.45 mmol) and cesium carbonate (180 mg, 0.55 mmol) were added respectively. The reaction was stirred at 50°C under nitrogen protection for 2 h. After the reaction was completed, the reaction mixture was filtered and concentrated under reduced pressure to give a crude product. The crude product was separated and purified by silica gel column chromatography purification system A to give the title compound **073f** (110 mg, 55.36%).

MS m/z (ESI): 345.1 (M+1).

### Step 6 Preparation of (R)-4-((4-(4-chloro-2-hydroxyphenyl)-7-(prop-1-yn-1-yl)phthalazin-1-yl)amino)-1-(2-hydroxyethyl)pyrrolidin-2-one (073)

Compound **073f** (30 mg, 0.087 mmol) was dissolved in a mixed solvent of dioxane and water (5 mL, V/V=10:1), and compounds (4-chloro-2-hydroxyphenyl)boronic acid (17 mg, 0.096 mmol), sodium carbonate (18 mg, 0.17 mmol) and 1,1'-bis(di-tert-butylphosphino)ferrocene palladium dichloride (13 mg, 0.02 mmol) were added. The reaction is stirred at 100°C under nitrogen protection for 1 h. After the reaction was completed, the reaction mixture was filtered, and concentrated under reduced pressure to give a crude product. The crude product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% FA); mobile phase 2: acetonitrile; 15-min gradient, gradient ratio: acetonitrile phase 20%-100%, flow rate: 20 mL/min) to give the title compound **073** (4 mg, yield: 10.5%).

MS m/z(ESI): 437.8(M+1)

¹H NMR (400 MHz, CD₃OD) δ 8.37 (s, 1H), 7.76 (d, 1H), 7.60 (d, 1H), 7.34 (d, 1H), 7.05 (d, 2H), 4.89 (s, 1H),4.16-4.12 (m, 1H), 3.75-3.74 (m, 2H), 3.67-3.65 (m, 1H), 3.50-3.49 (m, 2H), 3.05-2.99 (m, 1H), 2.74-2.69 (m, 1H), 2.12 (s, 3H).

### Example 45

### (R)-3-((4-(4-chloro-2-hydroxyphenyl)-7-(prop-1-yn-1-yl)phthalazin-1-yl)amino)-1-(2-hydroxyethyl)pyrrolidin-2-one (074)

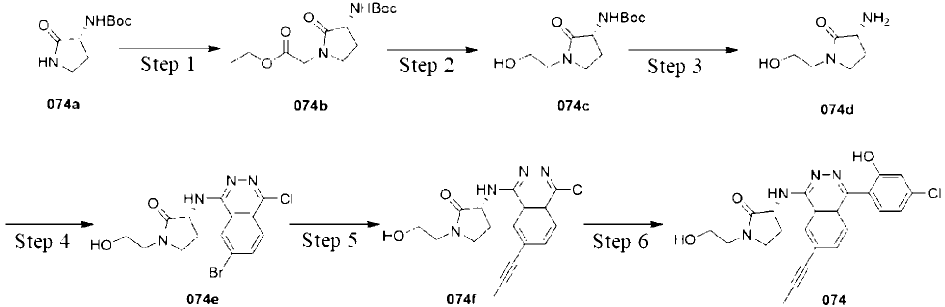

The synthetic route of **Example 44** was used. The raw material tert-butyl (*R*)-(5-oxopyrrolidin-3-yl)carbamate in step 1 was replaced with tert-butyl (*R*)-(2-oxopyrrolidin-3-yl)carbamate (500 mg, 2.5 mmol). The final product was purified by preparative high performance liquid chromatography (Waters MS-triggered Prep-LC with QDA detector, chromatographic column: Gemini 5 µm C18 100 x 21.2 mm; mobile phase 1: water (containing 0.1% FA); mobile phase 2: acetonitrile; 10-min gradient: acetonitrile phase 24%-100%, flow rate: 25 mL/min) to give the title compound **074** (2.5 mg, yield: 7%).

MS m/z (ESI): 437.1 (M+1).

¹H NMR (400 MHz, CD₃OD) δ 8.26 (s, 1H), 7.72 (d, 1H), 7.55 (d, 1H), 7.29 (d, 1H), 7.03-6.97 (m, 2H), 3.76 (dd, 2H), 3.70-3.61 (m, 2H), 3.52-3.44 (m, 2H), 2.65 (dt, 1H), 2.32-2.14 (m, 2H), 2.10 (s, 3H).

### Example 46

### (R)-3-((4-(4-chloro-2-hydroxyphenyl)-7-(prop-1-yn-1-yl)phthalazin-1-yl)amino)-1-(2-hydroxyethyl)piperidin-2-one (075)

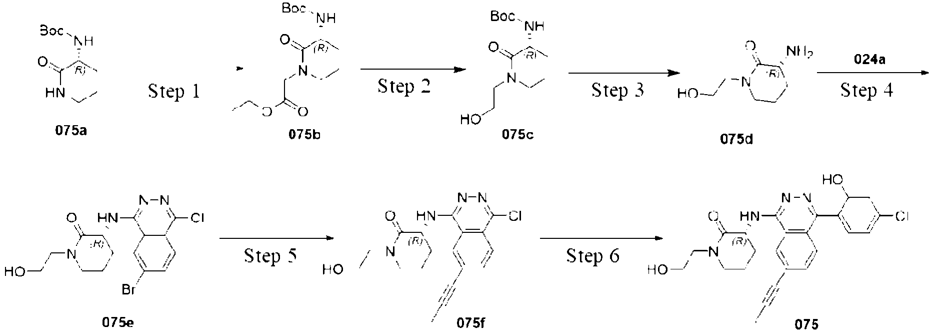

The synthetic route of **Example 44** was used. The raw material tert-butyl (*R*)-(5-oxopyrrolidin-3-yl)carbamate in step 1 was replaced with tert-butyl (*R*)-(2-oxopiperidin-3-yl)carbamate (1.5 g, 7 mmol). The final product was purified by preparative high performance liquid chromatography (Waters MS-triggered Prep-LC with SQD2 detector, chromatographic column: Xbridge 5 µm C18 150 x 19 mm; mobile phase 1: water (containing 0.1% formic acid); mobile phase 2: acetonitrile; 15-min gradient, gradient ratio: acetonitrile phase 20%-100%, flow rate: 20 mL/min) to give the title product **075** (1.42 mg, yield: 5%).

MS m/z (ESI): 451.1 (M+1).

¹H NMR (400 MHz, CD₃OD) δ 8.17 (s, 1H), 7.63 (d, 1H), 7.45 (d, 1H), 7.20 (d, 1H), 6.91 (d, 2H), 4.72-4.61 (m, 1H), 3.67-3.40 (m, 6H), 2.29-2.10 (m, 2H), 1.99 (s, 3H), 1.96-1.91 (m, 2H).

### Example 47

### (R)-5-((4-(4-chloro-2-hydroxyphenyl)-7-(prop-1-yn-1-yl)phthalazin-1-yl)amino)-1-(2-hydroxyethyl)piperidin-2-one (076)

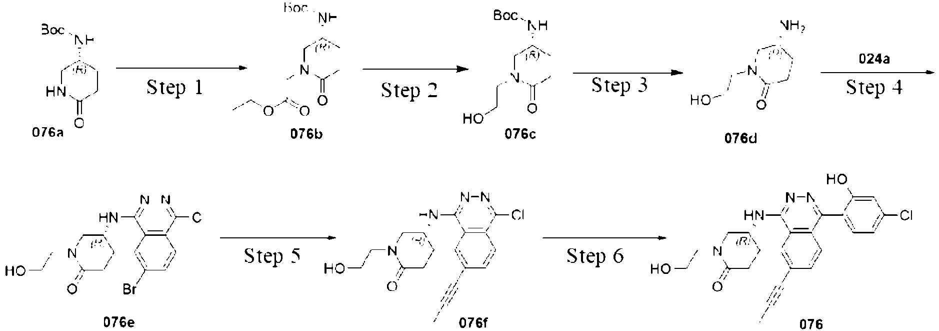

The synthetic route of **Example 44** was used. The raw material tert-butyl (*R*)-(5-oxopyrrolidin-3-yl)carbamate in step 1 was replaced with tert-butyl (*R*)-(6-oxopiperidin-3-yl)carbamate (600 mg, 3 mmol). The crude product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% aqueous ammonia); mobile phase 2: acetonitrile; 15-min gradient, gradient ratio: acetonitrile phase 30%-100%, flow rate: 25 mL/min) to give the title compound **076** (1.07 mg, yield: 2%).

MS m/z (ESI): 451.1 (M+1).

¹H NMR (400 MHz, CD₃OD) *δ* 8.25 (s, 1H), 7.63 (d, 1H), 7.46 (d, 1H), 7.20 (d, 1H), 6.90-6.86 (m, 2H), 4.68-4.60 (m, 1H), 3.84-3.82 (m, 1H), 3.65-3.63 (m, 2H), 3.58-3.26 (m, 3H), 2.49 (t, 2H), 2.08- 2.06 (m, 1H), 2.00 (s, 3H), 1.94-1.92 (m, 1H).

### Example 48

### 2-(4-(((1S,2S)-3-(difluoroethylidene)-2-hydroxycyclohexyl)amino)pyrido[3,4-d]pyridazin-1-yl)-5-(trifluoromethyl)phenol

### 2-(4-(((1R,2R)-3-(difluoroethylidene)-2-hydroxycyclohexyl)amino)pyrido[3,4-d]pyridazin-1-yl)-5-(trifluoromethyl)phenol

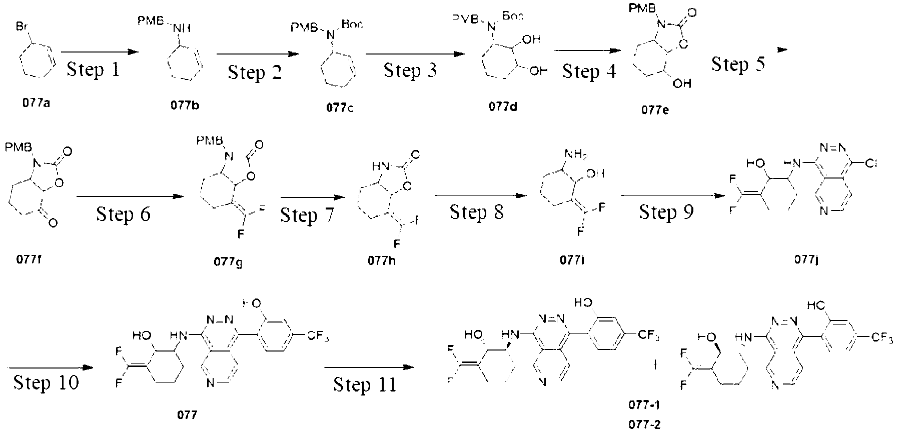

### Step 1 Preparation of N-(4-methoxybenzyl)cyclohex-2-en-1-amine (077b)

Compound 3-bromocyclohexene **077a** (10 g, 0.062 mol) was dissolved in acetonitrile (100 mL), and compound 4-methoxybenzylamine (17.04 g, 0.12 mol) and potassium carbonate (8.58 g, 0.062 mol) were added in sequence. The reaction mixture was stirred at room temperature for 4 h. After the reaction was completed, the reaction mixture was filtered, and the filtrate was concentrated under reduced pressure to give a crude product. The crude product was purified by silica gel column chromatography system B to give the title compound **077b** (10 g, 66.7%).

MS m/z(ESI): 218.1(M+1).

### Step 2 Preparation of tert-butyl cyclohex-2-en-1-yl(4-methoxybenzyl)carbamate (077c)

Compound **077b** (10 g, 0.046 mol) was dissolved in ethyl acetate (100 mL), to the reaction mixture was added di-tert-butyl dicarbonate (10.04 g, 0.046 mol), and the mixture was stirred at room temperature for 4 h. After the reaction was completed, the reaction mixture was concentrated under reduced pressure to give a crude product. The crude product was separated and purified by silica gel column chromatography system B to give the title compound **077c** (14.8 g, 91%).

MS m/z(ESI): 318.4(M+1).

### Step 3 Preparation of tert-butyl (2,3-dihydroxycyclohexyl)(4- methoxybenzyl)carbamate (077d)

Compound **077c** (14.8 g, 0.047 mol) was dissolved in a mixed solvent of tert-butanol and water (100 mL, V/V = 5:1), and 1-methylpiperidine 1-oxide (5.99 g, 0.051 mol) and potassium osmate dihydrate (1.46 g, 0.0047 mol) were added in sequence. The reaction was stirred at room temperature for 12 h. After the reaction was completed, the mixture was filtered and concentrated under reduced pressure to give a crude product. The crude product was purified by silica gel column chromatography separation system B to give the title compound **077d** (17.6 g, 91%, a pair of enantiomers).

MS m/z(ESI): 352.4(M+1).

### Step 4 Preparation of 7-hydroxy-3-(4-methoxybenzyl)hexahydrobenzo[d]oxazol-2(3H)-one (077e)

Compound **077d** (17.6 g, 0.0050 mol) was dissolved in *N*,*N-*dimethylformamide (80 mL), and compound NaH (3.59 g, 0.14 mol) was added. The reaction was stirred at room temperature for 2 h. After the reaction was completed, ice water was added to quench the reaction, and the mixture was extracted with ethyl acetate. The organic layer was collected, dried over anhydrous sodium sulfate and filtered. The filtrate was concentrated under reduced pressure to give a crude product. The crude product was purified by silica gel column chromatography separation system B to give the title compound **077e** (12.6 g, 77.3%, a pair of enantiomers).

MS m/z (ESI): 277.3 (M+1).

### Step 5 Preparation of 3-(4-methoxybenzyl)tetrahydrobenzo[d]oxazol-2,7(3H,4H)-dione (077f)

Compound **077e** (12 g, 0.043 mol) was dissolved in dichloromethane (100 mL), and compound pyridinium chlorochromate (28 g, 0.13 mol) was added. The reaction was stirred at room temperature for 12 h. After the reaction was completed, the reaction mixture was filtered and the filtrate was concentrated under reduced pressure to give a crude product. The crude product was purified by silica gel column chromatography separation system B to give the title compound **077f** (8.6 g, 64.9%, a pair of enantiomers).

MS m/z (ESI): 275.3 (M+1).

### Step 6 Preparation of 7-(difluoromethylene)-3-(4-methoxybenzyl)hexahydrobenzo[d]oxazol-2(3H)-one (077g)

Compound **077f** (2.4 g, 8.7 mmol) was dissolved in *N*-methylpyrrolidone (20 mL), and (triphenylphosphonio)difluoroacetate (3.72 g, 10.44 mmol) was added. The reaction mixture was stirred at 80°C under nitrogen atmosphere for 12 h. After the reaction was completed, saturated aqueous ammonium chloride solution was added, and the mixture was extracted with ethyl acetate. The organic layer was dried over anhydrous sodium sulfate and filtered. The filtrate was concentrated under reduced pressure to give a crude product. The crude product was purified by silica gel column chromatography separation system B to give the title compound **077g** (240 mg, 10.3%, a pair of enantiomers).

MS m/z (ESI): 310.1 (M+1).

### Step 7 Preparation of 7-(difluoromethylene)hexahydrobenzo[d]oxazol-2(3H)-one (077h)

Compound **077g** (100 mg, 0.32 mmol) was dissolved in a mixed solvent of acetonitrile and water (6 mL, V/V= 5:1), to the reaction mixture was added ammonium cerium nitrate (886.2 mg, 1.6 mmol), and the mixture was stirred at room temperature for 1 h. Saturated sodium bicarbonate solution was added to the reaction mixture, and the mixture was extracted with ethyl acetate. The organic phase was dried over anhydrous sodium sulfate, filtered, and the filtrate was concentrated under reduced pressure to give a crude product. The crude product was purified by silica gel column chromatography separation system B to give the title compound **077h** (30 mg, 44.1%).

MS m/z (ESI): 190.2 (M+1).

### Step 8 Preparation of 2-amino-6-(difluoroylidene)cyclohexan-1-ol (077i)

Compound **077h** (30 mg, 0.15 mmol ) was dissolved in 10% aqueous lithium hydroxide solution (2 mL), and the reaction was stirred at 80°C for 1 h. After the reaction was completed, the mixture was extracted with ethyl acetate, and the organic phases were combined, dried over anhydrous sodium sulfate, filtered, and concentrated under reduced pressure to give a crude compound **077i** (20 mg, 70%, a pair of enantiomers).

MS m/z (ESI): 164.2 (M+1).

### Step 9 Preparation of 2-((1-chloropyrido[3,4-d]pyridazin-4-yl)amino)-6-(difluoroylidene)cyclohexan-1-ol (077j)

Compound **077i** (40 mg, 0.24 mmol) was dissolved in *N*-methylpyrrolidone (2 mL), and compound **009a** (49 mg, 0.24 mmol) and *N,N*-diisopropylethylamine (95 mg, 0.73 mmol) were added. The reaction was stirred at 100°C for 1. After the reaction was completed, saturated aqueous ammonium chloride solution was added, and the mixture was extracted with ethyl acetate. The organic phase was dried over anhydrous sodium sulfate and filtered. The filtrate was concentrated under reduced pressure to give a crude product. The crude product was purified by silica gel column chromatography separation system B to give the title compound **077j** (40 mg, 50.3%, a pair of enantiomers).

MS m/z (ESI): 327.1 (M+1).

### Step 10 2-(4-((-3-(difluoroethylidene)-2-hydroxycyclohexyl)amino)pyrido[3,4-d]pyridazin-1-yl)-5-(trifluoromethyl)phenol 077

Compound **077j** (40 mg, 0.12 mmol) was dissolved in a mixed solvent of dioxane and water (2.2 mL, V/V=10:1), and compounds (4-trifluoromethyl-2-hydroxyphenyl)boric acid (30.2 mg, 0.15 mmol), sodium carbonate (38.92 mg, 0.36 mmol) and 1,1'-bis(di-tert-butylphosphino)ferrocene palladium dichloride (8.88 mg, 0.012 mmol) were added. The reaction was stirred under nitrogen protection at 100°C for 1 h. After the reaction was completed, the reaction mixture was filtered and the filtrate was concentrated under reduced pressure to give a crude product. The crude product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% aqueous ammonia); mobile phase 2: acetonitrile; 15-min gradient, gradient ratio: acetonitrile phase 35%-100%, flow rate: 25 mL/min) to give the title compound **077** (14 mg, yield: 8%, a pair of enantiomers).

MS m/z(ESI): 453.4(M+1).

### Step 11

### 2-(4-(((1S,2S)-3-(difluoroethylidene)-2-hydroxycyclohexyl)amino)pyrido[3,4-d]pyridazin-1-yl)-5-(trifluoromethyl)phenol

### 2-(4-(((1R,2R)-3-(difluoroethylidene)-2-hydroxycyclohexyl)amino)pyrido[3,4-d]pyridazin-1-yl)-5-(trifluoromethyl)phenol

Compound **077** (13 mg) was purified by preparative SFC chromatography (SFC 150 column: Daicel CHIRALCEL IC, 250mm× 30 mm ID, 10 µm; mobile phase 1: carbon dioxide; mobile phase 2: methanol (0.2% aqueous ammonia, 7M methanol solution); 6-min gradient, gradient ratio carbon dioxide: methanol phase = 80/20, flow rate: 2 mL/min) to give the title compound **077-1** (6.00 mg) and the title compound **077-2** (6.2 mg).

### 077-1: (Mono-configurational compound with shorter retention time)

MS m/z(ESI): 453.4(M+1).

¹H NMR (400 MHz, CD₃OD) δ 9.74 (s, 1H), 8.83 (d, 1H), 7.54 (d, 1H), 7.48 (d, 1H), 7.26-7.22 (m, 2H), 4.43-4.38 (m, 1H), 2.26-2.02 (m, 3H), 1.97-1.92 (m, 1H), 1.84-1.74 (m, 2H), 1.59-1.48 (m, 1H).

### 077-2: (Mono-configurational compound with longer retention time)

MS m/z(ESI): 453.4(M+1).

¹H NMR (400 MHz, CD₃OD) δ 9.68 (s, 1H), 8.80 (d, 1H), 7.53 (d, 1H), 7.44 (d, 1H), 7.26 (d, 1H), 7.21 (s, 1H), 4.57-4.55 (m, 1H), 2.26-2.12 (m,3H), 2.02-1.97 (m, 1H), 1.89-1.82 (m, 2H), 1.69-1.65 (m, 1H).

### Example 49

### 5-chloro-2-(4-(((1S,2S)-3-(difluoroethylidene)-2-hydroxycyclohexyl)amino)pyrido[3,4-d]pyridazin-1-yl)phenol

### 5-chloro-2-(4-(((1R,2R)-3-(difluoroethylidene)-2-hydroxycyclohexyl)amino)pyrido[3,4-d]pyridazin-1-yl)phenol

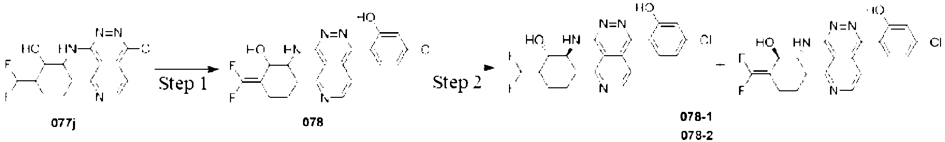

Compound **077j** (40 mg, 0.12 mmol) was dissolved in a mixed solvent of dioxane and water (2.2 mL, V/V=10:1), and compounds (4-chloro-2-hydroxyphenyl)boric acid (23 mg, 0.13 mmol), sodium carbonate (38.9 mg, 0.36 mmol) and 1,1'-bis(di-tert-butylphosphino)ferrocene palladium dichloride (9 mg, 0.012 mmol) were added. The reaction was stirred at 100°C under nitrogen protection for 1 h. After the reaction was completed, the reaction mixture was filtered and the filtrate was concentrated under reduced pressure to give compound **078.** The crude product was purified by preparative high performance liquid chromatography (Waters MS triggered Prep-LC with Acquity QDA detector, column: Welch 10 µm C18 250 x 21.2 mm; mobile phase 1: water (containing 0.1% aqueous ammonia); mobile phase 2: acetonitrile; 15-min gradient, gradient ratio: acetonitrile phase 27%-100%, flow rate: 25 mL/min) to give the racemic compound (15 mg), which was purified by preparative SFC chromatography (SFC 150 column: Daicel CHIRALCEL IC, 250mm× 30 mm ID, 10 µm; mobile phase 1: carbon dioxide; mobile phase 2: methanol (0.2% aqueous ammonia, 7M methanol solution); 6-min gradient, gradient ratio carbon dioxide: methanol phase = 80/20, flow rate: 2 mL/min) to give the title compound **078-1** (6.8 mg) and the title compound **078-2** (7.02 mg).

### 078-1: (Mono-configurational compound with shorter retention time)

MS m/z(ESI): 419.0(M+1).

¹H NMR (400 MHz, CD₃OD) δ 9.61 (s, 1H), 8.75 (d, 1H), 7.41 (d, 1H), 7.27 (d, 1H), 6.95-6.92 (m, 2H), 4.51 (d, 1H), 2.22-2.06 (m, 3H), 1.96-1.86 (m, 1H), 1.83-1.76 (m, 2H), 1.64-1.62 (m, 1H).

### 078-2: (Mono-configurational compound with longer retention time)

MS m/z(ESI): 419.0(M+1).

¹H NMR (400 MHz, CD₃OD) δ 9.58 (s, 1H), 8.71 (d, 1H), 7.38 (d, 1H), 7.25 (d, 1H), 6.92-6.89 (m, 2H), 4.50 (d, 1H), 2.18-2.08 (m, 3H), 1.94-1.88 (m, 1H), 1.77-1.73 (m, 2H), 1.61-1.58 (m, 1H).

### Example 50

### (S)-3-((4-(4-chloro-2-hydroxyphenyl)-7-(cyclopropylethynyl)phthalazin-1-yl)amino)propan-1,2-diol (079)

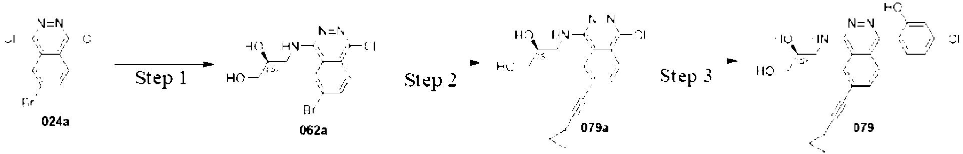

The synthetic route of **Example 32** was adopted. The raw material 1-(trimethylsilyl)propyne in step 2 was replaced with cyclopropylacetylene (52 mg, 0.782 mmol). The final product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% ammonia); mobile phase 2: acetonitrile; 15-min gradient, gradient ratio: acetonitrile phase 30%-100%, flow rate: 25 mL/min) to give the title compound **079** (31 mg, yield: 22%).

MS m/z (ESI): 410.1 (M+1).

¹H NMR (400 MHz, CD₃OD) δ 8.26 (d, 1H), 7.72 (dd, 1H), 7.56 (d, 1H), 7.31 (d, 1H), 7.04-7.00 (m, 2H), 4.04-3.97 (m, 1H), 3.84 (dd, 1H), 3.74 (dd, 1H), 3.63 (d, 2H), 1.57 (tt, 1H), 1.00-0.95 (m, 2H), 0.86-0.80 (m, 2H).

### Example 51

### (R)-5-chloro-2-(4-((3-(difluoromethylene)cyclohexyl)amino)pyridin[3,4-d]pyridazin-1-yl)phenol (080)

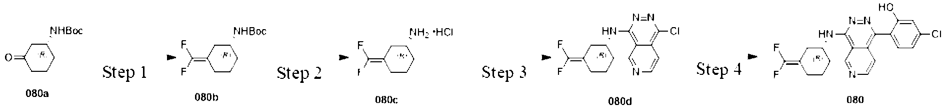

### Step 1 Preparation of tert-butyl (R)-(3-(difluoromethylene)cyclohexyl)carbamate (080b)

Under nitrogen atmosphere, potassium tert-butoxide (235 mg, 2.1 mmol) was dissolved in *N,N-*dimethylformamide (5 mL), the mixture was cooled to -55°C, and compound tert-butyl (*R*)-(3-oxocyclohexyl)carbamate **080a** (300 mg, 1.4 mmol) and compound 2-((difluoromethyl)sulfonyl)pyridine (289 mg, 1.5 mmol) were added in sequence. The reaction mixture was stirred at -55°C for 1 h. After the reaction was completed, saturated aqueous ammonium chloride solution was added, and the mixture was extracted with ethyl acetate. The organic phase was dried over anhydrous sodium sulfate and filtered. The filtrate was concentrated under reduced pressure to give a crude product. The crude product was purified by silica gel column chromatography separation system B to give the title compound **080b** (150 mg, 43%).

MS m/z (ESI): 248.1 (M+1).

### Step 2 Preparation of (R)-3-(difluoromethylene)cyclohexane-1-amine hydrochloride (080c)

Compound **080b** (150 mg, 0.61 mmol) was dissolved in 4 M hydrochloric acid in 1,4-dioxane (5 mL) and stirred at room temperature for 1 h. The reaction mixture was concentrated under reduced pressure to give crude compound **080c** (110 mg, 99%). The product was directly used in the next step without further purification.

MS m/z (ESI): 148.1 (M+1).

### Step 3 Preparation of (R)-1-chloro-N-(3-(difluoromethylene)cyclohexyl)pyrido[3,4-d]pyridazin-4-amine (080d)

Compound **080c** (110 mg, 0.6 mmol) was dissolved in *N*-methylpyrrolidone (5 mL), and compound **009a** (110 mg, 0.6 mmol) and *N,N*-diisopropylethylamine (0.3 mL, 1.8 mmol) were added. The reaction was stirred at 100°C for 3 h. After the reaction was completed, it was diluted with water, extracted with ethyl acetate, washed with saturated brine. The organic phases were combined, dried over anhydrous sodium sulfate, filtered, and concentrated under reduced pressure to remove the solvent. The crude product was separated and purified by silica gel column chromatography purification system B to give the title compound **080d** (80 mg, 43%).

MS m/z (ESI): 311.0 (M+1).

### Step 4 Preparation of (R)-5-chloro-2-(4-((3-(difluoromethylene)cyclohexyl)amino)pyridin[3,4-d]pyridazin-1-yl)phenol (080)

Compound **080d** (50 mg, 0.17 mmol) was dissolved in a mixed solvent of 1,4-dioxane and water (3.3 mL, V/V = 10:1), followed by the addition of (4-chloro-2-hydroxy-6-methylphenyl)boronic acid (30.5 mg, 0.18 mmol), potassium carbonate (51.16 mg, 0.48 mmol) and 1,1'-bis(diphenylphosphino)ferrocene palladium dichloride (11.68 mg, 0.016 mmol), and the reaction was carried out at 100°C with stirring under nitrogen protection for 1 h. After the reaction was completed, the reaction mixture was filtered, and the filtrate was collected and concentrated under reduced pressure to give a crude product. The crude product was purified by preparative high performance liquid chromatography (Waters MS triggered Prep-LC with Acquity QDA detector, chromatographic column: Welch 10 µm C18 250 x 21.2 mm; mobile phase 1: water (containing 0.1% aqueous ammonia); mobile phase 2: acetonitrile; 15-min gradient, gradient ratio: acetonitrile phase 50%-100%, flow rate: 25 mL/min) to give the title compound **080** (14 mg, yield: 21.75%).

MS m/z(ESI): 403.0(M+1).

¹H NMR (400 MHz, DMSO-*d₆*) δ 9.76 (s, 1H), 8.83 (d, 1H), 7.72 (d, 1H), 7.33 (d, 1H), 7.28 (d, 1H), 6.89-6.86 (m, 2H), 4.33-4.28 (m, 1H), 2.88(d, 1H), 2.39 (d, 1H), 2.13-2.11 (m, 1H), 2.02-1.87(m, 4H), 1.65-1.56 (m, 1H), 1.49-1.44 (m, 1H).

The compounds in the following table were prepared using the synthesis methods similar to those in the above examples.

| Compound | ¹H NMR |
|---|---|
| 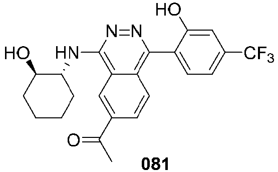 | ¹H NMR (400 MHz, DMSO-*d₆*) δ 10.41 (s, 1H), 9.01 (s, 1H), 8.22 (d, 1H), 7.61 (d, 1H), 7.54-7.50 (m,2H), 7.32-7.29(m, 2H), 4.25-4.23 (m, 1H),3.66 (t, 1H), 2.76 (s, 3H), 2.17-2.14 (m, 1H), 2.02-1.99 (m, 1H), 1.75-1.69 (m, 2H), 1.37-1.33(m, 4H). |
| 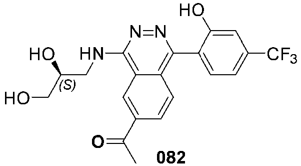 | ¹H NMR (400 MHz, DMSO-*d₆*) δ 9.03 (s, 1H), 8.24 (d, 2H), 7.54 (dd, 2H), 7.31 (d, 2H), 3.95-3.87 (m, 1H), 3.79 (d, 1H), 3.60 (d, 1H), 3.45 (d, 2H), 2.75 (s, 3H). |

### Biological Evaluation

### Test Example 1 Experiment on detection of IC₅₀ of NLRP3 inhibitors in reducing the level of IL-1β secretion by THP-1 cells

The chemical names and structural formulas of the compounds of the present invention used in the experiment are given in the preparation examples of the compounds.
1. Experimental principle: In this experiment, we use the human monocyte THP-1 to study the inhibitory activity of NLRP3 inhibitor on the secretion of IL-Iβ by cells. Human THP-1 cells are treated with crotonol-12-tetradecanoate-13-acetate(PMA) to differentiate into mature macrophage models. Then, according to the two stages of inflammasome formation in cells (assembly and activation), the cells are stimulated with lipopolysaccharide (LPS) and nigericin in turn, ultimately promoting the extracellular release of IL-1β by THP-1 cells. The first stage of inflammasome formation involves stimulating cells with LPS (lipopolysaccharide), an agonist of the Toll-like receptor TLR4, to activate the expression of the inflammasome-associated proteins NLRP3, caspase 1, and pro-IL-1β, a precursor of IL-1β. The second stage of inflammasome formation involves the addition of the potassium ion carrier nigericin, which causes a series of membrane potential changes in THP-1 cells, destroys the cell membrane structure, causes the efflux of cellular potassium ions, further stimulates the oligomerization of NLRP3 monomers to form NLRP3 oligomers, and begins to recruit ASC and pro-caspase-1 to assemble the NLRP3 inflammasome complex containing a large amount of pro-caspase-1. The pro-caspase-1 on the activated NLRP3 inflammasome is enzymatically self-activated to form a large amount of active caspase-1. The activated caspase-1 further enzymatically promotes the conversion of pro-IL-1β into mature IL-1β that can be secreted. In this process, NLRP3 inhibitors can effectively inhibit the maturation and activation of NLRP3 induced by nigericin and the activation of downstream caspase-1, thereby inhibiting the maturation and secretion of IL-1β.
2. Experimental reagents and instruments

| Materials and reagents | Supplier | Art. No. |
|---|---|---|
| THP-1 cell line | ATCC | TIB-202 |
| RPMI Medium 1640 | Invitrogen | A10491-01 |
| Penicillin-streptomycin | Gibco | 15140-122 |
| Fetal bovine serum | Gibco | 10099-141C |
| 2-Mercaptoethanol | sigma | 07604 |
| Phosphate buffered saline (PBS) | Gibco | 10010-031 |
| DMSO | Sigma | D8418-1L |
| Lipopolysaccharides (LPS) | sigma | L2880 |
| MCC950 | MCE | 256373-96-3 |
| Nigericin sodium salt | MCE | HY-100381 |
| PMA | sigma | P1585 |
| Human IL-1 beta Valukine ELISA kit | R&D | VAL101 |
| Consumables and Instruments | Supplier | Art. No. |
| 96-well plate | Corning | 3599 |
| Plate shaker | QILINBEIER | QB-9002 |
| Centrifuge | Eppendorf | 5810R |
| CO₂ incubator | Thermo Scientific | 371 |
| Microscope | OLYMPUS | CKX41 |
| Countess cell counting chamber slides | Gibco | C10281 |
| Envision plate reader | PerkinElmer | 2105 |
| Washer dispenser | BioTek | EL406 |

3. Experimental steps
(1) Cell suspensions were collected. The cells were centrifuged at 1000 rpm for 4 min, the supernatant was discarded, the cells were resuspended with 1 mL of test medium, and then the cell culture medium was gently pipetted up and down with a pipette and its tip to break up the cell aggregates. After counting the cells, the THP-1 cells were resuspended with complete RPMI medium 1640 containing PMA and seeded into 96-well plates and cultured in a 37°C, 5% CO₂ incubator for 72 h.
(2) The culture medium was taken out from each well with an automatic plate washer dispenser EL406 and each well was rinsed twice with PBS. To each well, 96 µL of RPMI 1640 medium containing LPS was added. The cells were cultured in a 37°C, 5% CO₂ incubator for 4 h.
(3) To each well, 2 µL of the compound and 2 µL of nigericin sodium salt were added, and the final compound concentration selected was 5000 nM, 1000 nM, 200 nM, 40 nM, 8 nM, 1.6 nM, 0.32 nM, 0 nM, respectively. The cells were cultured in a 37°C, 5% CO₂ incubator for 1 h.
(4) An appropriate amount of supernatant was taken out, diluted 100 times, and the content of IL-1β in the supernatant was detected using Human IL-1 beta Valukine ELISA kit.

4. Experimental results

The experimental results are shown in Table 1.

**Table 1 Test results of the experiment on detecting IC₅₀ of NLRP3 inhibitors using THP-1 cells**

| **Test compound** | **IC₅₀ (nM)** |
|---|---|
| Compound 001 | 88 |
| Compound 004 | 63 |
| Compound 005 | 37 |
| Compound 006 | 12 |
| Compound 007 | 29 |
| Compound 008 | 39 |
| Compound 009 | 28 |
| Compound 010 | 16 |
| Compound 014 | 98 |
| Compound 016 | 11 |
| Compound 017 | 17 |
| Compound 018 | 50 |
| Compound 019 | 111 |
| Compound 024 | 19 |
| Compound 024-1 | 108 |
| Compound 024-2 | 13 |
| Compound 026 | 35 |
| Compound 028 | 35 |
| Compound 035 | 14 |
| Compound 036 | 51 |
| Compound 038 | 42 |
| Compound 039 | 73 |
| Compound 040 | 54 |
| Compound 041 | 14 |
| Compound 042 | 14 |
| Compound 043 | 34 |
| Compound 051 | 46 |
| Compound 057 | 54 |
| Compound 058 | 158 |
| Compound 056 | 18 |
| Compound 061 | 23 |
| Compound 062 | 9.7 |
| Compound 063 | 15 |
| Compound 064 | 18 |
| Compound 065 | 13 |
| Compound 066 | 20 |
| Compound 067 | 66 |
| Compound 068 | 39 |
| Compound 069 | 31 |
| Compound 070 | 42 |
| Compound 071 | 23 |
| Compound 073 | 51 |
| Compound 074 | 7.2 |
| Compound 075 | 7 |
| Compound 076 | 35 |
| Compound 077-2 | 32 |
| Compound 078-1 | 41 |
| Compound 079 | 41 |
| Compound 080 | 36 |
| Compound 081 | 5.3 |
| Compound 082 | 32 |

Conclusion: The compounds of the present invention have obvious inhibitory activity on the maturation and secretion of IL-1β of THP-1 cells.

### Test Example 2: Mouse acute peritonitis model evaluation experiment

Male C57BL/6 mice were orally administered with a 10 mg/kg dose of the test compound or control vehicle, and then injected intraperitoneally with 1 µg of E. coli 055: B5 ultrapure lipopolysaccharide (LPS) suspension 1 h later, followed by an intraperitoneal injection of 0.5 ml of 30 mM disodium salt ATP suspension (in PBS, the pH of the ATP solution was adjusted to 7.2 before injection) 2 h later. After 30 min, all animals would be euthanized by carbon dioxide, and blood and peritoneal lavage fluid would be collected. The peritoneal lavage fluid was kept at 4°C.

Preparation of peritoneal lavage fluid: The peritoneal cavity would be lavaged with ice-cold PBS (3 mL) containing 25 U/mL heparin and 10% heat-inactivated FBS. Prior to use, one tablet of the protease inhibitor mixture was added per 50 mL of buffer. The collected lavage fluid would be centrifuged (centrifugation conditions: 1000 g, 4°C, 10 min) to remove cells and cell debris, and the clarified samples would be stored at -80°C for measurement of cytokine IL-1β.

The mouse IL-1β/IL-1F2 DuoSet ELISA kit (R&D Systems) was used to measure the changes in IL-1β cytokine levels in peritoneal lavage fluid and serum. By comparing with the Vehical group, the inhibition rate of the compound on IL-1β was calculated. The experimental results are shown in Table 2.

**Table 2**

| IL-1β | **062** | **079** | **081** | **082** | **063** | **064** |
|---|---|---|---|---|---|---|
| Peritoneal lavage fluid (inhibition rate) | 94% | 51% | 45% | 0% | 79% | 81% |
| Plasma (inhibition rate) | 96% | 21% | 61% | 12% | 74% | 90% |

Conclusion: Some compounds showed inhibitory effects on IL-1β in the in vivo animal model evaluation experiment, among which compound 062 had the most significant inhibitory effect.

### Test Example 3 MSU-induced rat gouty arthritis model evaluation test

In this test, male SD rats were used to establish an acute gouty arthritis model by injecting 64 mg/mL sodium urate (MSU) crystal suspension into the right hind ankle joint cavity of the rats. The animals were randomly divided into normal control group, model control group, different dose groups of test compound, 7 rats in each group. The compound group was orally administered with the compound 1 h before and after modeling, and the normal control group and model control group were given an equal amount of vehicle. The foot circumference and foot diameter were measured before and 24 h after modeling.

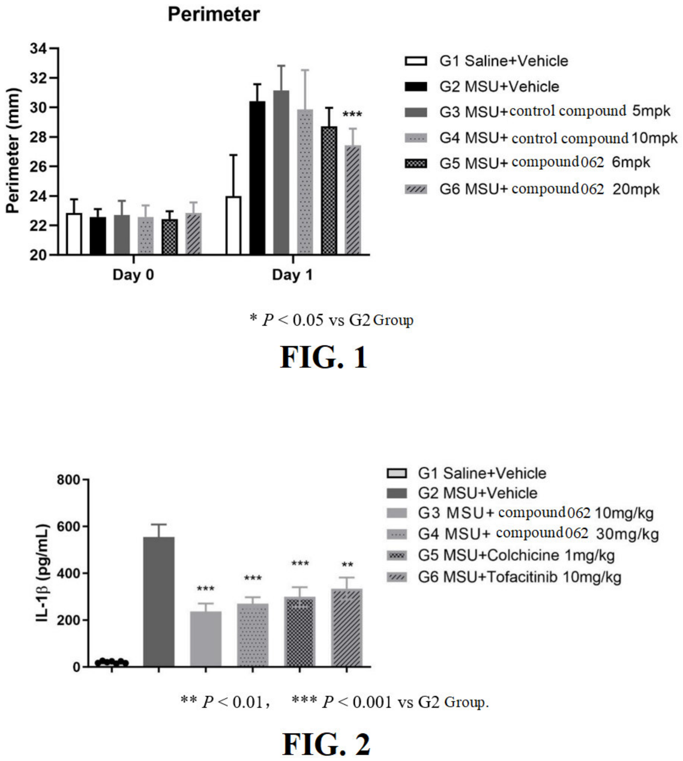

The experimental results are shown in FIG. 1. Compound 062 showed a dose-dependent improvement in ankle joint swelling in the MSU-induced acute rat gouty arthritis model, and the effect was significantly better than that of the control compound.

### Test Example 4 MSU-induced rat air pouch model test

Male SD rats were used in this experiment. After an adaptation period, a dorsal air pouch model was established. On the first, fourth and seventh days of the molding, 20 ml of sterile air was injected subcutaneously into the back of the rat with an injection needle to maintain the inflation of the air pouch. On the eighth day, different doses of the compound or vehicle control were administered by oral gavage. 25 mg of sodium urate suspension was injected into the dorsal air pouch, and 5 h later, the air pouch exudate was collected. The exudate was centrifuged at 1500 rpm for 20 min, and the supernatant was collected and the IL-1β production was detected by ELISA (R&D, Rat IL-1β kit).

The experimental results are shown in FIG. 2. Compound 062 significantly inhibited the production of IL-1β in the air pouch in the MSU-induced air pouch model, and its effect was better than that of colchicine and tofacitinib.

### Test Example 5 Zymosan A-induced rat pericarditis model test

In this experiment, SD rats were used and 150 µL of a mixture of 50 mg/mL ZymosanA and normal saline was injected into the pericardial cavity to induce a rat pericarditis model and conduct a pharmacodynamic observation experiment. The day of modeling was designated as Day 1. The animals were weighed and randomly divided into groups one day before modeling, namely, G1 normal group, G2 model group, G3 compound 062-10 mg/kg group, G4 compound 062-30 mg/kg group, G5 colchicine-100 µg/kg group, with 7 + 1* animals in each group (one extra animal was reserved for each group, considering that animals may die during model establishment). After the model was established, oral administration began, once a day, for 7 consecutive days. At the end of the experiment, the animals were euthanized, the muscles were cut open at the third and fourth rib regions to expose the heart, the pericardium was carefully lifted with forceps, and as much pericardial effusion (if any) as possible was extracted with a 1 mL syringe. The pericardial effusion was centrifuged at 5000 rpm at 4°C for 5 min, and the supernatant was frozen at -80°C for later detection of IL-1β levels. After the pericardial effusion was collected, the heart was perfused and removed (while ensuring the integrity of the pericardium), fixed with 10% formalin for subsequent pathological examination.

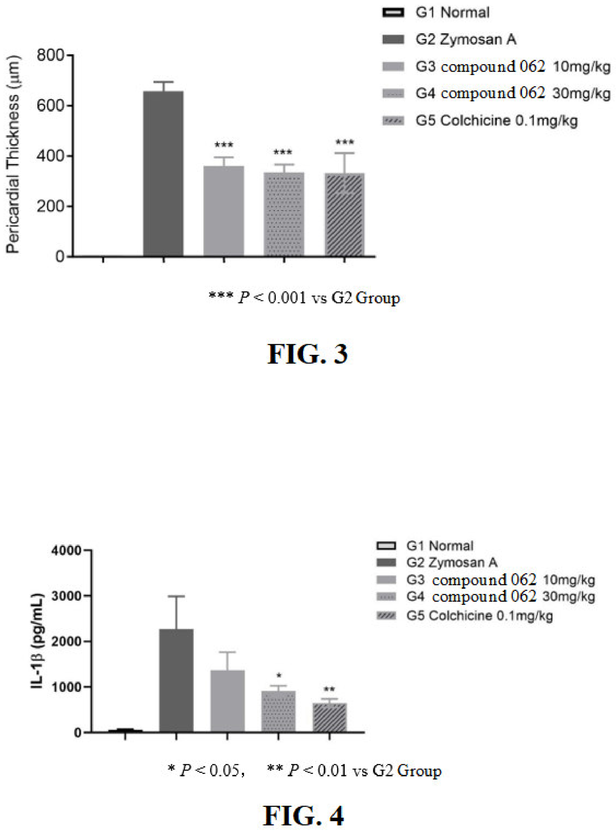

The experimental results are shown in FIGS. 3 and 4. Compound 062 significantly inhibited the production of IL-1β in the pericardium and pericardial effusion and improved the degree of pericardial inflammation in the ZymosanA-induced pericarditis model, with an effect comparable to that of colchicine.

### Test Example 6 Mouse NASH model test

After the animals arrived and acclimatized for 1 week, they were randomly divided into 2 groups. 48 mice were fed a high-fat diet (HFD) and had free access to 15% fructose water (w/w). After the animals were fed for 20 weeks, they were fasted for 6 h, and fasting blood glucose (FBG) was monitored by a blood glucose meter. Orbital blood was taken from all animals, and serum was collected by centrifugation to detect serum ALT levels. Animals were randomly divided into 6 groups according to their body weight, FBG and ALT levels. After that, the high fat feed (HFD) was replaced with the modified high fat feed (mHFD), and the mice had free access to 15% fructose water (v/w). The 8 mice in the other group were fed normal diet and water.

The first end point of the experiment: After 8 weeks of administration, the end point body weight was recorded and the animals were fasted for 6 h, and fasting blood glucose was measured with a blood glucose meter. The mice were then euthanized by CO₂ and serum was collected by orbital blood sampling and stored at -80°C for insulin and biochemical analysis. The animal's liver was taken and the liver weight was recorded. The liver was divided into four parts, one part was quick-frozen in liquid nitrogen for biochemical analysis, one part was placed in tissue fixative for pathological analysis, one part was stored in RNAlater for qPCR detection (backup for later use), and one part was quick-frozen in liquid nitrogen for possible subsequent detection.

The second end point of the experiment: 3 mice were kept in each group for 6 weeks for further observation. The end point body weight was recorded and the mice were fasted for 6 h. Fasting blood glucose was tested with a blood glucose meter. The mice were then euthanized by CO₂ and serum was collected by orbital blood sampling and stored at -80°C for insulin and biochemical analysis. The animal's liver was taken and the liver weight was recorded. The liver was divided into four parts, one part was quick-frozen in liquid nitrogen for biochemical analysis, one part was placed in tissue fixative for pathological analysis, one part was stored in RNAlater for qPCR detection (backup for later use), and one part was quick-frozen in liquid nitrogen for subsequent detection.

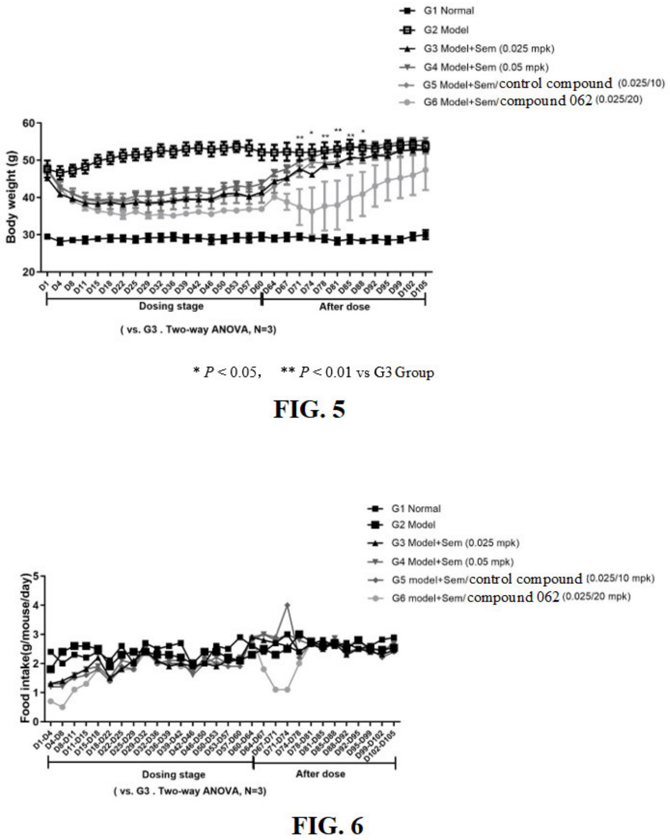

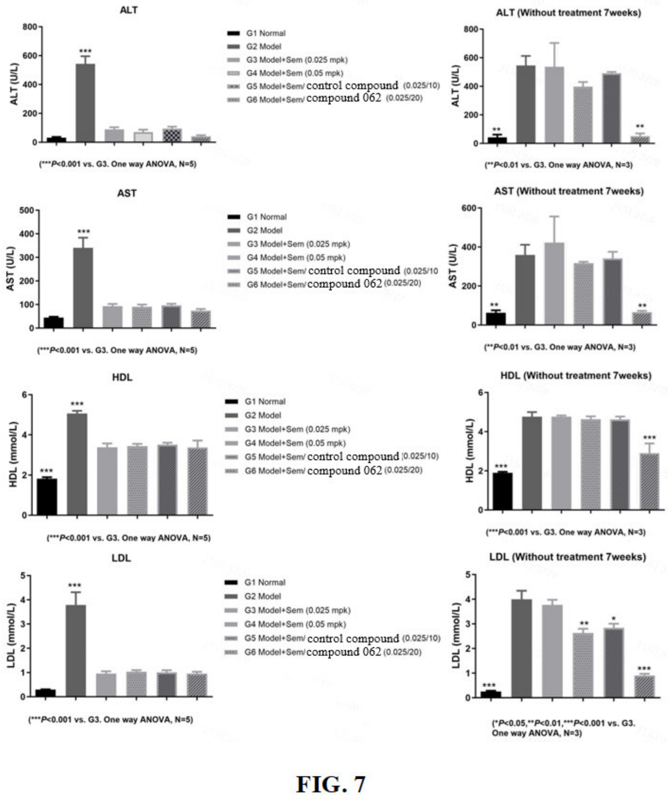

The experimental results are shown in FIGS. 5-7. The combination of compound 062 and 0.025 mpk of semaglutide significantly inhibited the rebound of mouse body weight after drug withdrawal without affecting food intake. However, neither the control compound combined with semaglutide nor the groups of low/high dose semaglutide alone inhibited the rebound of body weight after drug withdrawal. The blood biochemical indexes detected showed that the combination of compound 062 and 0.025mpk of Smeglutide significantly inhibited the levels of ALT, AST, LDL and HDL after drug withdrawal.

### Test Example 7 14-day toxicity experiment on rats

### 7.1 Test objective

SD rats were orally gavaged with compound 062 and the control compound once a day for a total of 14 times. The toxic reactions caused by compound 062 and the control compound were observed and their toxicokinetics were studied.

### 7.2 Test sample preparation and dosage setting

### 7.2.1 Preparation of vehicle formulations

### Vehicle 1: [0.2% CMC-Na]

Take the preparation of a formulation of 100 mL as an example: an appropriate amount of ultrapure water was measured and put into an appropriate container, 0.2 g of CMC-Na was weighed and added to the above container while stirring, and the mixture was continuously stirred until a clear solution was obtained, and water was added to make up to 100 mL for later use. The solution was stored at 2-8°C with a shelf life of 1 month.

Vehicle control group formulation [5% Solutol HS15 in 95% (0.2% CMC-Na)]:
Take the preparation of a formulation of 100 mL as an example: an appropriate amount of vehicle 1 was measured and put into a suitable container, a certain amount of Solutol HS15 was taken and fully melt at 40-60°C, then 5 mL of the melted Solutol HS15 was measured, and slowly added dropwise while stirring. The mixture was continuously stirred for at least 30 min to make a clear liquid. The container was covered with a lid or sealed with plastic wrap during stirring to prevent water evaporation. The formulation was stored at 2-8°C for later use, with a shelf life of 1 month.

### 7.2.2 Formulations for test sample 062 and test sample control compound

An appropriate amount of the test compound was weighed (weighed amount = theoretical required amount/content), and fully ground and dispersed in a mortar (agate or ceramic). Then, an appropriate amount of [5% Solutol HS15 in 95% (0.2% CMC-Na)] was added and the mixture was ground until there were no obvious large particles. Then the mortar was cleaned with [5% Solutol HS15 in 95% (0.2% CMC-Na)] in small amounts several times until there was no drug powder residue observed with the naked eye. The mixture was transferred to a suitable container, well stirred and mixed, and the remaining [5% Solutol HS15 in 95% (0.2% CMC-Na)] was further added to the required volume (streaking method). The system was stirred and mixed for at least 20 min, thereby giving the test sample formulation.

The prepared formulations should be labeled with concentration, and the label should at least include the test number, test sample code or name, concentration, dosage, storage conditions, preparation date, preparer and expiration date.

### 7.2.3 Dosage

The dosage is set as shown in Table 3.

**Table 3**

| Group No. | Group type | Dose (mg/kg) | Concentration (mg/mL) |
|---|---|---|---|
| 1 | Vehicle control group | / | / |
| 2 | 062 low dose group | 30 | 3 |
| 3 | 062 medium dose group | 60 | 6 |
| 4 | 062 high dose group | 100 | 10 |
| 5 | Control compound low dose group | 30 | 3 |
| 6 | Control compound medium dose group | 60 | 6 |
| 7 | Control compound high dose group | 100 | 10 |

### 7.3 Experimental animals

Species/strain: SD rats, SPF grade.

Sex and number of animals for selection: 67 female animals and 67 male animals.

Sex and number of animals used in the experiment: 56 female animals and 56 male animals.

Body weight and age at the time of grouping: male animals: 160-300 g, 6-9 weeks old; female animals: 160-300 g, 6-9 weeks old.

Animal Source: Zhejiang Vital River Laboratory Animal Technology Co., Ltd.

### 7.4. Experimental method

### 7.4.1 Dosing

Oral gavage. The drug was stirred for at least 30 min prior to administration and continuously stirred throughout the administration. The dosing frequency was once a day for 14 consecutive days, and the dosing amount was 10 mL/kg (the dosing amount was calculated based on the most recent weight).

### 7.4.2 Observation and inspection

### 7.4.2.1 Death and moribundity

The dosing period: all surviving animals were observed at least once a day.

### 7.4.2.2 General observations

Acclimation period: All animals were observed once a day (at least 1 day prior to grouping, including the day of grouping)

Dosing period: All surviving animals in the main test group were observed twice a day, once before and after dosing respectively, and observed in detail at least once a week.

Observation contents: The main observation contents include (but not limited to) survival status, injection site, skin, hair, eyes, ears, nose, mouth, chest, abdomen, urogenital area, limbs, as well as breathing, movement, urination, defecation, mental or behavioral changes, etc.

Note: If symptoms are observed at other times, the symptoms and observation time should be recorded promptly.

### 7.4.2.3 Weight

Acclimation period: All animals were weighed once before grouping, that is, grouping weight (D-1).

Dosing period: All surviving animals were weighed at least once a week. The body weight of animals in the toxicokinetics (TK) group was only used to calculate the dosage. The body weight data were not statistically analyzed and were only presented as individual data in the summary report.

Planned autopsy: All animals in the main experimental group will be weighed before the planned autopsy (fasted overnight), which is only used to calculate the amount of anesthesia. The fasting weight data will be presented in the summary report.

### 7.4.2.4 Food intake

Dosing period: The food intake of the surviving animals in the main test group was weighed at least once a week, and the average food intake was expressed in g/animal/day.

### 7.4.3 Toxicokinetic study (TK)

### 7.4.3.1 Blood collection date

Different time points before and after administration on D1 and D14.

### 7.4.3.2 Animals

All animals in the TK group survived.

### 7.4.3.3 Blood collection time point

Group 1: Before dosing and 4 h after dosing.

Group 2 to Group 7: Before dosing and 0.5 h, 1 h, 2 h, 4 h, 8 h and 24 h after dosing (before dosing on D2 and/or D15).

Note: The error range of blood collection time after completion of dosing is ±5 min at time point of 0.5 to 1 h, ±10 min at time points of 2 to 8 h, and ±1 h at time point of 24 h.

### 7.4.3.4 Blood collection site

Jugular vein.

### 7.4.3.5 Blood collection volume

Approximately 0.2 mL of whole blood/animal/time point.

### 7.4.3.6 Anticoagulants

EDTA-K2.

### 7.4.3.7 Preparation for blood collection and blood sample processing

Whole blood was collected into a blood collection tube containing EDTA-K2, the tube was immediately and gently inverted upside down several times to mix the system thoroughly, and placed on crushed ice/ice pack. Blood was centrifuged (2000 g, 10 min) at 4°C (set temperature, allowable temperature range: 2 to 8°C) within 1 h after blood collection. The collected plasma was divided into two portions into separate labeled EP tubes (one for testing and one for standby testing), and stored in an ultra-low temperature refrigerator (-70°C to -90°C) within 2 h after collection. The samples were stored in dry ice during transportation and transfer.

### 7.4.3.8 Treatment of animals in TK group

After the last blood sample was collected, the surviving TK animals were euthanized by carbon dioxide overdose.

### 7.4.3.9 Data processing

The drug concentration in plasma was analyzed by using LC-MS/MS, verified blood drug concentration data was provided, and the main toxicokinetic parameters (AUC0-t, Cmax and Tmax) were calculated and compared.

### 7.4.4 Clinical pathological examination

### 7. 4.4.1 Hematology and coagulation index test

End of dosing period: once;
Blood sampling route: abdominal aorta;
Animals: Plan to dissect surviving animals;
Blood was collected into tubes containing anticoagulant (EDTA-K2), and hematological indices were detected using a Sysmex XN-1000V fully automatic blood analyzer; blood was collected into anticoagulant tubes containing sodium citrate, and coagulation indices were detected using a Sysmex CS-5100 coagulometer. The specific detection indices are as follows:

| Index | Unit | **Provantis** system abbreviation | Analytical method |
|---|---|---|---|
| Red blood cell (RBC) | 10^6/µL | RBC. | Instrument count |
| Hemoglobin (HGB) | g/dL | HGB. | Instrument count |
| Hematocrit (HCT) | % | HCT. | Instrument count |
| Mean corpuscular volume (MCV) | fL | MCV. | Instrument count |
| Mean corpuscular hemoglobin (MCH) | pg | MCH. | Instrument count |
| Mean corpuscular hemoglobin concentration (MCHC) | g/dL | .MCHC | Instrument count |
| Reticulocyte percentage (RET%) | % | .RET% | Instrument count |
| Absolute reticulocytes (RET#) | 10^9/L | .#RET | Instrument count |
| Red cell distribution width - coefficient of variation (RDW-CV) | % | RDW-CV. | Instrument count |
| Platelet count (PLT&O) | 10^3/µL | PLT. | Instrument count |
| Mean platelet volume (MPV) | fL | MPV. | Instrument count |
| Plateletocrit (relative number) (PCT) | % | PCT. | Instrument count |
| White blood cell count (WBC) | 10^3/µL | WBC. | Instrument count |
| Absolute neutrophil count (NEUT#) | 10^3/µL | #NEU | Instrument count |
| Absolute lymphocyte count (LYMPH#) | 10^3/µL | #LYMP | Instrument count |
| Absolute monocyte count (MONO#) | 10^3/µL | #MONO | Instrument count |
| Absolute eosinophil count (EO#) | 10^3/µL | #EOS | Instrument count |
| Absolute basophil count (BASO#) | 10^3/µL | #BAS | Instrument count |
| Neutrophil percentage (NEUT%) | % | .NEU% | Instrument count |
| Lymphocyte percentage (LYMPH%) | % | .LYM% | Instrument count |
| Monocyte percentage (MONO%) | % | .MON% | Instrument count |
| Eosinophil percentage (EO%) | % | .EOS% | Instrument count |
| Basophil percentage (BASO%) | % | .BAS% | Instrument count |
| Prothrombin time (PT) | sec | PT. | Solidification method |
| Thrombin time (TT) | sec | TT | Solidification method |
| Activated partial thromboplastin time (APTT) | sec | .APTT | Solidification method |
| Fibrinogen (Fbgc) | g/L | .Fbgc | Solidification method |

### 7.4.4.2 Serum biochemical index detection

End of dosing period: once;
Blood sampling route: abdominal aorta;
Animals: Plan to dissect surviving animals;

The animals were fasted overnight, and whole blood was collected into a test tube containing a coagulant and a separation gel. Serum was obtained by centrifugation, and serum biochemical indices were detected using a Hitachi-7180 biochemical analyzer. The specific detection indices are as follows:

| Serum biochemical indexes | Unit | PROVANTIS corresponding abbreviation | Analytical method |
|---|---|---|---|
| Alanine aminotransferase (ALT) | U/L | ALT. | Rate method |
| Aspartate aminotransferase (AST) | U/L | AST. | Rate method |
| Alkaline phosphatase (ALP) | U/L | ALP. | Rate method |
| Creatine kinase (CK) | U/L | CK. | Rate method |
| Total serum bilirubin (TBIL) | µmol/L | TBIL | Vanadate oxidation method |
| Direct bilirubin (DBIL) | µmol/L | DBIL | Vanadate oxidation method |
| Total cholesterol (CHOL) | mmol/L | .CHOL | Oxidase method |
| Triacylglycerol (triglyceride) (TG) | mmol/L | TG. | Enzymatic removal of free glycerol |
| Blood glucose (GLU) | mmol/L | GLU. | Hexosidase method |
| Blood urea nitrogen (BUN) | mmol/L | BUN. | Urease method |
| Creatinine (CREA) | µmol/L | .CREA | Creatinase method |
| Sodium (Na) | mmol/L | Na. | Ion selective electrode method |
| Potassium (K) | mmol/L | K. | Ion selective electrode method |
| Chlorine (Cl) | mmol/L | Cl. | Ion selective electrode method |
| Calcium (Ca) | mmol/L | Ca. | MXB method |
| Inorganic phosphate (P) | mmol/L | P. | Direct UV method |
| Total protein (TP) | g/L | TP. | Biuret method |
| Albumin (ALB) | g/L | ALB. | Bromocresol green method |
| Globulin (GLO) | g/L | GLO. | Calculation |
| Albumin/globulin (A/G) | N/A | A/G. | Calculation |

### 7.4.5 End-of-trial procedure

### 7.4.5.1 Unplanned animal deaths

All animals with unplanned deaths (including dying animals) should undergo gross autopsy (see 7.4.5.2 for examination contents), and any abnormal organs/tissues should be collected and preserved. Blood should be collected from dying animals as much as possible for examination of clinical pathological indices.

The carcasses of animals that die outside working hours can be stored at 2-8°C until the next working day for gross autopsy.

### 7.4.5.2 Gross anatomy

Dissection time: end of dosing period (D15);
Anesthesia method: The specifications of the anesthetic mixture are ketamine of 37.5 mg/mL and xylazine of 2.5 mg/mL. The euthanasia dose is 2-4 mL/kg of the mixture (the anesthetic dose of the animal planned for dissection is calculated based on the body weight after fasting overnight, the concentration will be recorded in the original record and presented in the summary report, and the dose can be adjusted according to the anesthetic effect) by intraperitoneal injection.

Sacrificling method: The animals were sacrificed by bleeding from the abdominal aorta after anesthesia.

| Name | Lot No. | Source | Storage conditions | Valid until |
|---|---|---|---|---|
| Tamiankang Ketamine Hydrochloride Injection | 20220216 | Jiangsu CABK Pharmaceutical Co., Ltd. | Airtight storage | 2024.02.15 |
| Xylazine hydrochloride injection | 20230706 | Dunhua Shengda Animal Pharmaceutical Co., Limited | Airtight storage at 2-8°C | 2025.06.30 |
| Zoletil^{®}50 | 93LWA | VIRBAC Corporation, France | Room temperature, light-proof, airtight | 2024.11.30 |

Gross anatomical observations: All animals were examined and recorded for gross abnormalities of all tissues and organs sampled during autopsy, and the following features were observed (including but not limited to): 1. the body and the musculoskeletal system; 2. the animal's body surface and natural orifices; 3. the outer surface of the cranial cavity and brain; 4. the thoracic, abdominal and pelvic cavities and the organs within the cavities.

If any abnormality is found, the location, color, shape and size of the abnormal tissue or organ should be recorded, and the abnormal organ or tissue should be collected and preserved.

### 7.4.6 Statistical analysis

Body weight (including weight gain), hematology, coagulation, and serum biochemical indices are expressed as mean ± SD (according to sex). When n≥3, groups 2 to 7 were compared with group 1, the following statistical methods are used: ① Levene's test is used to detect the homogeneity of variance of the data. If the variance is homogeneous (P>0.05), one-way analysis of variance (ANOVA) is performed; if the variance is not homogeneous (*P*≤0.05), the data are subjected to common logarithmic transformation and Levene's test is used again to detect the homogeneity of variance of the data (Log data). If the variance is homogeneous (*P*>0.05), one-way analysis of variance (ANOVA) is performed using the Log data; if the variance not homogeneous (*P*≤0.05), the Log data are subjected to rank transformation, followed by Kruskal-Wallis test (Rank data). ② If the results of the analysis of variance (ANOVA) are statistically different (*P*≤0.05), Dunnett's t test is further used for inter-group comparison test (0.05 and 0.01 levels); if the results of the analysis of variance (ANOVA) are not statistically different (*P*>0.05), the statistics ends. ③If the results of the Kruskal-Wallis test are statistically significant (*P*≤0.05), the Rank data are further subjected to inter-group comparison using the Dunnett t test (0.05 and 0.01 levels); if the results of the Kruskal-Wallis test are not statistically significant (P>0.05), the statistics ends. When n = 2, data are presented only as mean ± SD by sex, and no statistical inference is performed.

Moribundity/death observations, food intake and general observations are not counted and are presented as individual data and/or frequency.

### 7.5 Experimental result

### 7.5.1 Toxicokinetic data of control compound and compound 062

Control compound:

| Dose (mpk) | 30 | | 60 | | 100 | |
|---|---|---|---|---|---|---|
| Day | Day 1 | Day 14 | Day 1 | Day 14 | Day 1 | Day 14 |
| Cₘₐₓ (ng/mL) | 17500 | 13200 | 21400 | 22450 | 23950 | 26700 |
| AUC(0-24) (h*ng/mL) | 248500 | 202000 | 360000 | 324500 | 405000 | 439000 |

Compound 062:

| Dose (mpk) | 30 | | 60 | | 100 | |
|---|---|---|---|---|---|---|
| Day | Day 1 | Day 14 | Day 1 | Day 14 | Day 1 | Day 14 |
| Cₘₐₓ (ng/mL) | 19900 | 31700 | 41100 | 68550 | 68150 | 127000 |
| AUC(0-24) (h*ng/mL) | 279000 | 384500 | 621500 | 1120500 | 1117000 | 2115000 |

### 7.5.2 Safety data of control compound and compound 062

| Observation index | Test sample 1 (control compound) | Test sample 2 (compound 062) |
|---|---|---|
| Dose design | Dosage (mg/kg/day): 30 (low), 60 (medium), 100 (high); | Dosage (mg/kg/day): 30 (low), 60 (medium), 100 (high); |
| | Dosing volume: 10 mL/kg | |
| | | Dosing volume: 10 mL/kg |
| Death/moribundity | High-dose group (100 mg/kg): One male animal (4101#) in the main test group was found dead before dosing on D4, and one male animal (4006#) in the TK group was found dead before dosing on D6. Gross autopsy showed discoloration of the lungs and/or liver. | None |
| | Clinical observation of dead animals before death: No obvious abnormality was observed in animal 4101# before death, and the body weight decreased slightly (vs before dosing: ↓6.25%); before death, animal 4006# showed swelling of limbs and ears, erect hair and arched back, decreased activity, and significant weight loss (vs before dosing: ↓28.4%) | |
| Clinical observation | 30 mg/kg: Both male and female animals (females: 2/5, males: 4/5) developed swelling of the left and right hind limbs and ears; At ≥60 mpk: Both males and females experienced limb swelling, decreased activity, and/or ear swelling; in addition, at 100 mpk: both males and females experienced bilateral ocular discharge, tearing, erect hair, and hunched backs. | No significant abnormalities observed |
| Weight/food intake | Body weight: ≥30 mg/kg: vs. control group, both male and female animals showed body weight reduction on D7 and D14 (D7: low: ↓ 7.7%, medium: ↓16.7%, high: ↓ 22.5%; D14: low: ↓ 8.5%, medium: ↓ 14.6%, high: ↓ 23.3%), low body weight gain, with the reduction significantly dose-related. | No significant abnormalities observed |
| | Food intake: ≥30 mg/kg: vs. control group, males and females: food intake decreased from D1 to D7, and recovered or showed a trend of recovery from D7 to D14 | |
| Hematology/coagulation | Males: vs. control group, dose ≥30 mg/kg, #MONO increased (low:↑ 154.4%, medium:↑215.8%, high:T 313.0%); ≥100 mg/kg: MONO% ↑160.3%, #EOS ↑816.7% and EOS% ↑608.3% were also observed, and the changes were dose-related; | No significant abnormalities observed |
| | Females: At a dose of ≥30 mg/kg, #MONO (low:↑ 187.7%, medium:T 237.0%, high:↑ 965.4.0%) and MONO% (low:↑63.6%, medium:↑ 100.6%, high:T 290.3%) increased; at a dose of ≥60 mg/kg, #NEU increased (medium: ↑ 251.8%, high:T 687.1%); at a dose of ≥100 mg/kg, #BAS (↑400.0%) and NEU% (↑195.6%) increased, and the changes were dose-related. | |
| Serum biochemistry | Serum biochemistry: vs. control group, females: at dose ≥ 100 mg/kg, ALB (128.5%) and A/G (↓53.6%) were significantly reduced, GLO (:↑ 61.3%) was significantly increased | No significant abnormalities observed |
| Gross anatomy | Animals with planned autopsy: no obvious abnormalities were found in the gross autopsy; animals with unplanned autopsy: discoloration of the lungs and/or liver was found in the gross autopsy. | No significant abnormalities observed |

Conclusion: Compound 062 has good safety. When the exposure in the high-dose group is 4.8 times that of the control compound, its safety is much better than that of the control compound.

In the absence of other instructions, the control compounds described in the present application document are compounds known in the prior art and have the following structure:

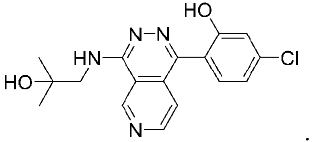

The embodiments of the technical solution of the present invention is described in an illustrative manner above. It is to be understood that the scope of protection of the present invention is not limited to the above-mentioned embodiments. Any modifications, equivalent substitutions, improvements, etc. made by those skilled in the art within the spirit and principles of the present invention should be included in the scope of protection of the claims of the present application.

## Claims

1. A compound represented by formula (I), a racemate, a stereoisomer, a tautomer, an isotope-labeled counterpart, a solvate, a polymorph, a pharmaceutically acceptable salt thereof or a prodrug thereof: 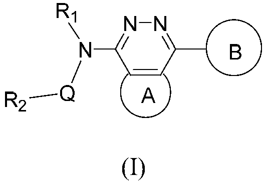
wherein ring A is selected from a C₃₋₁₄ carbocyclic ring, a C₆₋₁₄ aromatic ring, a 5- to 14-membered heteroaromatic ring, or a 3- to 14-membered heterocyclic ring, wherein the C₃₋₁₄ carbocyclic ring, C₆₋₁₄ aromatic ring, 5- to 14-membered heteroaromatic ring and 3- to 14-membered heterocyclic ring are each independently unsubstituted or optionally substituted with one, two or more Rₐ; each Rₐ is the same or different and is independently selected from H, deuterium, OH, halogen, cyano, NH₂, or the following groups which are unsubstituted or optionally substituted with one, two or more Rₐ₁: C₁₋₁₂ alkyl, C₂₋₁₂ alkenyl, C₂₋₁₂ alkynyl, C₁₋₁₂ alkoxy, Rₐ₁₁-C(=O)-NH-, Rₐ₁₂-C(=O)-, Rₐ₁₃-S(=O)₂-NH-, Rₐ₁₄-S(=O)₂-, -P(=O)(Rₐ₁₅)(Rₐ₁₆), (Rₐ₁₇)(Rₐ₁₈)N-C(=O)-, C₆₋₁₄ aryl, 5- to 14-membered heteroaryl, 3- to 14-membered heterocyclyl, or C₃₋₁₄ cycloalkyl; each Rₐ₁ is the same or different and is independently selected from H, deuterium, OH, halogen, CN, C₁₋₁₂ alkyl, C₃₋₁₂ cycloalkyl, C₁₋₁₂ alkoxy, or halo-C₁₋₁₂ alkyl; Rₐ₁₁, Rₐ₁₂, Rₐ₁₃, Rₐ₁₄, Rₐ₁₅, Rₐ₁₆, Rₐ₁₇, and Rₐ₁₈ are the same or different and are each independently selected from H, deuterium, halogen, CN, C₁₋₁₂ alkyl, or C₁₋₁₂ alkoxy;
ring B is selected from a C₆₋₁₄ aromatic ring, a 5- to 14-membered heteroaromatic ring, a 3- to 14-membered heterocyclic ring, or a C₃₋₁₄ carbocyclic ring, wherein the C₆₋₁₄ aromatic ring, 5- to 14-membered heteroaromatic ring, 3- to 14-membered heterocyclic ring and C₃₋₁₄ carbocyclic ring are each independently unsubstituted or optionally substituted with one, two or more R_{b}; each R_{b} is the same or different and is independently selected from H, deuterium, OH, halogen, cyano, NH₂, or the following groups which are unsubstituted or optionally substituted with one, two or more R_{b1}: C₁₋₁₂ alkyl, or C₁₋₁₂ alkoxy; each R_{b1} is the same or different and is independently selected from H, deuterium, halogen, CN, C₁₋₁₂ alkyl, or C₁₋₁₂ alkoxy;
R₁ is selected from H, deuterium, halogen, CN, hydroxyl, amino, C₁₋₆ alkyl, halo-C₁₋₆ alkyl, cyano-C₁₋₆ alkyl, or C₃₋₈ cycloalkyl;
Q is selected from a chemical bond or C₁₋₆ alkylene;
R₂ is selected from the following groups which are unsubstituted or optionally substituted with one, two or more R₂ₐ: C₁₋₁₂ alkyl, C₁₋₁₂ alkoxy, C₆₋₁₄ aryl, 5- to 14-membered heteroaryl, 3- to 14-membered heterocyclyl, or C₃₋₁₄ cycloalkyl; each R₂ₐ is the same or different and is independently selected from H, deuterium, halogen, CN, OH, carboxyl, or the following groups which are unsubstituted or optionally substituted with one, two or more R₂ₐ₁: C₁₋₁₂ alkyl, C₁₋₁₂ alkoxy, or R₂₁-C(=O)-(CH₂)ₘ-; or two R₂ₐ together with the carbon atom to which they are attached form C₃₋₈ cycloalkyl or 3-8 membered heterocyclyl; each R₂ₐ₁ is the same or different and is independently selected from H, deuterium, halogen, CN, carboxyl, C₁₋₁₂ alkyl, C₁₋₁₂ alkoxy, or R₂₂-C(=O)-; each R₂₁ and R₂₂ are the same or different and are each independently selected from H, deuterium, halogen, CN, OH, amino, C₁₋₁₂ alkylamino, (C₁₋₁₂ alkyl)₂amino, C₃₋₁₂ cycloalkylamino, C₁₋₁₂ alkyl, C₁₋₁₂ alkoxy, C₃₋₈ cycloalkyl, or 3- to 14-membered heterocyclyl; m is an integer selected from 0 to 6;
alternatively, R₂ is selected from 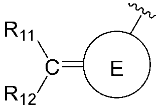 which is unsubstituted or optionally substituted with one, two or more Rₑ, wherein ring E is selected from C₃₋₈ cycloalkyl; R₁₁ and R₁₂ are the same or different and are each independently selected from H, halogen, C₁₋₆ alkyl, halo-C₁₋₆ alkyl, cyano-C₁₋₆ alkyl, C₃₋₈ cycloalkyl, halo-C₃₋₈ cycloalkyl or cyano-C₃₋₈ cycloalkyl; each Rₑ is the same or different and is independently selected from H, OH, halogen, cyano, NH₂, or the following groups which are unsubstituted or optionally substituted with one, two or more Rₑ₁: C₁₋₆ alkyl, or C₃₋₈ cycloalkyl; each Rₑ₁ is the same or different and is independently selected from H, OH, halogen, or cyano;
alternatively, R₂ is selected from 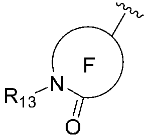 which is unsubstituted or optionally substituted with one, two or more R_{f}, wherein ring F is selected from 4- to 8-membered heterocyclic ring; R₁₃ is selected from H, or the following groups which are unsubstituted or optionally substituted with one, two or more R₁₃ₐ: C₁₋₆ alkyl, C₃₋₈ cycloalkyl, or R_{13b}-C(=O)-(CH₂)ₛ-; R_{13b} is selected from C₁₋₆ alkyl, C₃₋₈ cycloalkyl or amino; each R₁₃ₐ is the same or different and is independently selected from H, OH, halogen, cyano, or C₁₋₆ alkyl; s is selected from integers of 0 to 6; each R_{f} is the same or different and is independently selected from H, OH, halogen, cyano, NH₂, or the following groups which are unsubstituted or optionally substituted with one, two or more R_{f1}: C₁₋₆ alkyl, or C₃₋₈ cycloalkyl; each R_{f1} is the same or different and is independently selected from H, OH, halogen, or cyano.

2. The compound according to claim 1, wherein ring A is selected from a C₃₋₈ carbocyclic ring, a C₆₋₁₀ aromatic ring, a 5- to 10-membered heteroaromatic ring, or a 3- to 10-membered heterocyclic ring, wherein the C₃₋₈ carbocyclic ring, C₆₋₁₀ aromatic ring, 5- to 10-membered heteroaromatic ring and 3- to 10-membered heterocyclic ring are each independently unsubstituted or optionally substituted with one, two or more Rₐ; each Rₐ is the same or different and is independently selected from H, deuterium, OH, halogen, cyano, NH₂, or the following groups which are unsubstituted or optionally substituted with one, two or more Rₐ₁: C₁₋₆ alkyl, C₂₋₆ alkenyl, C₂₋₆ alkynyl, C₁₋₆ alkoxy, Rₐ₁₁-C(=O)-NH-, Rₐ₁₂-C(=O)-, Rₐ₁₃-S(=O)₂-NH-, Rₐ₁₄-S(=O)₂-, - P(=O)(Rₐ₁₅)(Rₐ₁₆), (Rₐ₁₇)(Rₐ₁₈)N-C(=O)-, C₆₋₁₀ aryl, 5- to 10-membered heteroaryl, 3- to 10-membered heterocyclyl, or C₃₋₁₀ cycloalkyl; each Rₐ₁ is the same or different and is independently selected from H, deuterium, OH, halogen, CN, C₁₋₆ alkyl, C₃₋₆ cycloalkyl, or C₁₋₆ alkoxy; Rₐ₁₁, Rₐ₁₂, Rₐ₁₃, Rₐ₁₄, Rₐ₁₅, Rₐ₁₆, Rₐ₁₇, and Rₐ₁₈ are the same or different and are each independently selected from H, deuterium, halogen, CN, C₁₋₆ alkyl, or C₁₋₆ alkoxy;
preferably, ring A is selected from a C₆₋₁₀ aromatic ring or a 5- to 10-membered heteroaromatic ring, wherein the C₆₋₁₀ aromatic ring and 5- to 10-membered heteroaromatic ring are each independently unsubstituted or optionally substituted with one, two or more Rₐ; each Rₐ is the same or different and is independently selected from H, deuterium, OH, halogen, cyano, NH₂, or the following groups which are unsubstituted or optionally substituted with one, two or more Rₐ₁: C₁₋₆ alkyl, C₂₋₆ alkenyl, C₂₋₆ alkynyl, Rₐ₁₂-C(=O)-, Rₐ₁₄-S(=O)₂-, (Rₐ₁₇)(Rₐ₁₈)N-C(=O)-, C₆₋₁₀ aryl, or 5- to 10-membered heteroaryl; each Rₐ₁ is the same or different and is independently selected from H, deuterium, OH, halogen, CN, C₁₋₆ alkyl, C₃₋₆ cycloalkyl, or C₁₋₆ alkoxy; Rₐ₁₂, Rₐ₁₄, Rₐ₁₇, and Rₐ₁₈ are the same or different and are each independently selected from H, C₁₋₆ alkyl, or C₁₋₆ alkoxy;
preferably, ring A is selected from a benzene ring or a pyridine ring which is unsubstituted or optionally substituted with one, two or more Rₐ; each Rₐ is the same or different and is independently selected from H, cyano, -C(=O)-NH₂, CH₃-S(=O)₂-, 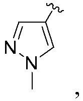 1-propynyl, 2-cyclopropylethynyl, 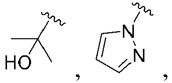 acetyl, 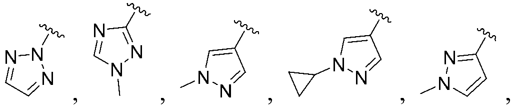 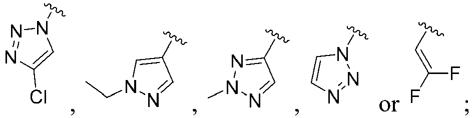
preferably, ring A is selected from 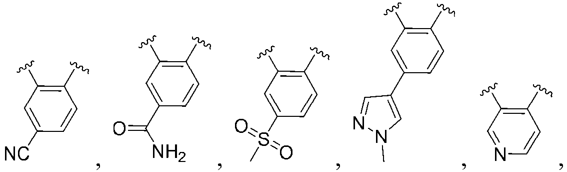 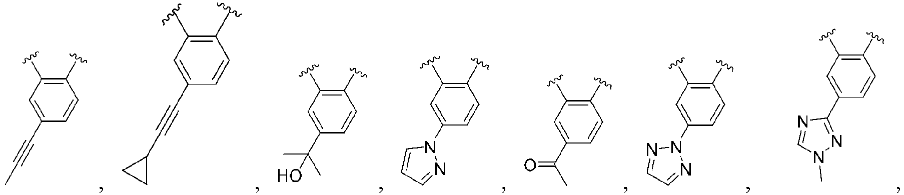 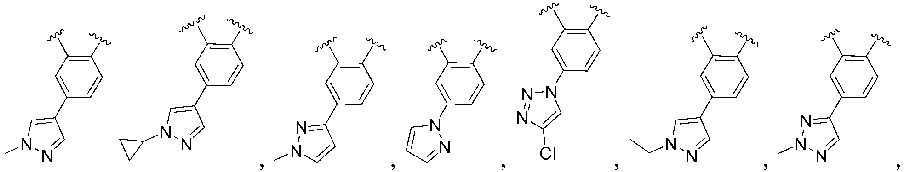 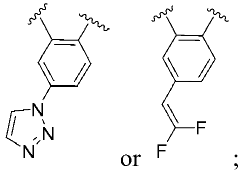
preferably, 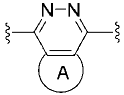 is selected from

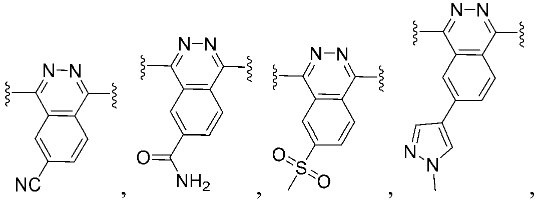

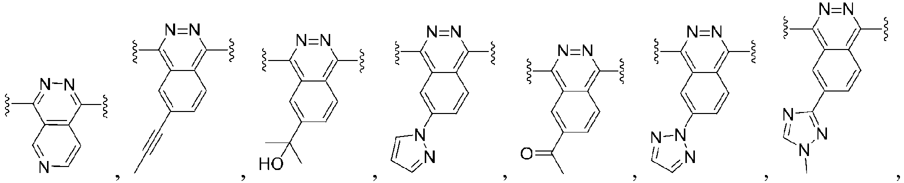

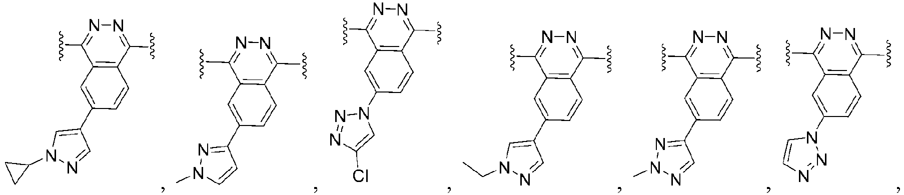

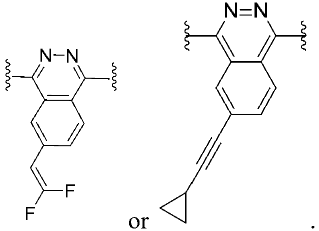

3. The compound according to claim 1 or 2, wherein ring B is selected from a C₆₋₁₀ aromatic ring, a 5- to 10-membered heteroaromatic ring or a C₃₋₁₀ carbocyclic ring, wherein the C₆₋₁₀ aromatic ring, 5- to 10-membered heteroaromatic ring and C₃₋₁₀ carbocyclic ring are each independently unsubstituted or optionally substituted with one, two or more R_{b}; each R_{b} is the same or different and is independently selected from H, OH, halogen, cyano, NH₂, or the following groups which are unsubstituted or optionally substituted with one, two or more R_{b1}: C₁₋₆ alkyl, or C₁₋₆ alkoxy; each R_{b1} is the same or different and is independently selected from H, halogen, CN, C₁₋₆ alkyl, or C₁₋₆ alkoxy;
preferably, ring B is selected from a benzene ring, a benzothiophene ring or an indane ring which is unsubstituted or optionally substituted with one, two or more R_{b}; each R_{b} is the same or different and is independently selected from H, OH, halogen, cyano, C₁₋₆ alkyl, halo-C₁₋₆ alkyl, or halo-C₁₋₆ alkoxy;
preferably, ring B is selected from a benzene ring, a benzothiophene ring or an indane ring which is unsubstituted or optionally substituted with one, two or more R_{b}; each R_{b} is the same or different and is independently selected from H, OH, F, Cl, methyl or CF₃;
preferably, ring B is selected from 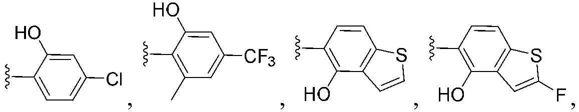 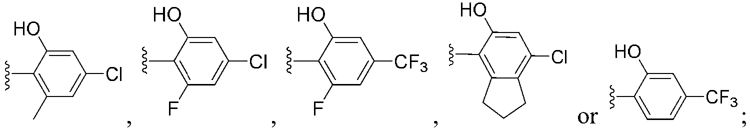
preferably, ring B is selected from 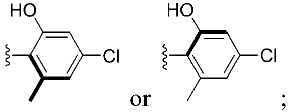
preferably, R₁ is selected from H, deuterium or methyl; preferably R₁ is H;
preferably, Q is selected from a chemical bond, -(CH₂)_{q}- or -(CH₂)_{q}-CH(CH₃)-, and q is selected from 0, 1 or 2;
preferably, Q is selected from a chemical bond, -CH₂- or -CH(CH₃)-.

4. The compound according to any one of claims 1 to 3, wherein R₂ is selected from the following groups which are unsubstituted or optionally substituted with one, two or more R₂ₐ: C₁₋₆ alkyl, C₁₋₆ alkoxy, C₆₋₁₀ aryl, 5- to 10-membered heteroaryl, 3- to 10 membered heterocyclyl, or C₃₋₁₀ cycloalkyl; each R₂ₐ is the same or different and is independently selected from H, deuterium, halogen, CN, OH, carboxyl, or the following groups which are unsubstituted or optionally substituted with one, two or more R₂ₐ₁: C₁₋₆ alkyl, C₁₋₆ alkoxy, or R₂₁-C(=O)-(CH₂)ₘ-; or two R₂ₐ together with the carbon atom to which they are attached form C₃₋₈ cycloalkyl; each R₂ₐ₁ is the same or different and is independently selected from H, deuterium, halogen, CN, carboxyl, C₁₋₆ alkyl, C₁₋₆ alkoxy, or R₂₂-C(=O)-; each R₂₁ and R₂₂ are the same or different and are each independently selected from H, OH, amino, C₁₋₆ alkylamino, (C₁₋₆ alkylamino)₂amino, C₃₋₆ cycloalkylamino, C₁₋₆ alkyl, C₁₋₆ alkoxy, C₃₋₈ cycloalkyl, or 3- to 8-membered heterocyclyl; m is selected from 0, 1 or 2;
preferably, R₂ is selected from the following groups which are unsubstituted or optionally substituted with one, two or more R₂ₐ: C₁₋₆ alkyl, C₆₋₁₀ aryl, 5- to 10-membered heteroaryl, 3- to 8-membered heterocyclyl, or C₃₋₈ cycloalkyl; each R₂ₐ is the same or different and is independently selected from H, CN, OH, carboxyl, or the following groups which are unsubstituted or optionally substituted with one, two or more R₂ₐ₁: C₁₋₆ alkyl, or R₂₁-C(=O)-(CH₂)ₘ-; or two R₂ₐ together with the carbon atom to which they are attached form C₃₋₆ cycloalkyl; each R₂ₐ₁ is the same or different and is independently selected from H, deuterium, halogen, CN, carboxyl, C₁₋₆ alkyl, C₁₋₆ alkoxy, C₃₋₆ cycloalkylamino, 3- to 6-membered N-containing heterocyclyl, or R₂₂-C(=O)-; each R₂₁ and R₂₂ are the same or different and are each independently selected from H, OH, amino, methyl, methoxy, or cyclopropyl; m is selected from 0, 1 or 2;
preferably, R₂ is selected from the following groups which are unsubstituted or optionally substituted with one, two or more R₂ₐ: C₁₋₆ alkyl, tetrahydropyrrolyl, piperidinyl, phenyl, cyclohexyl, cyclobutyl or tetrahydropyranyl; each R₂ₐ is the same or different and is independently selected from H, CN, OH, carboxyl, or the following groups which are unsubstituted or optionally substituted with one, two or more R₂ₐ₁: C₁₋₆ alkyl, or R₂₁-C(=O)-(CH₂)ₘ-; or two R₂ₐ together with the carbon atom to which they are attached form C₃₋₆ cycloalkyl; each R₂ₐ₁ is the same or different and is independently selected from H, deuterium, halogen, CN, carboxyl, C₁₋₆ alkyl, C₁₋₆ alkoxy, or R₂₂-C(=O)-; each R₂₁ and R₂₂ are the same or different and are each independently selected from H, OH, amino, methyl, methoxy, cyclopropyl, cyclopropylamino, or tetrahydropyrrolyl; m is selected from 0, 1 or 2;
preferably, R₂ is selected from the following groups which are unsubstituted or optionally substituted with one, two or more R₂ₐ: C₁₋₆ alkyl, tetrahydropyrrolyl, piperidinyl, phenyl, cyclohexyl, cyclobutyl or tetrahydropyranyl; each R₂ₐ is the same or different and is independently selected from H, OH, carboxyl, methyl, 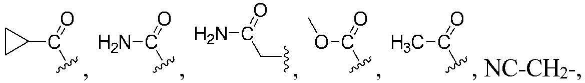 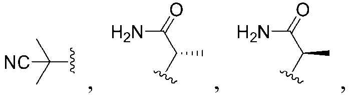 HOOC-CH₂-, HOOC-CH₂-CH₂-, 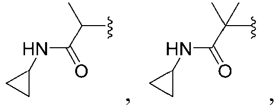 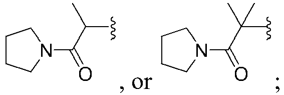 or two R₂ₐ together with the carbon atom to which they are attached form C₃₋₆ cycloalkyl;
preferably, R₂ is selected from 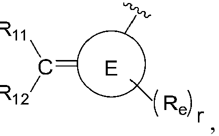 wherein ring E is selected from C₄₋₇cycloalkyl, and r is selected from integers of 0 to 6;
preferably, R₁₁ and R₁₂ are the same or different and are independently selected from H or halogen (such as F, Cl);
preferably, each Rₑ is the same or different and is independently selected from H or OH;
preferably, R₂ is selected from 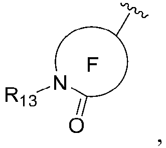 wherein ring F is selected from 4- to 7-membered heterocyclic ring;
preferably, R₂ is selected from 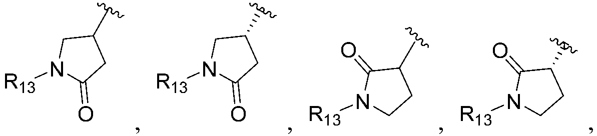 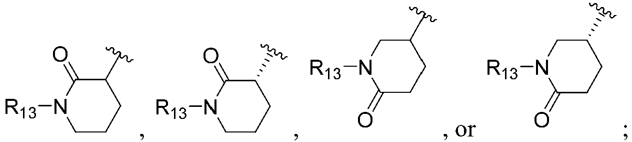
preferably, R₁₃ is selected from H, or the following groups which are unsubstituted or optionally substituted with one, two or more R₁₃ₐ: C₁₋₃ alkyl, C₃₋₆ cycloalkyl, or R_{13b}-C(=O)-(CH₂)ₛ-; R_{13b} is selected from C₁₋₃ alkyl, C₃₋₆ cycloalkyl or amino; each R₁₃ₐ is the same or different and is independently selected from H, OH, halogen, cyano, or C₁₋₃ alkyl; s is selected from 0, 1, 2 or 3;
preferably, R₁₃ is selected from hydroxy-C₁₋₆alkyl-, or NH₂-C(=O)-(CH₂)ₛ-, wherein s is selected from 0, 1, 2 or 3;
preferably, R₁₃ is selected from HOCH₂CH₂-, 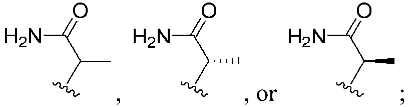
preferably, R₂ is selected from 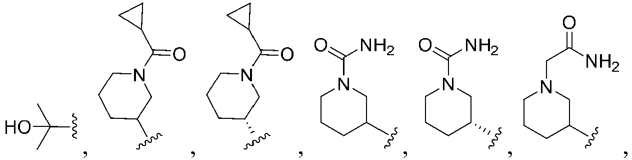 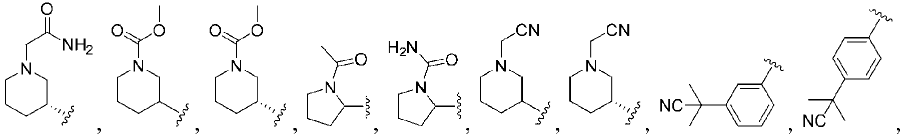 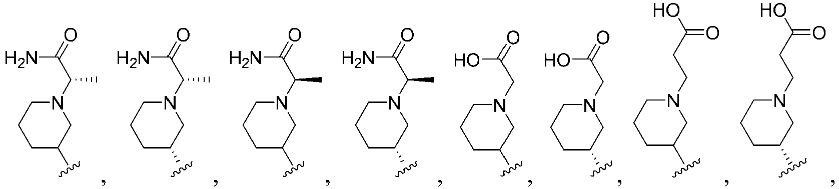 HOOC-CH₂-, 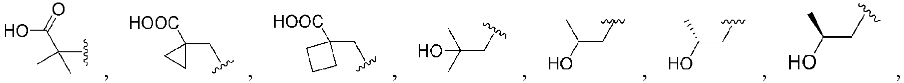 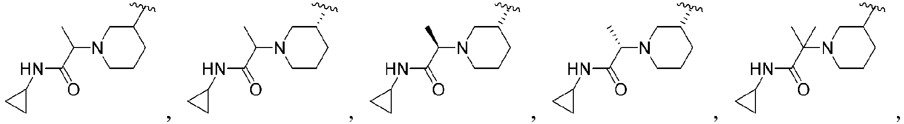 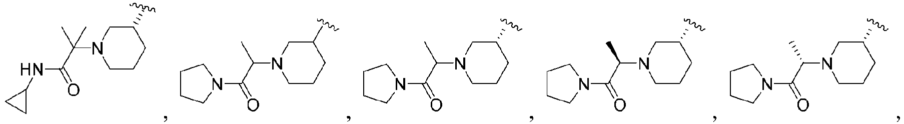 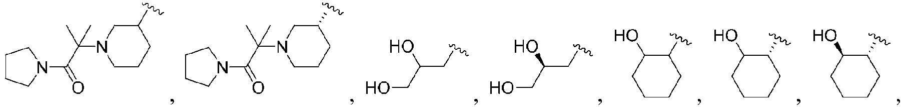 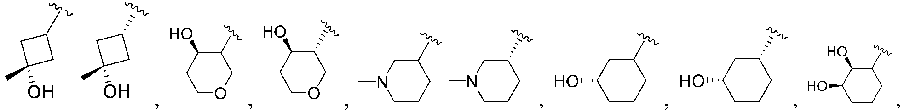 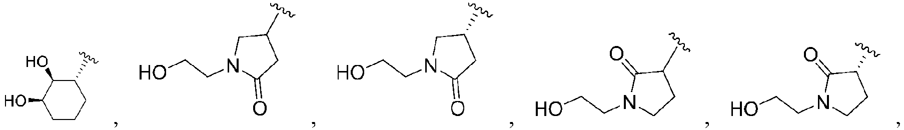 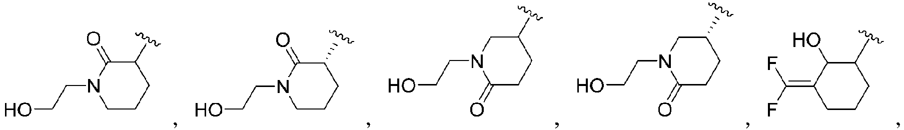 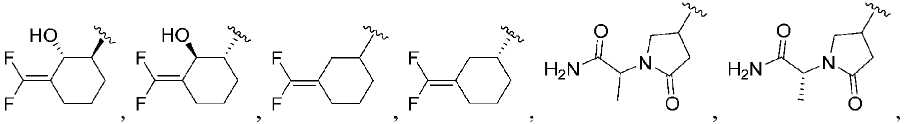 or

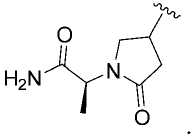

5. The compound according to any one of claims 1 to 4, wherein the compound represented by formula (I) is selected from the following structures: 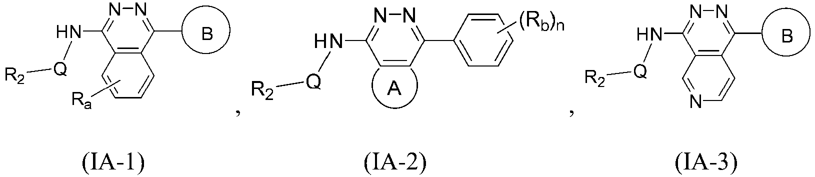
wherein, ring A, ring B, Q, R₂, Rₐ, and R_{b} are independently as defined in any one of claims 1 to 4; n is selected from 0, 1, 2, 3, 4 or 5;
preferably, the compound represented by formula (I) has the following structures: 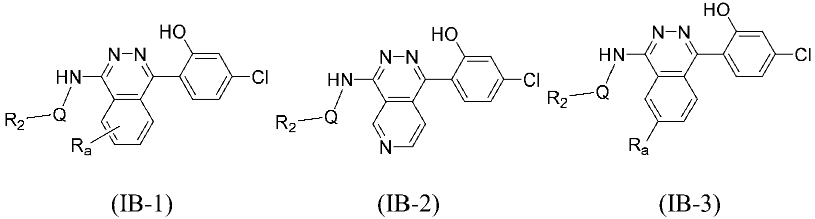 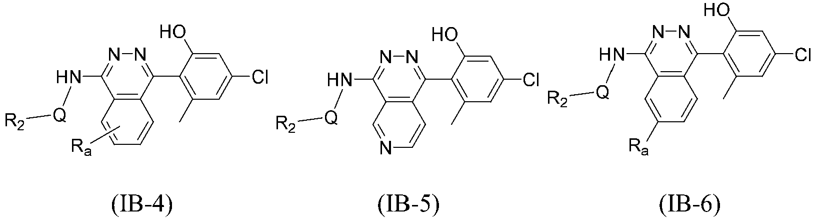
wherein Q, R₂, and Rₐ are each independently as defined in any one of claims 1 to 4;
preferably, the compound represented by formula (I) has the following structures: 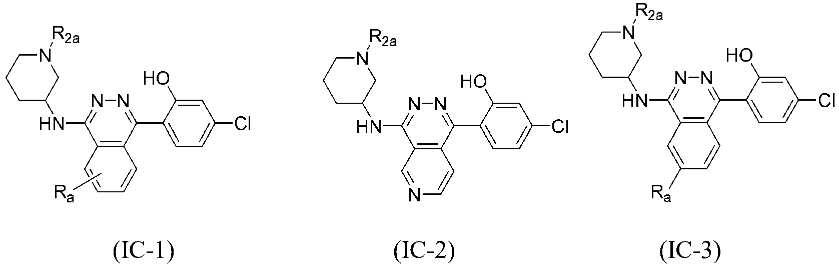 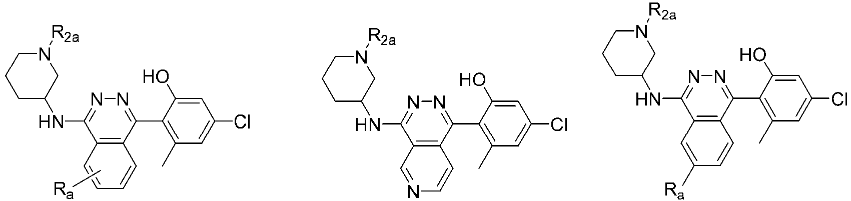 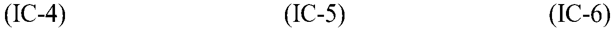
wherein R₂ₐ and Rₐ are each independently as defined in any one of claims 1 to 4;
preferably, the compound represented by formula (I) has the following structures: 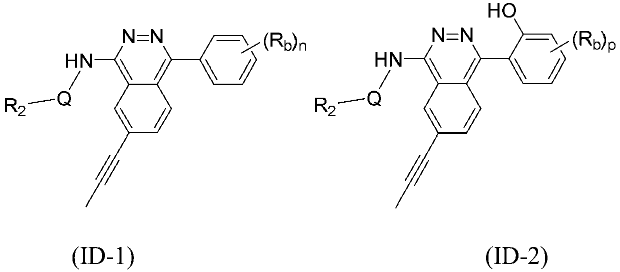 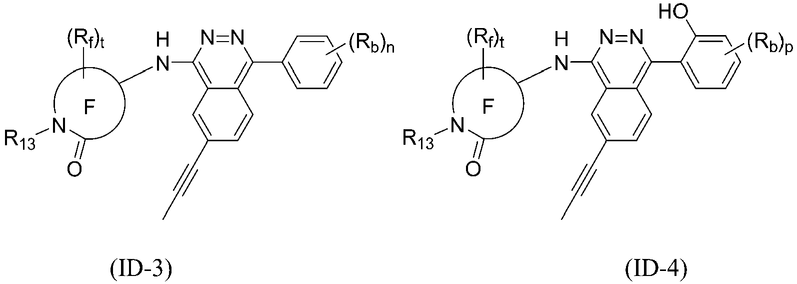
wherein ring F, Q, R₂, R_{b}, R₁₃, and R_{f} are each independently as defined in any one of claims 1 to 4; n is selected from 0, 1, 2, 3, 4 or 5; p is selected from 0, 1, 2, 3 or 4; and t is selected from 0, 1, 2, 3 or 4.

6. The compound according to any one of claims 1 to 5, wherein the compound has the following structures: 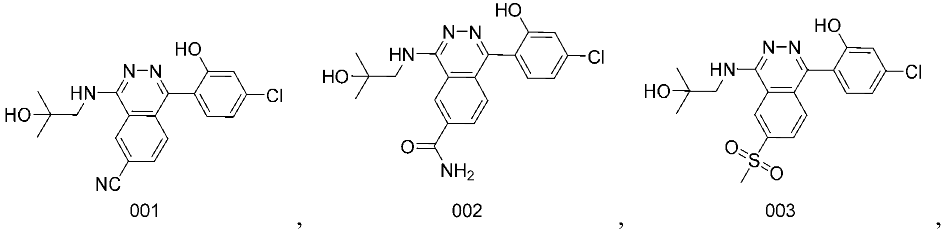 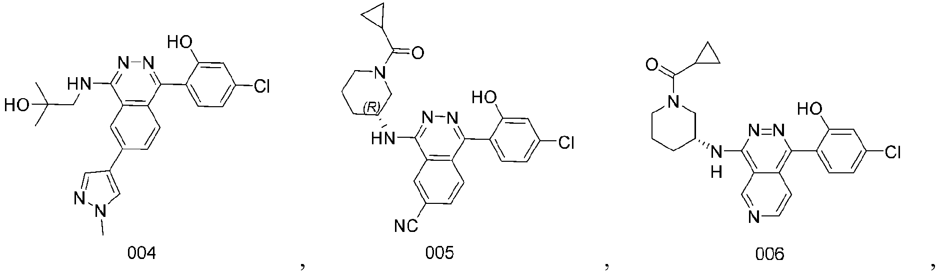 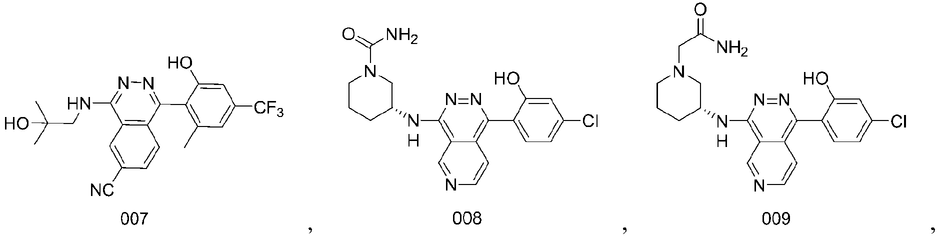 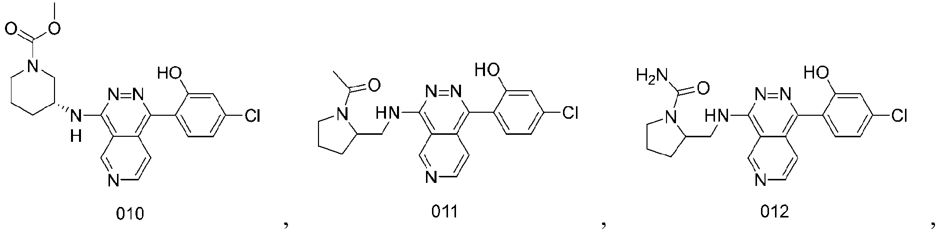 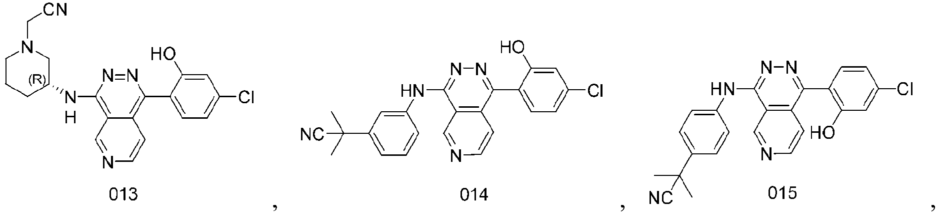 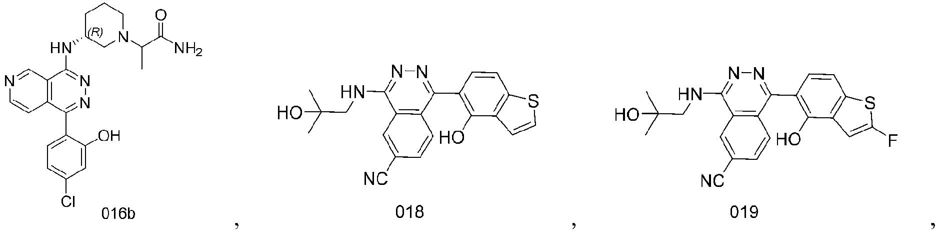 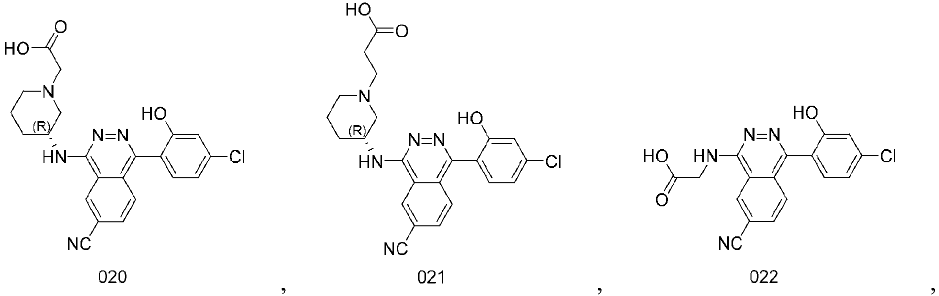 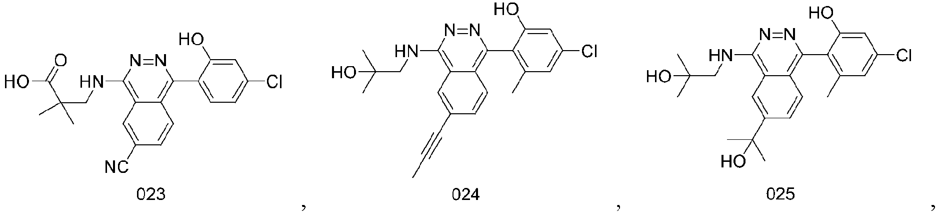 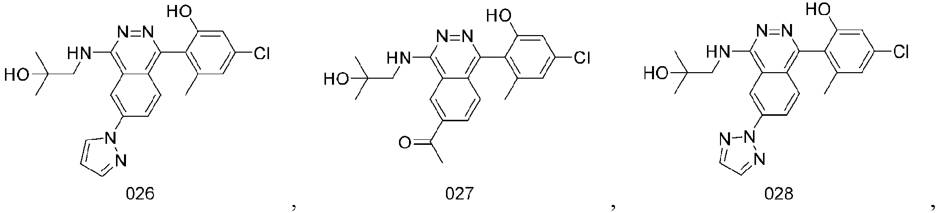 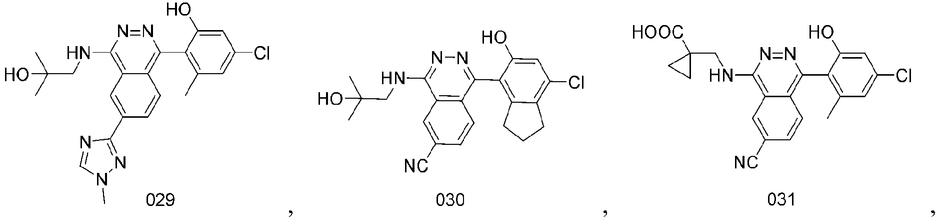 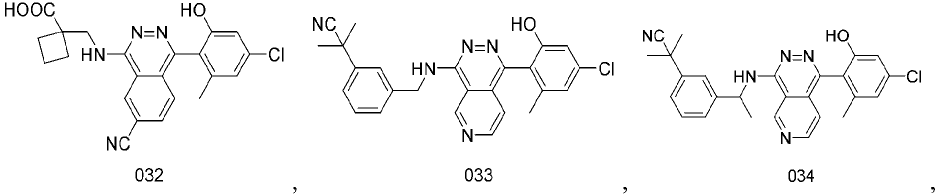 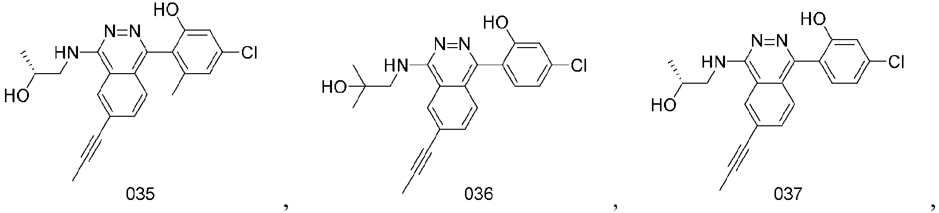 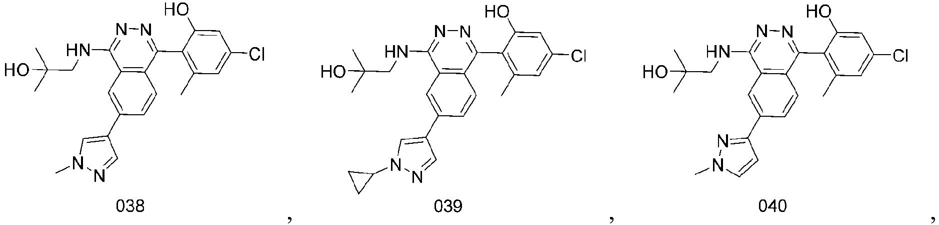 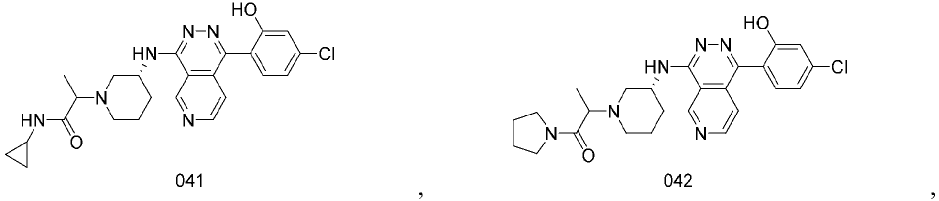 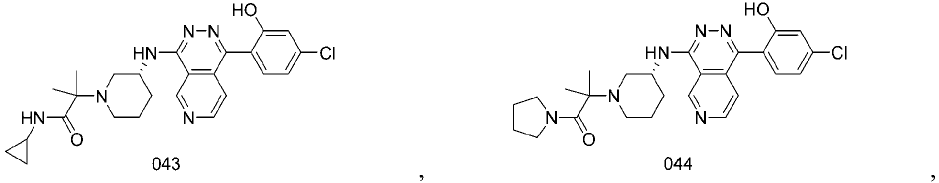 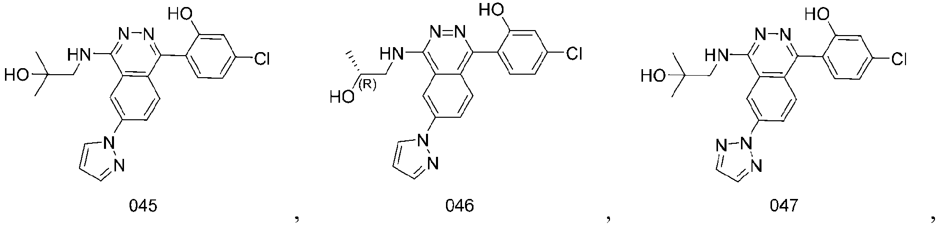 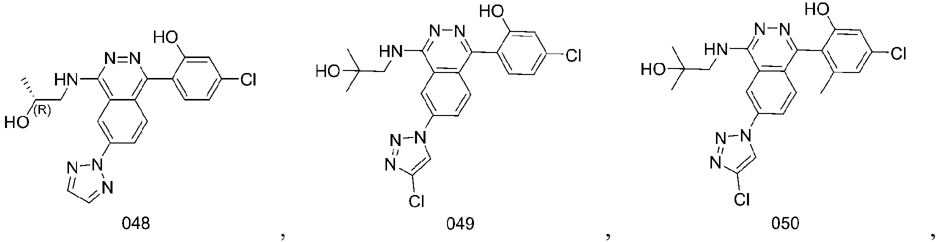 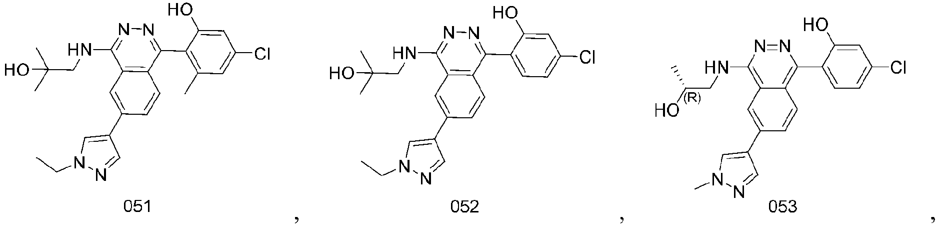 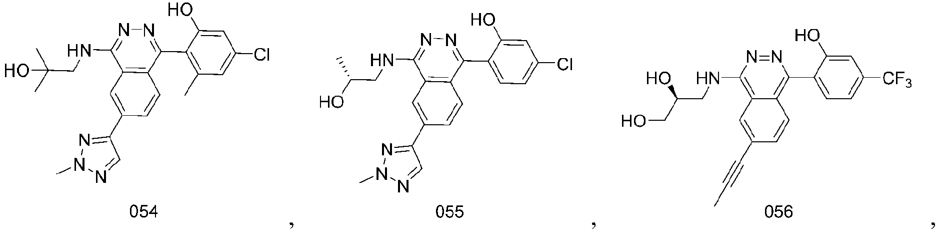 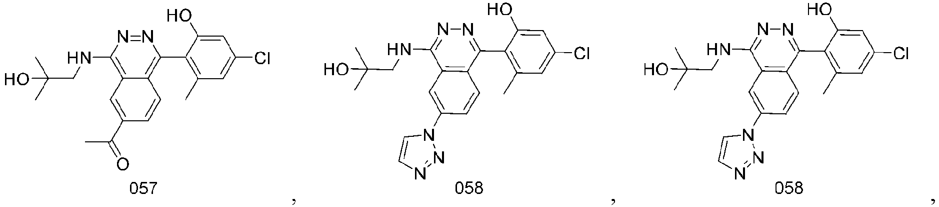 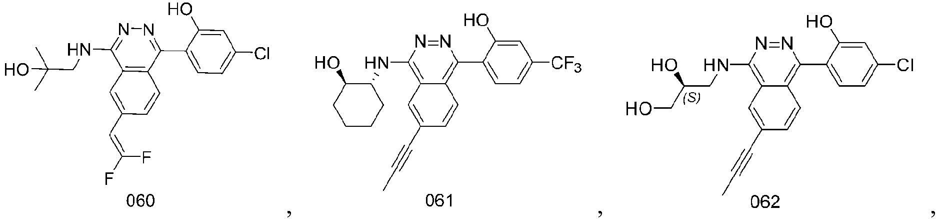 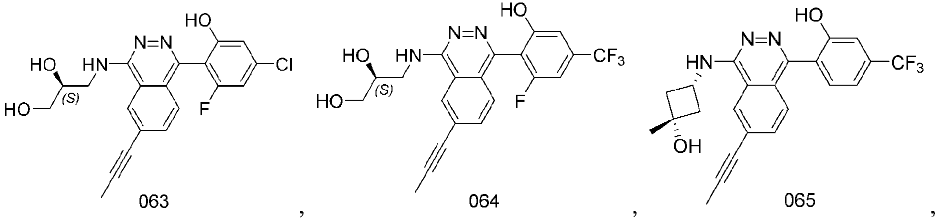 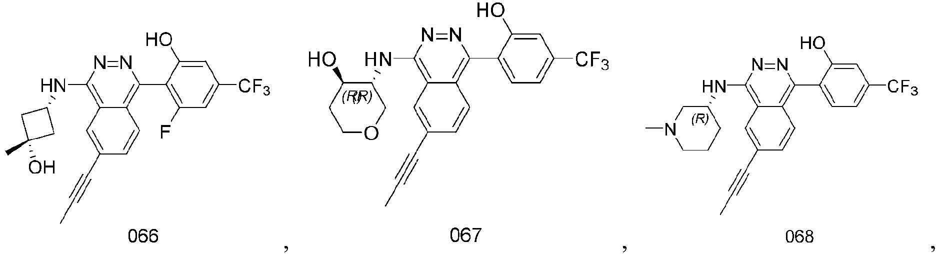 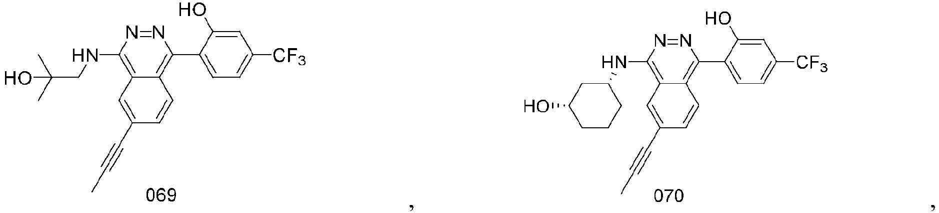 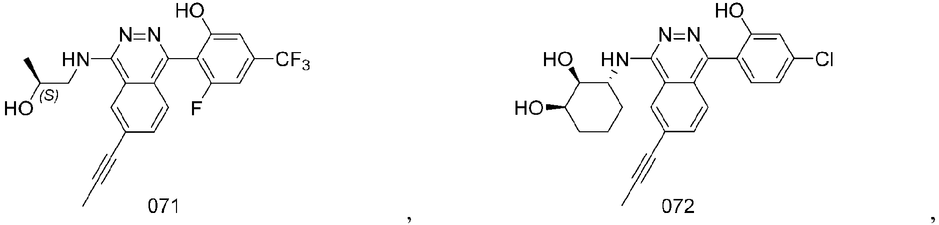 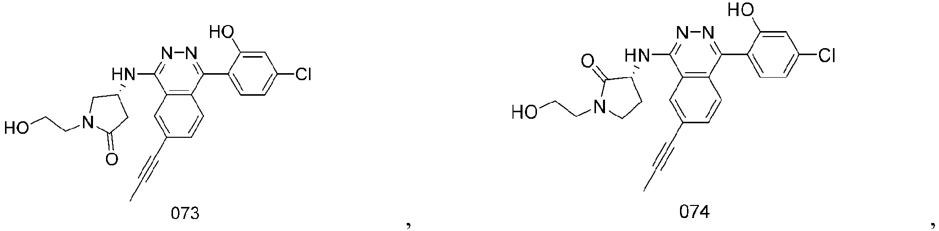 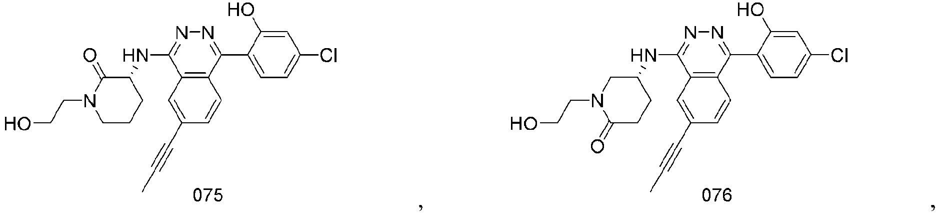 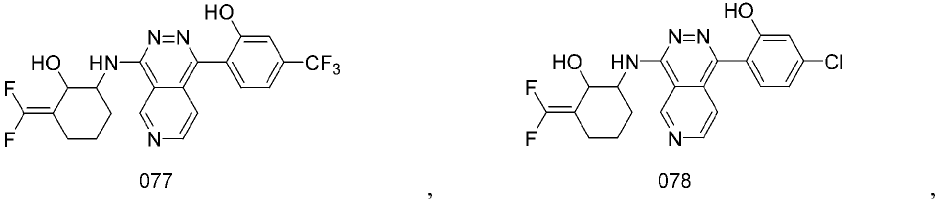 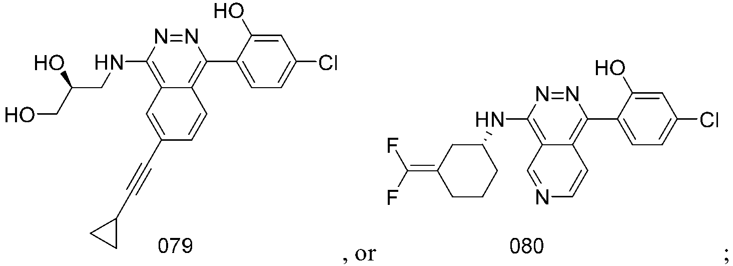 preferably, the compound represented by formula (I) is selected from the following structures: 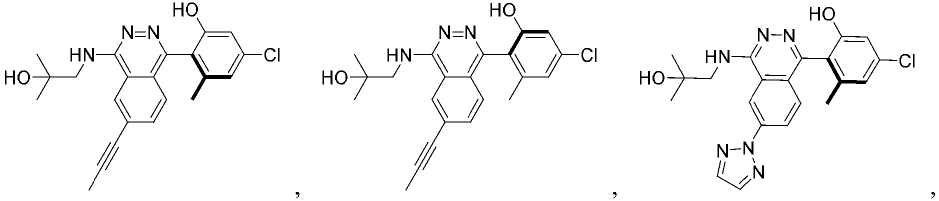 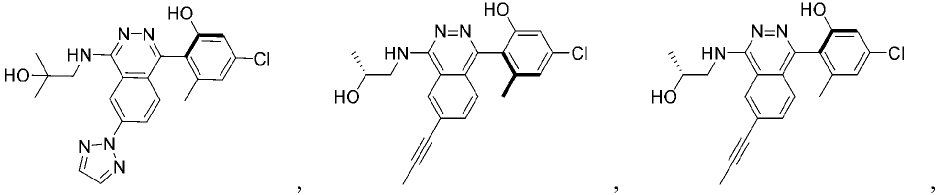 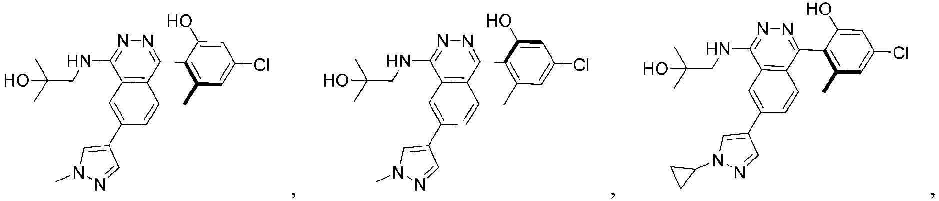 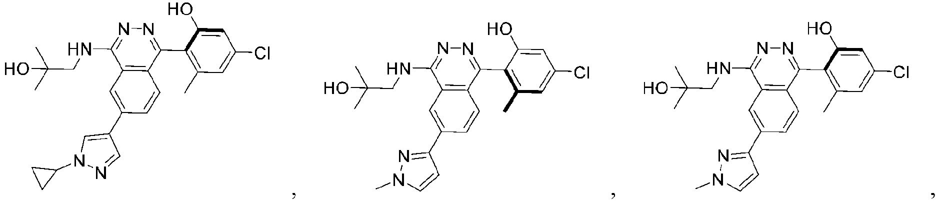 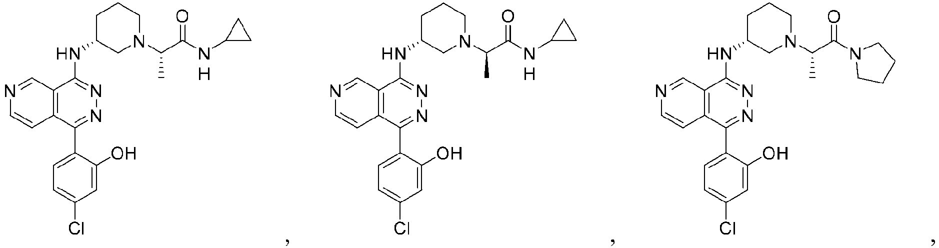 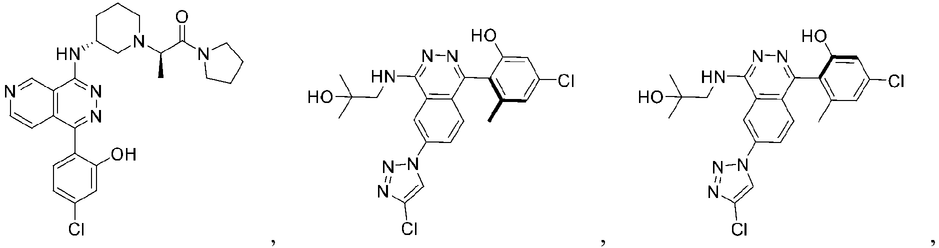 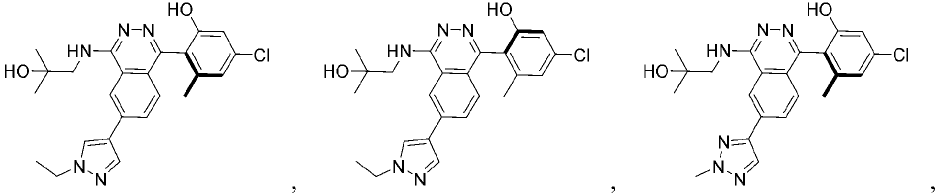 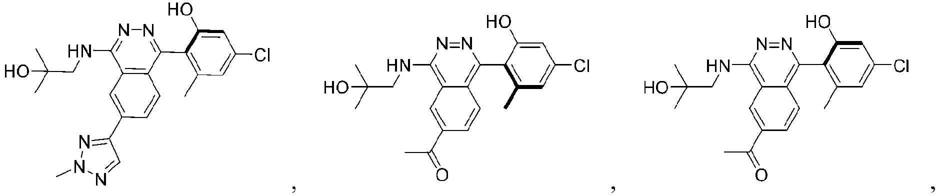 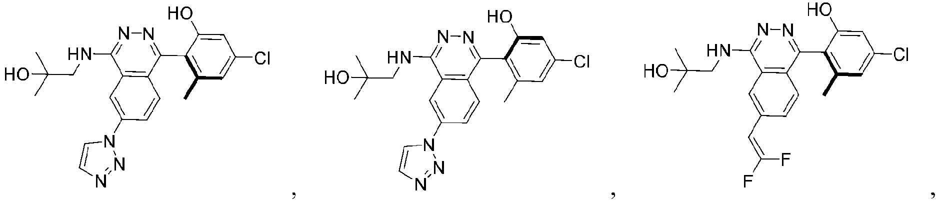 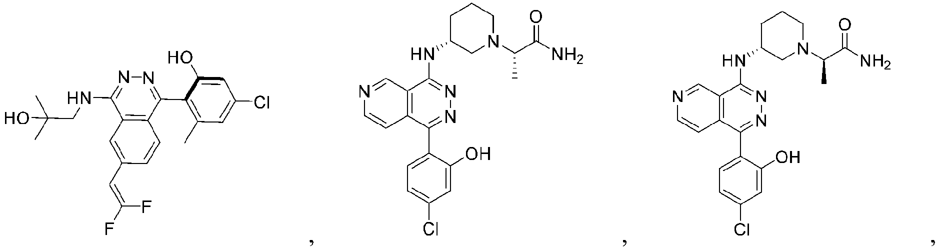 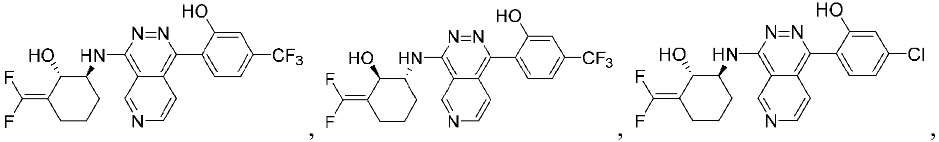 or

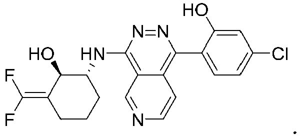

7. A method for preparing the compound according to any one of claims 1 to 6, comprising the following steps: 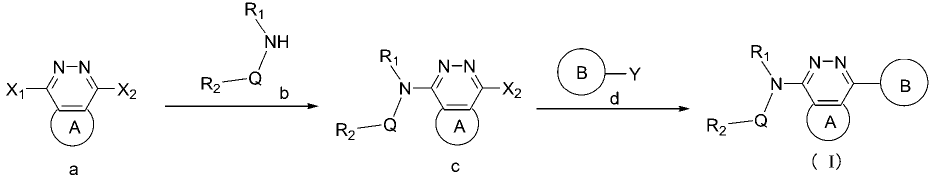
(1) allowing compound a to react with compound b to give compound c; and
(2) allowing compound c to react with compound d to give the compound represented by formula (I);
wherein ring A, ring B, R₁, R₂ and Q are each independently as defined herein; X₁ and X₂ are the same or different and are each independently selected from leaving groups, such as halogen, such as F, Cl, Br, or I; Y is selected from leaving groups, such as halogen, such as F, Cl, Br, or I; or borono ( 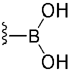 ).

8. A pharmaceutical composition comprising a therapeutically effective amount of at least one of the compound, racemate, stereoisomer, tautomer, isotope-labeled counterpart, solvate, polymorph, pharmaceutically acceptable salt thereof or prodrug thereof according to any one of claims 1 to 6;
preferably, the pharmaceutical composition further comprises one or more pharmaceutically acceptable excipients.

9. A method for treating NLRP3-mediated diseases, comprising administering to a patient a prophylactically or therapeutically effective amount of at least one of the compound, racemate, stereoisomer, tautomer, isotope-labeled counterpart, solvate, polymorph, pharmaceutically acceptable salt thereof or prodrug thereof according to any one of claims 1 to 6 or the pharmaceutical composition according to claim 8;
preferably, the NLRP3-mediated disease is selected from: auto inflammatory febrile syndromes such as cryopyrin-associated periodic syndrome (CAPS), sickle cell disease, systemic lupus erythematosus (SLE), chronic liver disease, nonalcoholic steatohepatitis (NASH), gout, gouty arthritis, pericarditis, type I and type II diabetes and related complications (e.g. nephropathy, retinopathy), neuroinflammation-related disorders (e.g. multiple sclerosis, brain infection, acute injury, neurodegenerative diseases, Alzheimer's disease), atherosclerosis and cardiovascular risk (e.g. hypertension), obesity, hidradenitis suppurativa, wound healing and scarring, and cancer (e.g. carcinoma of large intestine, lung cancer, myeloproliferative neoplasms, leukemia, myelofibrosis).

10. Use of at least one of the compound, racemate, stereoisomer, tautomer, isotope-labeled counterpart, solvate, polymorph, pharmaceutically acceptable salt thereof or prodrug thereof according to any one of claims 1 to 6 or the pharmaceutical composition according to claim 8 in the preparation of a drug;
preferably, the use is for use in the preparation of a drug for the treatment of NLRP3-mediated conditions and/or diseases, such as in the preparation of an NLRP3 inhibitor drug;
preferably, the disease is, for example autoinflammatory febrile syndromes such as cryopyrin-associated periodic syndrome (CAPS), sickle cell disease, systemic lupus erythematosus (SLE), chronic liver disease, nonalcoholic steatohepatitis (NASH), gout, gouty arthritis, pericarditis, type I and type II diabetes and related complications (e.g. nephropathy, retinopathy), neuroinflammation-related disorders (e.g. multiple sclerosis, brain infection, acute injury, neurodegenerative diseases, Alzheimer's disease), atherosclerosis and cardiovascular risk (e.g. hypertension), obesity, hidradenitis suppurativa, wound healing and scarring, and cancer (e.g. carcinoma of large intestine, lung cancer, myeloproliferative neoplasms, leukemia, myelofibrosis).
